# EUROPEAN PATENT SPECIFICATION

(11) **EP 1 417 344 B1**
(45) Date of publication and mention of the grant of the patent: **15.06.2011**
(21) Application number: 02758924.1
(22) Date of filing: 17.08.2002
(51) Int. Cl.: C12N 15/10, C12N 15/12, C12N 15/62, C12N 15/63, C07K 14/47, C12Q 1/68, C12P 21/02

(54) **ZINC FINGER DOMAIN LIBRARIES**
ZINKFINGERDOMÄNENBIBLIOTHEKEN
BIBLIOTHEQUES DE DOMAINES A DOIGTS DE ZINC

(30) Priority: 17.08.2001 US 313402 P; 22.04.2002 US 374355 P
(43) Date of publication of application: 12.05.2004
(73) Proprietor: Toolgen, Inc., Yuseong-gu, Daejeon 305-390 (KR)
(72) Inventor: KIM, Jin-Soo, Samsung Apartment 113-103, 305-390 Taejon (KR); BAE, Kwang-Hee, Sejong Apartment 105-1104, 305-390 Taejon (KR); PARK, Kyung-Soon, Lucky Hana Apartment 107-1307, 305-390 Taejon (KR); KWON, Young-Do, 305-390 Taejon (KR); RYU, Eun-Huyn, 300-130 Taejon (KR); HWANG, Moon-Sun, 305-390 Taejon (KR)
(74) Representative: Goddar, Heinz J.
(86) International application number: PCT/KR2002/001560
(87) International publication number: WO 2003/016571

(56) References cited:
- WO-A-01/53480
- WO-A-01/60970
- WO-A-98/53057
- WO-A-98/54311
- WO-A1-00/15777
- WO-A1-98/53057
- WO-A1-98/54311
- WO-A2-01/25417
- WO-A2-01/40798
- WO-A2-99/48909
- US-A- 5 789 538
- US-B1- 6 242 568
- DATABASE UniProt [Online] 1 June 2001 (2001-06-01), "Mus musculus 8 days embryo whole body cDNA, RIKEN full-length enriched library, clone:5730557K01 product:hypothetical protein, full insert sequence. (Fragment)." XP002307068 retrieved from EBI accession no. UNIPROT:Q9CS56 Database accession no. Q9CS56
- DATABASE Geneseq [Online] 26 June 2001 (2001-06-26), "Human protein sequence SEQ ID NO:17276." XP002307069 retrieved from EBI accession no. GSP:AAB95192 Database accession no. AAB95192
- DATABASE UniProt [Online] 1 May 1999 (1999-05-01), "WUGSC:H_DJ0855D21.3 protein (Fragment)." XP002307070 retrieved from EBI accession no. UNIPROT:O95015 Database accession no. O95015
- DATABASE Geneseq [Online] 8 February 2001 (2001-02-08), "Human ORFX ORF299 polypeptide sequence SEQ ID NO:598." XP002307071 retrieved from EBI accession no. GSP:AAB40535 Database accession no. AAB40535
- DATABASE UniProt [Online] 1 June 1994 (1994-06-01), "Zinc finger protein 46 (Zfp-46) (Zinc finger protein MLZ-4)." XP002307072 retrieved from EBI accession no. UNIPROT:Q03309 Database accession no. Q03309
- KLUG A.: 'ZINC FINGER PEPTIDES FOR THE REGULATION OF GENE EXPRESSION' J. MOL. BIOL. vol. 293, no. 2, 1999, LONDON, GB, pages 215 - 218, XP002930866
- BARBATO G. ET AL J. BIOMOL. NMR vol. 8, no. 1, 1996, pages 36 - 48, XP002981700
- DESJARLAIS J.R. ET AL: 'LENGTH-ENCODED MULTIPLEX BINDING SITE DETERMINATION: APPLICATION TO ZINC FINGER PROTEINS.' PROC. NATL. ACAD. SCI. USA vol. 91, no. 23, 1994, WASHINGTON, US, pages 11099 - 11103, XP000749605
- CHOO Y. ET AL: 'Selection of DNA binding sites for zinc fingers using rationally randomized DNA reveals coded interactions.' PROC. NATL. ACAD. SCI. USA vol. 91, no. 23, 1994, WASHINGTON, US, pages 11168 - 11172, XP002075339

## Description

### FIELD OF THE INVENTION

This invention relates to DNA-binding proteins such as transcription factors.

### BACKGROUND OF THE INVENTION

Most genes are regulated at the transcriptional level by polypeptide transcription factors that bind to specific DNA sites within in the gene, typically in promoter or enhancer regions. These proteins activate or repress transcriptional initiation by RNA polymerase at the promoter, thereby regulating expression of the target gene. Many transcription factors, both activators and repressors, are modular in structure. Such modules can fold as structurally distinct domains and have specific functions, such as DNA binding, dimerization, or interaction with the transcriptional machinery. Effector domains such as activation domains or repression domains retain their function when transferred to DNA-binding domains of heterologous transcription factors (Brent and Ptashne, (1985) Cell 43:729-36; Dawson et al., (1995) Mol. Cell Biol. 15:6923-31). The three-dimensional structures of many DNA-binding domains, including zinc finger domains, homeodomains, and helix-turn-helix domains, have been determined from NMR and X-ray crystallographic data.

### SUMMARY OF THE INVENTION

The object of the invention is defined in the claims.

The invention provides a rapid and scalable cell-based method for identifying and constructing chimeric proteins, e.g., transcription factors. Such transcription factors can be used, for example, for altering the expression of endogenous genes in biomedical and bioengineering applications. The transcription factors are assayed *in vivo,* i.e., in intact, living cells in culture. A database can be constructed from the results of the assays. The database can be used to design other chimeric transcription factors. Libraries of chimeric transcription factors can be prepared and screened. It is also possible that the chimeric proteins bind and regulate molecules other than DNA, e.g., proteins and RNA, in particular small non-coding RNAs (ncRNAs).

In one aspect, the invention features libraries of nucleic acids that encode chimeric zinc finger proteins. The term "library" refers to a physical collection of similar, but non-identical biomolecules. The collection can be, for example, together in one vessel or physically separated (into groups or individually) in separate vessels or on separate locations on a solid support. Duplicates of individual members of the library may be present in the collection.

In particular, the invention is directed to a library of zinc finger polypeptides and to a library of polynucleotides encoding zinc finger polypeptides, as defined in the claims. The invention is further directed to a zinc finger polypeptide and to a polynucleotide encoding a zinc finger polypeptide, as defined in the claims. The invention is also directed to a method of producing said library of polynucleotides, to a method of generating a zing finger polypeptide and to a method of identifying a nucleic acid encoding a zinc finger polypeptide, as defined in the claims.

A first library includes a plurality of nucleic acids, each nucleic acid encoding a polypeptide comprising at least a first, second, and third zinc finger domain$. As used herein, "first, second and third" denotes three separate domains that can occur in any order in the polypeptide: e.g., each domain can occur N-terminal or C-terminal to either or both of the others. The first zinc finger domain differs between the nucleic acids of the plurality. The second zinc finger domain differs between the nucleic acids of the plurality. At least 10 different first zinc finger domains are represented in the library. In one implementation, at least 0.5, 1, 2, 5%, 10%, or 25% of the members of the library have one or both of the following properties: (1) each represses transcription of at least one p1G reporter plasmid at least 1.25 fold *in vivo;* and (2) each binds at least one target site with a dissociation constant of no more than 7, 5, 3, 2, 1, 0.5, or 0.05 nM. The first and second zinc finger domains can be from different naturally-occurring proteins or are positioned in a configuration that differs from their relative positions in a naturally-occurring protein. For example, the first and second zinc finger domains may be adjacent in the polypeptide, but may be separated by one or more intervening zinc finger domains in a naturally occurring protein.

As used herein, the "dissociation constant" refers to the equilibrium dissociation constant of a polypeptide for binding to a 28-basepair double-stranded DNA that includes one 9-basepair target site. The dissociation constant is determined by gel shift analysis using a purified protein that is bound in 20 mM Tris pH 7.7, 120 mM NaCl, 5 mM MgCl₂, 20 µM ZnSO₄, 10% glycerol, 0.1% Nonidet P-40, 5 mM DTT, and 0.10 mg/mL BSA (bovine serum albumin) at room temperature. Additional details are provided in Example 10 and Rebar and Pabo ((1994) Science 263:671-673).

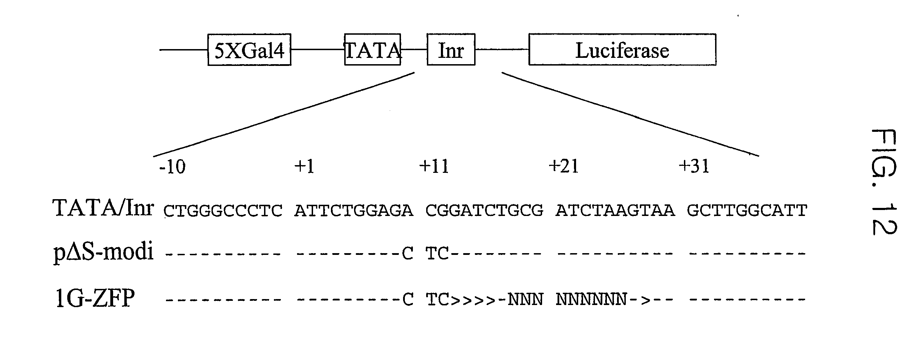

As used herein, the "repression of transcription of a p1G reporter plasmid" refers to the fold repression of the luciferase reporter gene of a p1G reporter plasmid that has a given 9-basepair target site positioned downstream of the TATA box as depicted in Fig. 12. The fold repression is determined by the assay set forth in Example 68, requiring the transfection of HEK 293 cells with appropriate expression and reporter plasmids.

The first and second zinc finger domains naturally-occurring domains, e.g., as described below.

A second library includes a plurality of nucleic acids, each nucleic acid encoding a polypeptide that includes at least a first and a second zinc finger

domains. The first and second zinc finger domains of each polypeptide (1) are identical to zinc finger domains of different naturally occurring proteins (and generally do not occur in the same naturally occurring protein or are positioned in a configuration that differs from their relative positions in a naturally-occurring protein), (2) differ by no more than four, three, two, or one amino acid residues from domains of naturally occurring proteins, or (3) are non-adjacent zinc finger domains from a naturally occurring protein. Identical zinc finger domains refer to zinc finger domains that are identical at each amino acid from the first metal coordinating residue (typically cysteine) to the last metal coordinating residue (typically histidine). The first zinc finger domain differs between the nucleic acids of the plurality, and the second zinc finger domain differs between the nucleic acids of the plurality. The naturally occurring protein can be any eukaryotic zinc finger protein: for example, a fungal (e.g., yeast), plant, or animal protein (e.g., a mammalian protein, such as a human or murine protein). Each polypeptide can further include a third, fourth, fifth, and/or sixth zinc finger domain. Each zinc finger domain can be a mammalian, e.g., human, zinc finger domain. Typically when the first and second zinc finger domains are from the same naturally occurring protein that are in an artificial configuration, e.g., one that was previously N-terminal is now C-terminal, domains that were previously non-adjacent are adjacent, and so forth.

For the first and second featured libraries described above, the first and/or second zinc finger domain of each polypeptide can be selected from Tables 5, 6, and 7. In another example, the first and/or second zinc finger domain of at least one polypeptide can be selected from Tables 5, 6, and 7. In one embodiment, at least 1%, 5%, 10%, 25%, 50%, 75%, or all of the zinc finger domains listed in Tables 5, 6, and 7 are encoded by at least one nucleic acid of the plurality. The plurality of first zinc finger domains encoded by respective members of the plurality of nucleic acids can include a sufficient number of different zinc finger domains to bind specifically to at least 10, 20, 30, 40, or 50 different 3-base pair DNA sites.

A given zinc finger domain is said to "bind specifically" to a given 3-base pair DNA site if a chimeric protein that includes fingers 1 and 2 of Zif268 and the given zinc finger domain has an affinity of at least 5 nM for a target site that includes both the given 3-base pair DNA site and the 5-bp sequence, 5'-GGGCG-3', that is recognized by fingers I and 2 of Zif268. Fingers 1 and 2 of Zif268 have the polypeptide sequence: ERPYACPVESCDRRFSRSDELTRHIRIHTGQKPFQCRICMRNFSRSDHLTTHI includes a phagemid nucleic acid. A virus or virus particle can infect a mammalian cell (e.g., a retrovirus and adenovirus) or a bacterial cell (e.g., a bacteriophage). In another example, the polypeptide is covalently attached to the nucleic acid, e.g., by a puromycin linkage.

In another embodiment, each polypeptide further includes an activation or repression domain. The first and the second zinc finger domains of each polypeptide can be adjacent to each other or separated, e.g., by an intervening domain or linker.

In one embodiment, each nucleic acid of the library is in a cell. The nucleic acid can be expressed within the cell. The cell can also include a heterologous reporter construct that includes a target DNA site operably linked to a reporter gene. The cell can be a yeast cell; an animal cell such as a cell of a mammal, a bird, or an insect; a bacterial cell; or a plant cell.

All nucleic acids of the library can be located within a single container or on a single surface. In another implementation, subsets of the plurality of nucleic acids are located in separate containers, on separate surfaces, or on separate parts of the same surface. In still another implementation, each nucleic acid of the library is addressable, e.g., uniquely addressable. An "addressable" element is located at a defined spatial location that can be accessed under appropriate conditions to retrieve the addressed element. For example, each nucleic acid can be located in a well of a microtitre plate, on a planar array, or within a frozen sample of cells.

Each nucleic acid of the library can be referenced in a machine-readable medium by a pointer associated with information about the ability of the polypeptide encoded by the nucleic acid to recognize a target site. The information can include, for example, a value representing the binding affinity of the polypeptide for the target site, a value predictive of the ability of the polypeptide to recognize the target site, or a set of values reflecting the effect of the polypeptide on the expression of endogenous genes within a cell (e.g., a human cell).

Similarly, the invention provides a library of polypeptides. The library includes a plurality of polypeptides that are each encoded by a nucleic acid of a nucleic acid library featured herein. The polypeptide library can also include any of appropriate feature of a nucleic acid library described herein, except that the feature is embodied at the protein level.

The invention also provides a kit that includes a library described herein, and a machine readable medium that includes, encoded therein, information about the ability of a polypeptide to recognize a target DNA site, wherein each nucleic RTH (SEQ ID NO:198). The terms "recognize" and "specifically bind" are used interchangeably and refer to the discrimination for a binding site of a zinc finger domain in the above Zif268 fusion assay.

Optionally, the plurality of nucleic acids collectively encode a sufficient number of different zinc finger domains to recognize at least 10, 20, 30, 40, or 50 different 3-base pair DNA sites. In one embodiment, the plurality of nucleic acids collectively encode a sufficient number of different zinc finger domains to recognize no more than 40, 30, 20, 10, or 5 different 3-base pair DNA sites.

The plurality of nucleic acids can collectively encode at least 5, 10, 20, 30, or 40 different first zinc finger domains and/or at least 5, 10, 20, 30, or 40 different second zinc finger domains. The plurality of nucleic acids can include at least 10, 50, 200, 500, 1000, 5000, 10 000, 20 000, 25 000, or 40 000 different nucleic acids (i.e., with different sequences). In some cases, the plurality can include no more than 100, 500, 2000, 5000, 15 000, 30 000, or 50 000 nucleic acids. The plurality of nucleic acids can constitute at least 20%, 50%, 70%, 80%, 90%, 95%, or 100% of the library by molar ratio.

In one embodiment, the polypeptides encoded by nucleic acids of the plurality include different numbers of zinc finger domains. For example, polypeptides encoded by a first subset can include four zinc finger domains, and polypeptides encoded by a second subset can include five zinc finger domains. Another combinations is one with three, four, and five domains, or four, five and six domains.

In one embodiment, the polypeptides encoded by nucleic acids of the plurality include different types of transcriptional regulatory domains. For example, polypeptides encoded by a first subset can include a transcriptional activation domain, and polypeptides encoded by a second subset can include a transcriptional repression domain. Still another subset can be devoid of a transcriptional regulatory domain. This embodiment enables screening of the library without bias for a particular type of transcription factor.

In one embodiment, each nucleic acid is immobilized on a solid support. In still another embodiment, each nucleic acid is attached to the polypeptide that it encodes. The attachment can be covalent or non-covalent. For example, the polypeptide encoded by each nucleic acid can be attached to the exterior of a virus or viral particle, and the nucleic acid is packaged within the virus or viral particle. A "virus" refers to a genetic entity that can infect a host cell and replicate. A "virus particle" refers to a genetic entity that can infect a host cell, but cannot replicate. An example of a virus particle is a filamentous phage coat package that acid or polypeptide of the library is referenced in a machine readable medium by a pointer associated with information about the ability of the respective polypeptide encoded by the nucleic acid to recognize a target DNA site. For example, the information can include a value representing the binding affinity of the polypeptide for the target site or a value predictive of the ability of the polypeptide to recognize the target site. The kit can further include computer-readable instructions that enable a user to interface with the information.

In another aspect, the invention features a polypeptide that includes a first and a second zinc finger domain. Each zinc finger domain has a sequence selected from Tables 5, 6, and 7. The first and second zinc finger domains are from different naturally occurring polypeptides. The polypeptide can further include third, fourth, and fifth zinc finger domains. Each of these domains can also have a sequence selected from Tables 5, 6, and 7. Typically, the zinc finger domains are positioned adjacent to each other to form an array of zinc finger domains. Such an array is a polypeptide unit that is uninterrupted by other types of structural or functional protein domains.

The invention also features a polypeptide that includes a first, second, and third zinc finger domains. Each domain is naturally occurring. At least two of the domains occur in different naturally occurring polypeptides. Further, the polypeptide has one or both of the following properties: (1) each represses transcription of at least one p1G reporter plasmid at least 1.25, 1.5, 1.7, 1.9, 2.0, or 2.5 fold *in vivo;* and (2) each binds at least one target nucleic acid site with a dissociation constant of no more than 7, 5, 3, 2, 1, 0.5, or 0.05 nM. The target site can be DNA or RNA. The first, second, and/or third finger can be selected from Tables 5, 6, and 7. In one embodiment, the first, second, and third zinc finger domains of each given polypeptide are represented by the domains listed together in a row of Table 10, e.g.,. a row above row 113, e.g., a row that includes all human zinc finger domains. In one embodiment, the polypeptide only includes domains from naturally occurring polypeptides (e.g. mammalian, e.g., human polypeptides).

In one embodiment, the first domain is N-terminal to the second domain, the second domain is N-terminal to the third domain, and the second domain occurs in a different naturally occurring polypeptide than the first domain. Each of the first, second, and third domains can occur in a different naturally occurring polypeptide.than the other two.

With respect to any featured polypeptide, the polypeptide can further include a heterologous sequence, e.g., a nuclear localization signal, a small molecular binding domain (e.g., a steroid binding domain), an epitope tag or purification handle, a catalytic domain (e.g., a nucleic acid modifying domain, a nucleic acid cleavage domain, or a DNA repair catalytic domain), a transcriptional function domain (e.g., an activation domain, a repression domain, and so forth), a protein transduction domain (e.g., from HIV tat), and/or a regulatory site (e.g., a phosphorylation site, ubiquitination site, or protease cleavage site).

The polypeptide can be attached (covalently or non-covalently) to a solid support, e.g., a bead, matrix, or planar array. The polypeptide can also be attached to a label such as a radioactive compound, a fluorescent compound, another detectable entity, or a component of a detection system (e.g., a chemiluminescent agent).

The invention also includes an isolated nucleic acid sequence encoding one of the aforementioned polypeptides. The nucleic acid can further include an operably linked regulatory sequence, e.g., a promoter, a transcriptional enhancer, a 5' untranslated region, a 3' untranslated region, a virus packaging sequence, and/or a selectable marker. The nucleic acid can be packaged in a virus, e.g., a virus that can infect a mammalian cell, e.g., a lentivirus, retrovirus, pox virus, or adenovirus.

The invention further provides a cell that contains the nucleic acid. The cell can be within a tissue in a subject organism or in culture. The cell can be an animal (e.g., mammalian), plant, or microbial (e.g., fungal or bacterial) cell. The invention still further includes a non-human transgenic mammal, e.g., a mouse, rat, pig, rabbit, cow, goat, or sheep. The genetic complement of the transgenic mammal includes the nucleic acid sequence encoding the chimeric zinc finger polypeptide described above and elsewhere herein. The invention also includes method of producing the polypeptide, e.g., by expressing the nucleic acid, and of using the polypeptide, e.g., to regulate endogenous genes or viral genes in a cell.

In still another aspect, the invention features a method of evaluating one or more polypeptides encoded by a nucleic acid library. The method includes: providing the library, in which each of a plurality of library nucleic acids is located in a cell (with one or more (but not all) of the members of the library in any given cell); expressing each of the plurality of nucleic acids in the cell in which it resides; and identifying a cell having altered expression of the reporter gene relative to a cell that is void of a library member, thereby identifying a nucleic acid encoding a polypeptide that recognizes the target DNA site.

In another aspect, the invention features a method of constructing a library of chimeric zinc finger proteins. The method includes: providing a set of nucleic acids, each comprising a sequence encoding a zinc finger domain selected from Tables 5, 6, and 7; and joining each nucleic acid of the set to one or more, preferably two, three, or four other nucleic acids of the set to form a plurality of chimeric nucleic acids. Each chimeric nucleic acid can be located in a vector, e.g., a mammalian expression vector.

In one embodiment, the method further includes, after the joining, introducing one or more of the plurality of chimeric nucleic acids into cells and expressing the one or more chimeric nucleic acids. In another embodiment, the method further includes individually introducing one or more of the chimeric nucleic acids into a cell, e.g., a mammalian cell; expressing the chimeric nucleic acids; and monitoring expression of a gene or protein in the cell. The cell can include a reporter construct, e.g., a construct that includes a target site inferred to be recognized by the polypeptide encoded by the chimeric nucleic acid such that the polypeptide binds to the target site and either induces or represses expression of the reporter gene.

In yet another aspect, the invention features a method of characterizing a chimeric zinc finger protein, e.g., a zinc finger protein described herein. The method includes: introducing a nucleic acid that encodes the protein into a cell; expressing the nucleic acid; and determining the profile of expression of endogenous genes in the cell. Such an expression profile includes a plurality of values, wherein each value corresponds to the level of expression of a different gene, splice-variant or allelic variant of a gene (i.e., mRNA level) or the abundance of a translation product (i.e., protein level). The value can be a qualitative or quantitative assessment of the level of expression of the gene or the translation product of the gene, i.e., an assessment of the abundance of 1) an mRNA transcribed from the gene, or 2) the polypeptide encoded by the gene.

The method can further include comparing the determined expression profile to at least one reference expression profile, to thereby characterize the chimeric zinc finger protein. The reference profile can be the expression profile of a related cell that lacks a heterologous chimeric zinc finger protein or that includes a control vector. The comparison can identify the regulation of one or more genes as altered by the chimeric zinc finger protein. In one embodiment, the sample expression profile is compared to a reference profile to produce a difference profile. The sample expression profile can also be compared in multi-dimensional space to a cluster of reference profiles. In one embodiment, the sample expression profile is determined using a nucleic acid array. In another embodiment, the sample expression profile is determined using a method and/or apparatus that does not require an array (e.g., SAGE or quantitative PCR with multiple primers).

The method can further include determining or inferring a target binding site for the polypeptide, and identifying occurrences of the target binding site in a regulatory nucleic acid sequence of a gene whose regulation is altered by the polypeptide. The method can be used to distinguish direct from indirect targets.

In another aspect, the invention features a method that includes: providing a plurality of nucleic acids, each nucleic acid of the plurality encoding a polypeptide comprising a first and a second zinc finger domain, wherein the first and second zinc finger domains of the polypeptide encoded by each nucleic acid of the plurality are identical to zinc finger domains from different naturally occurring mammalian proteins, the first zinc finger domain differs between the nucleic acids of the plurality, and the second zinc finger domain differs between the nucleic acids of the plurality; introducing each nucleic acid of the plurality into a cell that has a given trait prior to the introducing thereby providing a plurality of cells; expressing the introduced nucleic acid in each cell of the plurality of cells; and identifying a cell from the plurality of cells in which the given trait is altered. The method can include other features described herein. Exemplary traits include enhanced sensitivity or resistance to a condition (e.g., stress), altered proliferative ability, altered pathogenicity, and altered product production (e.g., metabolite production).

In yet another aspect, the invention features a method of identifying a chimeric zinc finger protein that can bind to a particular target site. The method includes: providing data records, each record associating an identifier for a naturally-occurring human zinc finger domain and at least one 3- or 4-basepair subsite that is recognized by the zinc finger domain referenced by the identifier; parsing the target site into at least two 3- or 4-basepair subsites; for each of the subsites, retrieving a set of the identifiers from the data records, the set comprising identifiers for the zinc finger domains that recognize the subsite; and designing a polypeptide that comprises a zinc finger domain for each of the subsites, the zinc finger domain being referenced by an identifier from the set for the respective subsite.

The data records can include a record that identifies a human zinc finger domain selected from Tables 5, 6, and 7. The method can further include the step of synthesizing a nucleic acid that encodes the polypeptide and/or synthesizing the polypeptide *in vitro.* The method can also include the step of assessing the binding of the polypeptide to the target site, e.g., using an *in vitro* binding assay or an *in vivo* assay such as an assay for reporter gene expression. The synthesized polypeptide can further include an activation or repression domain.

In one embodiment, the method further includes assessing the ability of the polypeptide to alter the expression of one or more endogenous genes. The assessing can include profiling the expression of multiple endogenous genes, e.g., using nucleic acid microarrays. The method can also further include contacting the polypeptide with a DNA that includes the target site, e.g., *in vitro.*

In another embodiment, the method further includes retrieving a nucleic acid encoding the polypeptide from an addressed library of nucleic acids, each nucleic acid of the library including a sequence encoding first and second zinc finger domains.

The invention also features a method that includes: storing data records in a machine readable medium, each record associating a zinc finger domain identifier and at least one 3- or 4-basepair subsite that is recognized by the zinc finger domain referenced by the identifier; retrieving from the stored records one or more identifiers that are associated with a subsite of interest; and constructing a nucleic acid encoding a polypeptide that includes (a) a zinc finger domain referenced by one of the one or more retrieved identifiers and (b) a second DNA binding domain. The second DNA binding domain can be a zinc finger domain.

The constructing can include constructing a plurality of nucleic acids, each nucleic acid of the plurality comprising a sequence encoding a zinc finger domain referenced by the retrieved identifiers. The method can further include, for each nucleic acid of the plurality, expressing the nucleic acid in a cell and assessing the change in the level of transcription of a given gene when the nucleic acid is expressed, relative to the level of transcription of the given gene when the nucleic acid is absent or not expressed. The assessing can further include assessing the level of transcription of multiple genes, e.g., by profiling.

In another aspect, the invention features a computer-based method that includes: storing information that includes associations between (a) each of a plurality of naturally occurring zinc finger domains referenced in Tables 5, 6, and 7 and (b) one or more subsites recognized by the domain; receiving a user query that comprises a string specifying a target nucleic acid sequence; and filtering the information to identify combinations of zinc finger domains predicted to recognize a site within the target nucleic acid sequence.

The method can further include displaying the combinations to a user or physically locating a library nucleic acid or polypeptide that includes one of the identified combinations of zinc finger domains from an addressed library of nucleic acids or polypeptides.

In still another aspect, the invention features a database that is stored on machine-readable medium. The database includes (i) data representing (a) individual naturally-occurring zinc finger domains, (b) nucleic acid sites, and (c) chimeric polypeptides that comprise a plurality of naturally-occurring zinc finger domains; and (ii) associations that relate (1) individual zinc finger domains and nucleic acid sites recognized by the respective individual domains; (2) chimeric polypeptides and their respective component zinc finger domains; and (3) chimeric polypeptides and nucleic acid sites recognized by the respective chimeric polypeptides. The database can enable a user to identify combinations of zinc finger domains predicted to recognize a site within the target nucleic acid sequence.

The data can further represent (d) addressable locations, the locations being associated with resident chimeric polypeptides of an arrayed polypeptide library or (e) an expression profile, as described above. Each expression profile can be associated with a chimeric polypeptide.

Also featured is a library that includes a plurality of polypeptides, each polypeptide comprising a first and a second zinc finger domain, wherein the first and second zinc finger domains of each polypeptide are identical to mammalian zinc finger domains that are from different naturally occurring polypeptides, the first zinc finger domain differs between polypeptides of the plurality, and the second zinc finger domain differs between polypeptides of the plurality. Each polypeptide of the library can be attached to a solid support (e.g., a bead, matrix, or planar array).

The invention further provides a method of profiling a test nucleic acid. The method includes: contacting the test nucleic acid with the polypeptides of a library described herein; and identifying one or more polypeptides that specifically bind to the test nucleic acid. The polypeptides can be immobilized on an addressable array or attached to a virus particle.

The invention features a method of identifying a peptide domain that recognizes a target site on a DNA. This method is sometimes referred to herein as the "domain selection method" or the *"in vivo* screening method." The method includes providing (1) cells containing a reporter construct and (2) a plurality of hybrid nucleic acids. The reporter construct has a reporter gene operably linked to a promoter that has both a recruitment site and a target site. The reporter gene is expressed above a given level when a transcription factor recognizes (i.e., binds to a degree above background) both the recruitment site and the target site of the promoter, but not when the transcription factor recognizes only the recruitment site of the promoter. Each hybrid nucleic acid of the plurality encodes a non-naturally occurring protein with the following elements: (i) a transcription activation domain, (ii) a DNA binding domain that recognizes the recruitment site, and (iii) a test zinc finger domain. The amino acid sequence of the test zinc finger domain varies among the members of the plurality of hybrid nucleic acids. The method further includes: contacting the plurality of nucleic acids with the cells under conditions that permit at least one of the plurality of nucleic acids to enter at least one of the cells; maintaining the cells under conditions permitting expression of the hybrid nucleic acids in the cells; and identifying a cell that expresses the reporter gene above the given level as an indication that the cell contains a hybrid nucleic acid encoding a test zinc finger domain that recognizes the target site.

The DNA binding domain, i.e., the domain that recognizes the recruitment site and does not vary among members of the plurality, can include, for example, one, two, three, or more zinc finger domains. The cells utilized in the method can be prokaryotic or eukaryotic. Exemplary eukaryotic cells are yeast cells, e.g. *Saccharomyces cerevisiae, Schizosaccharomyces pombe,* or, *Pichia pasteuris;* insect cells such as Sf9 cells; and mammalian cells such as fibroblasts or lymphocytes.

The "given level" is the amount of expression observed when the transcription factor recognizes the recruitment site, but not the target site. The "given level" in some cases may be zero (at least within the limits of detection of the assay used).

The method can include an additional step of amplifying a source nucleic acid encoding the test zinc finger domain from a nucleic acid, e.g., genomic DNA, an mRNA mixture, or a cDNA mixture, to produce an amplified fragment. The source nucleic acid can be amplified using an oligonucleotide primer. The oligonucleotide primer can be one of a set of degenerate oligonucleotides (e.g., a pool of specific oligonucleotides having different nucleic acid sequences, or a specific oligonucleotide having a non-natural base such as inosine) that anneals to a nucleic acid encoding a conserved domain boundary. Alternatively, the primer can be a specific oligonucleotide. The amplified fragments are utilized to produce a hybrid nucleic acid for inclusion in the plurality of hybrid nucleic acids used in the aforementioned method.

The method can further include the steps of (i) identifying a candidate zinc finger domain amino acid sequence in a sequence database; (ii) providing a candidate nucleic acid encoding the candidate zinc finger domain amino acid sequence; and (iii) utilizing the candidate nucleic acid to construct a hybrid nucleic acid for inclusion in the plurality of hybrid nucleic acids used in the aforementioned method. The database can include records for multiple amino acid sequences, e.g., known and/or predicted proteins, as well as multiple nucleic acid sequences such as cDNAs, ESTs, genomic DNA, or genomic DNA computationally processed to remove predicted introns.

If desired, the method can be repeated to identify a second test zinc finger domain that recognizes a second target site, e.g., a site other than that recognized by the first test zinc finger domain. Subsequently, a nucleic acid can be constructed that encodes both the first and the second identified test zinc finger domains. The encoded hybrid protein would specifically recognize a target site that includes the target site of the first test zinc finger domain and the target site of the second test zinc finger domain.

The invention also features a method of determining whether a test zinc finger domain recognizes a target site on a promoter. This method is sometimes referred to herein as the "site selection method." The method includes the steps of providing a reporter construct and a hybrid nucleic acid. The reporter gene is operably linked to a promoter that includes a recruitment site and a target site, and is expressed above a given level when a transcription factor recognizes both the recruitment site and the target site of the promoter, but not when the transcription factor recognizes only the recruitment site of the promoter. The hybrid nucleic acid encodes a non-naturally occurring protein with the following elements: (i) a transcription activation domain, (ii) a DNA binding domain that recognizes the recruitment site, and (iii) a test zinc finger domain. The method further includes: contacting the reporter construct with a cell under conditions that permit the reporter construct to enter the cell; prior to, after, or concurrent with the aforementioned step, contacting the hybrid nucleic acid with the cell under conditions that permit the hybrid nucleic acid to enter the cell; maintaining the cell under conditions permitting expression of the hybrid nucleic acid in the cell; and detecting reporter gene expression in the cell. A level of reporter gene expression greater than the given level is an indication that the test zinc finger domain recognizes the target site.

The reporter construct and the hybrid nucleic acid can be contained in separate plasmids. The two plasmids can be introduced into the cell simultaneously or consecutively. One or both plasmids can contain selectable markers. The reporter construct and the hybrid nucleic acid can also be contained on the same plasmid, in which case only one contacting step is required to introduce both nucleic acids into a cell. In yet another implementation, one or both of the nucleic acids are stably integrated into a genome of a cell. For this method, as for any *in vivo* method described herein, the transcriptional activation domain can be replaced with a transcriptional repression domain, and a cell is identified in which the level of reporter gene expression is decreased to a level below the given level.

Another method of the invention facilitates the rapid determination of a binding preference of a test zinc finger domain by fusing two cells. The method includes: providing a first cell containing the reporter gene; providing a second cell containing the hybrid nucleic acid; fusing the first and second cells to form a fused cell; maintaining the fused cells under conditions permitting expression of the hybrid nucleic acids in the fused cell; and detecting reporter gene expression in the fused cell, wherein a level of reporter gene expression greater than the given level is an indication that the test zinc finger domain recognizes the target site. For example, the first and second cells can be tissue culture cells or fungal cells. An exemplary implementation of the method utilizes *S*. *cerevisiae* cells. The first cell has a first mating type, e.g., MAT**a**; the second cell has a second mating type different from the first, e.g., MATα. The two cells are contacted with one another, and yeast mating produces a single cell (e.g., MAT**a**/α) with a nucleus containing the genomes of both the first and second cells. The method can including providing multiple first cells, all of the same first mating type where each first cell has a reporter construct with a different target site. Multiple second cells, all of the same second mating type and each having a different test zinc finger domain, are also provided. A matrix is generated of multiple pair-wise matings, e.g., all possible pair-wise matings. The method is applied to determine the binding preference of multiple test zinc finger domains for multiple binding sites, e.g., a complete set of possible target sites.

The invention also provides a method of assaying a binding preference of a test zinc finger domain. The method includes providing (1) cells, essentially all of which contain a hybrid nucleic acid, and (2) a plurality of reporter constructs. Each reporter construct of the plurality has a reporter gene operably linked to a promoter with a recruitment site and a target site. The reporter gene is expressed above a given level when a transcription factor recognizes both the recruitment site and the target site of the promoter, but not when the transcription factor binds only the recruitment site of the promoter. The second target site varies among the members of the plurality of reporter constructs. The hybrid nucleic acid encodes a hybrid protein with the following elements: (i) a transcription activation domain, (ii) a DNA binding domain that recognizes the recruitment site, and (iii) a test zinc finger domain. The method further includes: contacting the plurality of reporter constructs with the cells under conditions that permit at least one of the plurality of reporter constructs to enter at least one of the cells; maintaining the cells under conditions permitting expression of the nucleic acids in the cells; identifying a cell that contains a reporter construct in the cell and that expresses the reporter construct above the given level as an indication that the reporter construct in the cell has a target site recognized by the zinc finger domain.

A plurality of cells, each with a different target site, can be identified by the above method if the test zinc finger domain has a binding preference for more than one target site. The method can further include identifying the cell that exhibits the highest level of reporter gene expression. Alternatively, a threshold level of reporter gene expression is determined, e.g., an increase in reporter gene expression of 2, 4, 8, 20, 50, 100, 1000 fold or greater, and all cells exhibiting reporter gene expression above the threshold are selected.

The target binding site, for example, can be between two and six nucleotides long. The plurality of reporter constructs can include every possible combination of A, T, G, and C nucleotides at two, three, or four or more positions of the target binding site.

In another aspect, the invention features a method of identifying a plurality of zinc finger domains. The method includes: carrying out the domain selection method to identify a first test zinc finger domain and carrying out the domain selection method again to identify a second test zinc finger domain that recognizes a target site different from a target site of the first test zinc finger domain. Also featured is a method of generating a nucleic acid encoding a chimeric zinc finger protein, the method includes carrying out the domain selection method twice to identify a first and second test zinc finger domain and constructing a nucleic acid encoding a polypeptide including the first and second test zinc finger domains. The nucleic acid can encode a hybrid protein that includes the two domains that specifically recognize a site that includes two subsites. The subsites are the target site of the first test zinc finger domain and target site of the second test zinc finger domain. The method can be repeated to identify additional zinc finger domains and construct a nucleic acid encoding a polypeptide including three, four, five, six, or more zinc finger domain, e.g., to specifically recognize a nucleic acid binding site.

In still another aspect, the invention features a method of identifying a DNA sequence recognized by zinc finger domains. The method includes: carrying out the site selection method to identify a first binding preference for a first test zinc finger domain, and carrying out the site selection method again to identify a second binding preference for a second test zinc finger domain. A nucleic acid can be constructed which encodes both the first and the second identified test zinc finger domains. The nucleic acid can encode a hybrid protein including the two domains that specifically recognizes a site that includes the target site of the first test zinc finger domain and target site of the second test zinc finger domain. The method can be repeated to identify additional zinc finger domains and construct a nucleic acid encoding a polypeptide including three, four, five, six, or more zinc finger domains, e.g., to specifically recognize a nucleic acid binding site.

The invention also features a method of identifying a peptide domain that recognizes a target site on a DNA. The method includes providing (1) cells containing a reporter construct and (2) a plurality of hybrid nucleic acids. The reporter construct has a reporter gene operably linked to a promoter that has both a recruitment site and a target site. The reporter gene is expressed below a given level when a transcription factor recognizes (i.e., binds to a degree above background) both the recruitment site and the target site of the promoter, but not when the transcription factor recognizes only the recruitment site of the promoter. Each hybrid nucleic acid of the plurality encodes a non-naturally occurring protein with the following elements: (i) a transcription repression domain, (ii) a DNA binding domain that recognizes the recruitment site, and (iii) a test zinc finger domain. The amino acid sequence of the test zinc finger domain varies among the members of the plurality of hybrid nucleic acids. The method further includes: contacting the plurality of nucleic acids with the cells under conditions that permit at least one of the plurality of nucleic acids to enter at least one of the cells; maintaining the cells under conditions permitting expression of the hybrid nucleic acids in the cells; and identifying a cell that expresses the reporter gene below the given level as an indication that the cell contains a hybrid nucleic acid encoding a test zinc finger domain that recognizes the target site. Additional embodiments of this method are as for the similar method utilizing a transcription activation domain. Likewise, any other selection method described herein can be performed using a transcriptional repression domain in place of a transcriptional activation domain.

In another aspect, the invention features certain purified polypeptides and isolated nucleic acids. A purified polypeptide of the invention can include a polypeptide having one or more of the following amino acid sequences:
Xₐ-X-Cys-X₂₋₅-Cys-X₃-Xₐ-X-Cys-X-Ser-Asn-X_{b}-X-Arg-His-X₃₋₅-His (SEQ ID NO:68),
Xₐ-X-Cys-X₂₋₅-Cys-X₃-Xₐ-X-His-X-Ser-Asn-X_{b}-X-Lys-His-X₃₋₅-His (SEQ ID NO:69),
Xₐ-X-Cys-X₂₋₅-Cys-X₃-Xₐ-X-Ser-X-Ser-Asn-X_{b}-X-Arg-His-X₃₋₅-His (SEQ ID NO:70),
Xₐ-X-Cys-X₂₋₅-Cys-X₃Xₐ-X-Gln-X-Ser-Thr-X_{b}-X-Val-His-X₃₋₅-His (SEQ ID NO:71),
Xₐ-X-Cys-X₂₋₅-Cys-X₃Xₐ-X-Val-X-Ser-X_{c}-X_{b}-X-Arg-His-X₃₋₅-His (SEQ ID NO:72),
Xₐ-X-Cys-X₂₋₅-Cys-X₃-Xₐ-X-Gln-X-Ser-His-X_{b}-X-Arg-His-X₃₋₅-His (SEQ ID NO:73),
Xₐ-X-Cys-X₂₋₅-Cys-X₃-Xₐ-X-Gln-X-Ser-Asn-X_{b}-X-Val-His-X₃₋₅-His (SEQ ID NO:74),
Xₐ-X-Cys-X₂₋₅-Cys-X₃-Xₐ-X-Gln-X-Ser-X_{c}-X_{b}-X-Arg-His-X₃₋₅-His (SEQ ID NO:75),
Xₐ-X-Cys-X₂₋₅-Cys-X₃-Xₐ-X-Gln-X-Ala-His-X_{b}-X-Arg-His-X₃₋₅-His (SEQ ID NO:150),
Xₐ-X-Cys-X₂₋₅-Cys-X₃-Xₐ-X-Gln-X-Phe-Asn-X_{b}-X-Arg-His-X₃₋₅-His (SEQ ID NO:151),
Xₐ-X-Cys-X₂₋₅-Cys-X₃-Xₐ-X-Gln-X-Ser-His-X_{b}-X-Thr-His-X₃₋₅-His (SEQ ID NO:152),
Xₐ-X-Cys-X₂₋₅-Cys-X₃-Xₐ-X-Gln-X-Ser-His-X_{b}-X-Val-His-X₃₋₅-His (SEQ ID NO:153),
Xₐ-X-Cys-X₂₋₅-Cys-X₃-Xₐ-X-Gln-X-Ser-Asn-X_{b}-X-Ile-His-X₃₋₅-His (SEQ ID NO:154),
Xₐ-X-Cys-X₂₋₅-Cys-X₃-Xₐ-X-Gln-X-Ser-Asn-X_{b}-X-Arg-His-X₃₋₅-His (SEQ ID NO:155),
Xₐ-X-Cys-X₂₋₅-Cys-X₃-Xₐ-X-Gln-X-Thr-His-X_{b}-X-Gln-His-X₃₋₅-His (SEQ ID NO:156),
Cys-X₂₋₅-Cys-X₃-Xₐ-X-Gln-X-Thr-His-X_{b}-X-Arg-His-X₃₋₅-His (SEQ ID NO:157),
Xₐ-X-Cys-X₂₋₅-Cys-X₃-Xₐ-X-Arg-X-Asp-Lys-X_{b}-X-Ile-His-X₃₋₅-His (SEQ ID NO:158),
Xₐ-X-Cys-X₂₋₅-Cys-X₃-Xₐ-X-Arg-X-Ser-Asn-X_{b}-X-Arg-His-X₃₋₅-His (SEQ ID NO:159),
Xₐ-X-Cys-X₂₋₅-Cys-X₃-Xₐ-X-Gln-X-Gly-Asn-X_{b}-X-Arg-His-X₃₋₅-His (SEQ ID NO:161),
Xₐ-X-Cys-X₂₋₅-Cys-X₃-Xₐ-X-Arg-X-Asp-Glu-X_{b}-X-Arg-His-X₃₋₅-His (SEQ ID NO:162),
Xₐ-X-Cys-X₂₋₅-Cys-X₃-Xₐ-X-Arg-X-Asp-His-X_{b}-X-Arg-His-X₃₋₅-His (SEQ ID NO:163),
Xₐ-X-Cys-X₂₋₅-Cys-X₃-Xₐ-X-Arg-X-Asp-His-X_{b}-X-Thr-His-X₃₋₅-His (SEQ ID NO:164),
Xₐ-X-Cys-X₂₋₅-Cys-X₃-Xₐ-X-Arg-X-Asp-Lys-X_{b}-X-Arg-His-X₃₋₅-His (SEQ ID NO:165),
Xₐ-X-Cys-X₂₋₅-Cys-X₃-Xₐ-X-Arg-X-Ser-His-X_{b}-X-Arg-His-X₃₋₅-His (SEQ ID NO:166),
Xₐ-X-Cys-X₂₋₅-Cys-X₃-Xₐ-X-Arg-X-Thr-Asn-X_{b}-X-Arg-His-X₃₋₅-His (SEQ ID NO:160),
Xₐ-X-Cys-X₂₋₅-Cys-X₃-Xₐ-X-His-X-Ser-Ser-X_{b}-X-Arg-His-X₃₋₅-His (SEQ ID NO:167),
Xₐ-X-Cys-X₂₋₅-Cys-X₃-Xₐ-X-Ile-X-Ser-Asn-X_{b}-X-Arg-His-X₃₋₅-His (SEQ ID NO:168),
Xₐ-X-Cys-X₂₋₅-Cys-X₃-Xₐ-X-Lys-X-Ser-Asn-X_{b}-X-Arg-His-X₃₋₅-His (SEQ ID NO:169),
Xₐ-X-Cys-X₂₋₅-Cys-X₃-Xₐ-X-Gln-X-Ser-Asn-X_{b}-X-Lys-His-X₃₋₅-His (SEQ ID NO:170),
Xₐ-X-Cys-X₂₋₅-Cys-X₃-Xₐ-X-Gln-X-Ser-His-X_{b}-X-Thr-His-X₃₋₅-His (SEQ ID NO:171),
Xₐ-X-Cys-X₂₋₅-Cys-X₃-Xₐ-X-Val-X-Ser-Asn-X_{b}-X-Val-His-X₃₋₅-His (SEQ ID NO:172),
Xₐ-X-Cys-X₂₋₅-Cys-X₃-Xₐ-X-Asp-X-Ser-Cys-X_{b}-X-Arg-His-X₃₋₅-His (SEQ ID NO:193),
Xₐ-X-Cys-X₂₋₅-Cys-X₃-Xₐ-X-Ile-X-Ser-Asn-X_{b}-X-Val-His-X₃₋₅-His (SEQ ID NO:194),
Xₐ-X-Cys-X₂₋₅-Cys-X₃-Xₐ-X-Trp-X-Ser-Asn-X_{b}-X-Arg-His-X₃₋₅-His (SEQ ID NO:195), or
Xₐ-X-Cys-X₂₋₅-Cys-X₃-Xₐ-X-Asp-X-Ser-Ala-X_{b}-X-Arg-His-X₃₋₅-His (SEQ ID NO:196),
wherein Xₐ is phenylalanine or tyrosine, X_{b} is a hydrophobic residue, and X_{c} is serine or threonine. Nucleic acids of the invention include nucleic acids encoding the aforementioned polypeptides. In one embodiment, the amino acid sequence listed above is a naturally occurring sequence.

In addition, purified polypeptides of the invention can have an amino acid sequence at least 50%, 60%, 70%, 80%, 90%, 93%, 95%, 96%, 98%, 99%, or 100% identical to SEQ ID NOs: 23, 25, 27, 29, 31, 33, 35, 37, 39, 41, 43, 45, 47, 49, 51, 53, 55, 57, 59, 61, 63, 65, 67, 103, 105, 107, 111, 113, 115, 117, 119, 121, 123, 125, 127, 129, 131, 133, 135, 137, 141, 143, 145, 147, 149, 173, 179, 183, 189, or 191. The polypeptides can be identical to SEQ ID NOs: 23, 25, 27, 29, 31, 33, 35, 37, 39, 41, 43, 45, 47, 49, 51, 53, 55, 57, 59, 61, 63, 65, 67, 103, 105, 107, 111, 113, 115, 117, 119, 121, 123, 125, 127, 129, 131, 133, 135, 137, 141, 143, 145, 147, 149, 173, 179, 183, 189, or 191 at the amino acid positions corresponding to the nucleic acid contacting residues of the polypeptide. Alternatively, the polypeptides differ from SEQ ID NOs: 23, 25, 27, 29, 31, 33, 35, 37, 39, 41, 43, 45, 47, 49, 51, 53, 55, 57, 59, 61, 63, 65, 67, 103, 105, 107, 111, 113, 115, 117, 119, 121, 123, 125, 127, 129, 131, 133, 135, 137, 141, 143, 145, 147, 149, 173, 179, 183, 189, or 191 at at least one of the residues corresponding to the nucleic acid contacting residues of the polypeptide. The polypeptides can also differ at at least one residue other than a DNA contacting residue (see below for further explanation). For example, within a given zinc finger domain, the polypeptide may differ by a single amino acid from the amino acid sequences referenced above, or by two, three, or four amino acids from the sequences referenced above. The difference may be due to a conservative substitution as defined herein. In one embodiment, the amino acids differences with respect to the sequences referenced above are located between the second zinc-coordinating cysteine and the -1 DNA contacting position (referring to the numbering system for DNA contacting positions described below). The comparison of sequences and determination of percent identity between two sequences can be accomplished using a mathematical algorithm. In particular, the percent identity between two amino acid sequences is determined using the Needleman and Wunsch ((1970) J. Mol. Biol. 48:444-453) algorithm which has been incorporated into the GAP program in the GCG software package, using a Blossum 62 scoring matrix with a gap penalty of 12, a gap extend penalty of 4, and a frameshift gap penalty of 5.

The purified polypeptides can also include one or more of the following: a heterologous DNA binding domain, a nuclear localization signal, a small molecular binding domain (e.g., a steroid binding domain), an epitope tag or purification handle, a catalytic domain (e.g., a nucleic acid modifying domain, a nucleic acid cleavage domain, or a DNA repair catalytic domain) and/or a transcriptional function domain (e.g., an activation domain, a repression domain, and so forth). In one embodiment, the polypeptide further include a second zinc finger domain, e.g., a domain having a sequence described herein. For example, the polypeptide can include an array of zinc fingers that include two or more zinc finger domains. Each domain can have a sequence selected from the group consisting of SEQ ID NOs:68-75, 150-172, and 193-196, or subsets thereof. Further, each domain can have a sequence selected from the group consisting of SEQ ID NOs: 23, 25, 27, 29, 31, 33, 35, 37, 39, 41, 43, 45, 47, 49, 51, 53, 55, 57, 59, 61, 63, 65, 67, 103, 105, 107, 111, 113, 115, 117, 119, 121, 123, 125, 127, 129, 131, 133, 135, 137, 141, 143, 145, 147, 149, 173, 179, 183, 189, and 191, and subsets thereof.

As described herein, the polypeptide can be produced in a cell and can regulate a gene in the cell, e.g., an endogenous gene, by binding to a target site, e.g., a site that includes a subsite that the respective zinc finger domain(s) recognizes. See, e.g, Tables 5, 6, and 7.

The invention also includes isolated nucleic acid sequences encoding the aforementioned polypeptides, and isolated nucleic acid sequences that hybridize under high astringency conditions to a single stranded probe, the sequence of the probe consisting of SEQ ID NO:22, 24, 26, 28, 30, 32, 34, 36, 38, 40, 42, 44, 46, 48, 50, 52, 54, 56, 58, 60, 62, 64, 66, 102, 104, 106, 110, 112, 114, 116, 118, 120, 122, 124, 126, 128, 130, 132, 134, 136, 140, 142, 144, 146, 148, 150, 174, 176, 178, 180, 182, 184, 186, 188, 190, 192 or the complements thereof. The invention further includes a method of expressing in a cell a polypeptide of the invention fused to a heterologous nucleic acid binding domain. The method includes introducing into a cell a nucleic acid encoding the aforementioned fusion protein. A nucleic acid of the invention can be operably regulated by a heterologous nucleic acid sequence, e.g., an inducible promoter (e.g., a steroid hormone regulated promoter, a small-molecule regulated promoter, or an engineered inducible system such as the tetracycline Tet-On and Tet-Off systems).

The term "base contacting positions," "DNA contacting positions," or "nucleic acid contacting positions" refers to the four amino acid positions of zinc finger domains that structurally correspond to the positions of amino acids arginine 73, aspartic acid 75, glutamic acid 76, and arginine 79 of SEQ ID NO:21. These positions are also referred to as positions -1, 2, 3, and 6, respectively. To identify positions in a query sequence that correspond to the base contacting positions, the query sequence is aligned to the zinc finger domain of interest such that the cysteine and histidine residues of the query sequence are aligned with those of finger 3 of If268. The ClustalW WWW Service at the European Bioinformatics Institute (Thompson et al. (1994) Nucleic Acids Res. 22:4673-4680) provides one convenient method of aligning sequences.

Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; a group of amino acids having acidic side chains is aspartic acid and glutamic acid; and a group of amino acids having sulfur-containing side chains is cysteine and methionine. Depending on circumstances, amino acids within the same group may be interchangeable. Some additional conservative amino acids substitution groups are: valine-leucine-isoleucine; phenylalanine-tyrosine; lysine-arginine; alanine-valine; aspartic acid-glutamic acid; and asparagine-glutamine.

The term "heterologous polypeptide" refers either to a polypeptide with a non-naturally occurring sequence (e.g., a hybrid polypeptide) or a polypeptide with a sequence identical to a naturally occurring polypeptide but present in a milieu in which it does not naturally occur.

The term "hybrid" refers to a non-naturally occurring polypeptide that comprises amino acid sequences derived from either (i) at least two different naturally occurring sequences; (ii) at least one artificial sequence (i.e., a sequence that does not occur naturally) and at least one naturally occurring sequence; or (iii) at least two artificial sequences (same or different). Examples of artificial sequences include mutants of a naturally occurring sequence and *de novo* designed sequences.

As used herein, the term "hybridizes under stringent conditions" refers to conditions for hybridization in 6X sodium chloride/sodium citrate (SSC) at 45°C, followed by two washes in 0.2 X SSC, 0.1% SDS at 65°C.

The term "binding preference" refers to the discriminative property of a polypeptide for selecting one nucleic acid binding site relative to another. For example, when the polypeptide is limiting in quantity relative to two different nucleic acid binding sites, a greater amount of the polypeptide will bind the preferred site relative to the other site in an *in vivo* or *in vitro* assay described herein.

As used herein, "degenerate oligonucleotides" refers to both (a) a population of different oligonucleotides that each encode a particular amino acid sequence, and (b) a single species of oligonucleotide that can anneal to more than one sequence, e.g., an oligonucleotide with an unnatural nucleotide such as inosine.

The present invention provides numerous benefits. The ability to select a DNA binding domain that recognizes a particular sequence permits the design of novel polypeptides that bind to specific site on a DNA. Thus, the invention facilitates the customized generation of novel polypeptides that can regulate the expression of a selected target, e.g., a gene required by a pathogen can be repressed, a gene required for cancerous growth can be repressed, a gene poorly expressed or encoding an unstable protein can be activated and overexpressed, and so forth.

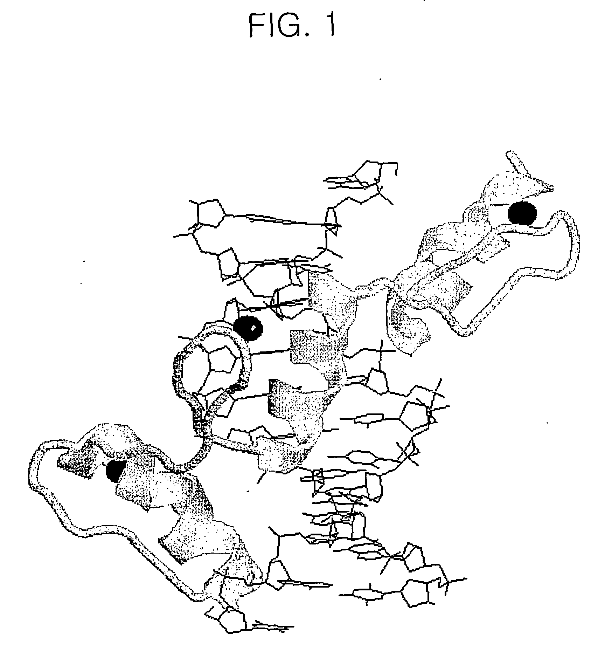

The use of zinc finger domains is particularly advantageous. First, the zinc finger motif recognizes very diverse DNA sequences. Second, the structure of naturally occurring zinc finger proteins is modular. For example, the zinc finger protein Zif268, also called "Egr-1," is composed of a tandem array of three zinc finger domains. Fig. 1 is the x-ray crystallographic structure of zinc finger protein Zif268, consisting of three fingers complexed with DNA (Pavletich and Pabo, (1991) Science 252:809-817). Each finger independently contacts 3-4 basepairs of the DNA recognition site. High affinity binding is achieved by the cooperative effect of having multiple zinc finger modules in the same polypeptide chain.

It is frequently the ultimate goal to obtain a DNA binding polypeptide that functions within a cell. Advantageously, the *in vivo* selection method identifies up-front DNA binding polypeptides that are functional at a specific DNA site within a cell. The factors associated with recognition in a cell, particularly a eukaryotic cell, can be vastly different from the factors present during an *in vitro* selection scenario. For example, in a eukaryotic nucleus, a polypeptide must compete with the myriad other nuclear proteins for a specific nucleic acid binding site. A nucleosome or another chromatin protein can occupy, occlude, or compete for the binding site. Even if unbound, the conformation of a nucleic acid in the cell is subject to bending, supercoiling, torsion, and unwinding. The polypeptide itself is exposed to proteases and chaperones, among other factors. Moreover, the polypeptide is confronted with an entire genome of possible binding sites, and hence must be endowed with a high specificity for the desired site in order to survive the selection process. In contrast to *in vivo* selection, an *in vitro* selection can select for the highest affinity binder rather than the highest specificity binder.

The use of a reporter gene to indicate the binding ability of an expressed polypeptide chimera not only is efficient and simple, but also obviates the need to develop a complex interaction code that accounts for the energetics of the protein-nucleic acid interface and the immense number of peripheral factors, such as surrounding residues and nucleotides that also affect the binding interface. (Segal et al. (1999) Proc. Natl. Acad. Sci. USA 96:2758-2763).

The present invention avails itself of all the zinc finger domains present in the human genome, or any other genome. This diverse sampling of sequence space occupied by the zinc finger domain structural fold may have the additional advantages inherent in eons of natural selection. Moreover, by utilizing domains from the host species, a DNA binding protein engineered for a gene therapy application by the methods described herein has a reduced likelihood of being regarded as foreign by the host immune response.

A DNA binding protein identified by a method described herein can be used in a variety of applications. For example, the DNA binding protein can be used to alter the expression of an endogenous gene in a cultured cell or in a cell in a host organism. The DNA binding protein can be used to alter the phenotype of a cell, e.g., enhanced sensitivity or resistance to a condition (e.g., stress), altered proliferative ability, altered pathogenicity, and altered product production (e.g., metabolite production).

All patents, patent applications, and references cited herein are incorporated by reference in their entirety for all purposes. The details of one or more embodiments of the invention are set forth in the accompanying drawings and the description below. Other features, objects, and advantages of the invention will be apparent from the description and drawings, and from the claims.

### BRIEF DESCRIPTION OF THE DRAWINGS

Fig. 1 is a depiction of the three dimensional structure of the Zif268 zinc finger protein that consists of three finger domains and binds the DNA sequence, 5'-GCG TGG GCG T-3' (SEQ ID NO:197). The black circles represent the location of the zinc ion.
Fig. 2 is an illustration of the hydrogen-bonding interactions between amino acid residues of Zif268 and DNA bases. Amino acid residues at positions - 1, 2, 3, and 6 along the α-helix interact with the bases at specific positions. The bold lines represent ideal hydrogen bonding, while the dotted lines represent potential hydrogen bonding.
Fig. 3 is a recognition code table that summarizes the interactions between DNA bases and amino acid residues at positions -1, 2, 3, and 6 along the α-helix of a zinc finger domain.

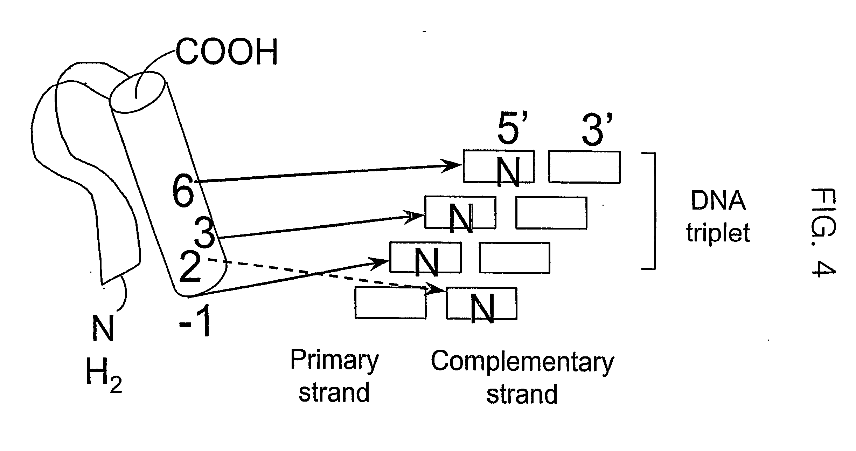

Fig. 4 is a depiction of the positions of amino acid residues and their corresponding 3 base triplets. The bold lines represent the main interactions observed, while the dotted line represents an auxiliary interaction.
Fig. 5 is a diagram illustrating the principles of the *in vivo* selection system disclosed herein. Of the various zinc finger mutants, zinc finger domain A recognizes the target sequence (designated XXX X) and activates the transcription of *HIS3* reporter gene. As a result, yeast colonies grow on a medium lacking histidine. In contrast, zinc finger domain B does not recognize the target sequence and thus the reporter gene remains repressed. As a result, no colonies grow on a medium lacking histidine. AD represents the transcriptional activation domain.

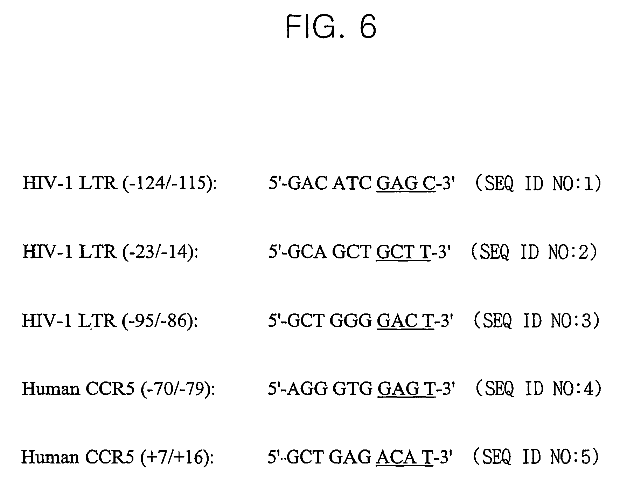

Fig. 6 is a list of 10-bp sequences (SEQ ID NOs:1-5, respectively) found in long terminal repeats (LTR) of HIV-1 and in the promoter region of CCR5, a human gene encoding a coreceptor for HIV-1. The underlined portions represent 4-bp target sequences used in the present selection.
Fig. 7 is a depiction of the base sequences of the binding sites linked to the reporter gene (SEQ ID NOs:6-17, respectively). Each binding site consists of a tandem array of 4 composite binding sequences. Each composite binding sequence was constructed by connecting truncated binding sequence 5'-GG GCG-3' recognized by finger 1 and finger 2 of Zif268 to 4-bp target sequences.
Fig 8 is a diagram of pPCFMS-Zif, a plasmid that can be used for the construction of a library of hybrid plasmids (SEQ ID NOs:18 and 19).
Fig 9 is a representation of the base sequence for the gene coding for Zif268 zinc finger protein inserted into pPCFMS-Zif and the corresponding translated amino acid sequences (SEQ ID NOs:20 and 21, respectively). Sites recognized by restriction enzymes are underlined.
Fig. 10 is a photograph of a culture plate having yeast cells obtained from retransformation and cross transformation using zinc finger proteins selected by the *in vivo* selection system.
Fig. 11A is a listing of the nucleotide sequence of polylinker region of P3 (SEQ ID NO:251). The sequence outside of this region is identical to that of the parental vector, pcDNA3 (Invitrogen). Each enzyme site is italicized and HA tag is underlined. Both initiation and stop codons are indicated by bold letters. The nuclear localization signal (NLS) is also indicated.
Fig. 11B is a schematic of one exemplary method for zinc finger protein library construction.
Fig. 12 is a schematic of reporter constructs and segments of their sequence in the initiator region. 5XGa14, TATA and Inr indicate: five GAL4 binding sites, the TATA box and the transcriptional initiator, respectively. NNNNNNNNN indicates the site for the cognate binding site for a specific ZFP. The positions are numbered with respect to the transcription start point (+1) and identical nucleotides are indicated by "-". ">" represents a deletion of a corresponding nucleotide.

### DETAILED DESCRIPTION OF THE INVENTION

The invention features a novel screening method for determining the nucleic acid binding preferences of test zinc finger domains. The method is easily adapted to a variety of protein domains, a variety of sources for these domains, and a number of library designs, reporter genes, and selection and screening systems. The screening method can be implemented as a high-throughput platform. Information obtained from the screening method is readily applied to a method of designing artificial nucleic acid binding proteins, typically DNA binding proteins, but also in some cases RNA binding proteins or even proteins that interact with other proteins. The design method appropriates the binding preferences of test zinc finger domains to guide the modular assembly of a chimeric nucleic acid binding protein. A designed protein can be further optimized or varied with the screening method.

### DNA binding domains

The invention utilizes collections of nucleic acid binding domains with differing binding specificities. A variety of protein structures are known to bind nucleic acids with high affinity and high specificity. These structures are used repeatedly in a myriad of different proteins to specifically control nucleic acid function (for reviews of structural motifs which recognize double stranded DNA, see, e.g., Pabo and Sauer (1992) Annu. Rev. Biochem. 61:1053-95; Patikoglou and Burley (1997) Annu. Rev. Biophys. Biomol. Struct. 26:289-325; Nelson (1995) Curr Opin Genet Dev. 5:180-9). A few non-limiting examples of nucleic acid binding domains include:

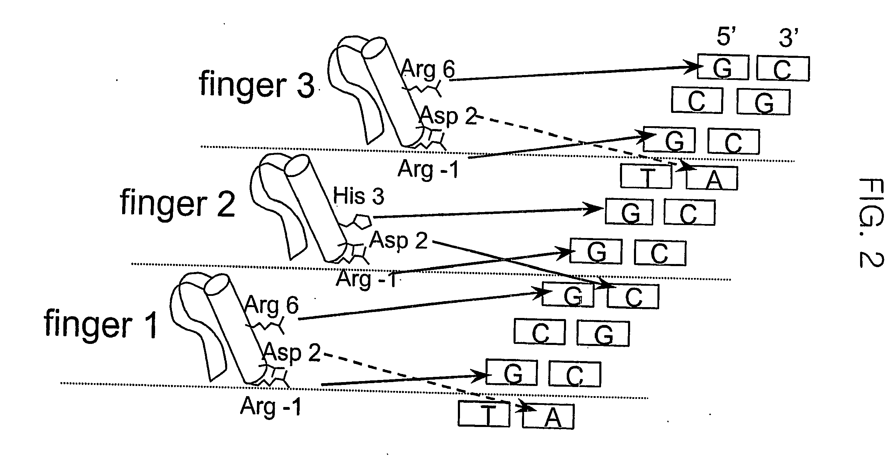

**Zinc fingers**. Zinc fingers are small polypeptide domains of approximately 30 amino acid residues in which there are four residues, either cysteine or histidine, appropriately spaced such that they can coordinate a zinc ion (Fig. 1; for reviews, see, e.g., Klug and Rhodes, (1987) Trends Biochem. Sci.12:464-469(1987); Evans and Hollenberg, (1988) Cell 52:1-3; Payre and Vincent, (1988) FEBS Lett. 234:245-250; Miller et al., (1985) EMBO J. 4:1609-1614; Berg, (1988) Proc. Natl. Acad. Sci. U.S.A. 85:99-102; Rosenfeld and Margalit, (1993) J. Biomol. Struct. Dyn. 11:557-570). Hence, zinc finger domains can be categorized according to the identity of the residues that coordinate the zinc ion, e.g., as the Cys₂-His₂ class, the Cys₂-Cys₂ class, the Cys₂-CysHis class, and so forth. The zinc coordinating residues of Cys₂-His₂ zinc fingers are typically spaced as follows:
Xₐ-X-C-X₂₋₅-C-X₃-Xₐ-X₅-ψ-X₂-H-X₃₋₅-H,
where ψ (psi) is a hydrophobic residue (Wolfe et al., (1999) Annu. Rev. Biophys. Biomol. Struct. 3:183-212) (SEQ ID NO:76), "X" represents any amino acid, Xₐ is phenylalanine or tyrosine, the subscript number indicates the number of amino acids, and a subscript with two hyphenated numbers indicates a typical range of intervening amino acids. Typically, the intervening amino acids fold to form an anti-parallel β-sheet that packs against an α-helix, although the anti-parallel β-sheets can be short, non-ideal, or non-existent. The fold positions the zinc-coordinating side chains so they are in a tetrahedral conformation appropriate for coordinating the zinc ion. The base contacting residues are at the N-terminus of the finger and in the preceding loop region (Fig. 2).

For convenience, the primary DNA contacting residues of a zinc finger domain are numbered: -1, 2, 3, and 6 based on the following example:

As noted in the example above, the DNA contacting residues are Cys (C); Ser (S), Asn (N), and Arg (R). The above motif can be abbreviated CSNR. As used herein, such abbreviation is a shorthand that refers to a particular polypeptide sequence from the second residue preceding the first cysteine (Xₐ, above, initial residue of SEQ ID NO:68) to the ultimate metal-chelating histidine (ultimate residue of SEQ ID NO:68). Where two different sequences have the same motif, a number may be used to indicate each sequence (e.g., CSNR1, CSNR2). In certain contexts where made explicitly apparent, the four letter abbreviation refers to the motif in general.

A zinc finger DNA-binding protein may consist of a tandem array of three or more zinc finger domains.

The zinc finger domain (or "ZFD") is one of the most common eukaryotic DNA-binding motifs, found in species from yeast to higher plants and to humans: By one estimate, there are at least several thousand zinc finger domains in the human genome alone, possibly at least 4,500. Zinc finger domains can be identified in or isolated from zinc finger proteins. Non-limiting examples of zinc finger proteins include CF2-II; Kruppel; WT1; basonuclin; BCL-6/LAZ-3; erythroid Kruppel-like transcription factor; transcription factors Sp1, Sp2, Sp3, and Sp4; transcriptional repressor YY1; EGR1/Krox24; EGR2/Krox20; EGR3/Pilot; EGR4/AT133; Evi-1; GLI1; GLI2; GLI3; HIV-EP1/ZNF40; HIV-EP2; KR1; ZfX; ZfY; and ZNF7.

Computational methods described below can be used to identify all zinc finger domains encoded in a sequenced genome or in a nucleic acid database. Any such zinc finger domain can be utilized. In addition, artificial zinc finger domains have been designed, e.g., using computational methods (e.g., Dahiyat and Mayo, (1997) Science 278:82-7).

Although many zinc finger domains bind to DNA sites, a number of zinc finger domains can bind other ligands, e.g., RNA sites and other proteins. In some implementations, a chimeric zinc finger domain protein is engineered to bind to a non-DNA ligand, e.g., a target protein or a target RNA site. The target RNA site can be a site on a ncRNA, e.g., a naturally occurring ncRNA.

**Homeodomains.** Homeodomains are simple eukaryotic domains that consist of a N-terminal arm that contacts the DNA minor groove, followed by three α-helices that contact the major groove (for a review, see, e.g., Laughon, (1991) Biochemistry 30:11357-67). The third α-helix is positioned in the major groove and contains critical DNA-contacting side chains. Homeodomains have a characteristic highly-conserved motif present at the turn leading into the third α-helix. The motif includes an invariant tryptophan that packs into the hydrophobic core of the domain. This motif is represented in the Prosite database (see Falquet et al. (2002) Nucleic Acids Res. 30:235-238) as PDOC00027 ([L/I/V/M/F/Y/G]-[A/S/L/V/R]-X(2)-[L/I/V/M/S/T/A/C/N]-X-[L/I/V/M]-X(4)-[L/I/V] [R/K/N/Q/E/S/T/A/L/Y]-[L/I/V/F/S/T/N/K/H]-W-[F/Y/V/C]-X-[N/D/Q/T/A/H]-X(5)- [R/K/N/A/I/M/W]; SEQ ID NO:77). Homeodomains are commonly found in transcription factors that determine cell identity and provide positional information during organismal development. Such classical homeodomains can be found in the genome in clusters such that the order of the homeodomains in the cluster approximately corresponds to their expression pattern along a body axis. Homeodomains can be identified by alignment with a homeodomain, e.g., Hox-1, or by alignment with a homeodomain profile or a homeodomain hidden Markov Model (HMM; see below), e.g., PF00046 of the Pfam database or "HOX" of the SMART database, or by the Prosite motif PDOC00027 as mentioned above.

**Helix-turn-helix proteins**. This DNA binding motif is common among many prokaryotic transcription factors. There are many subfamilies, e.g., the LacI family, the AraC family, to name but a few. The two helices in the name refer to a first α-helix that packs against and positions a second α-helix in the major groove of DNA. These domains can be identified by alignment with a HMM, e.g., HTH_ARAC, HTH_ARSR, HTH_ASNC, HTH_CRP, HTH_DEOR, HTH_DTXR, HTH_GNTR, HTH_ICLR, HTH_LACI, HTH_LUXR, HTH_MARR, HTH_MERR, and HTH_XRE profiles available in the SMART database.

**Helix-loop-helix proteins**. This DNA binding domain is commonly found among homo- and hetero-dimeric transcription factors, e.g., MyoD, fos, jun, E11, and myogenin. The domain consists of a dimer, each monomer contributing two α-helices and intervening loop. The domain can be identified by alignment with a HMM, e.g., the "HLH" profile available in the SMART database. Although helix-loop-helix proteins are typically dimeric, monomeric versions can be constructed by engineering a polypeptide linker between the two subunits such that a single open reading frame encodes both the two subunits and the linker.

### Identification of DNA-binding domains

A variety of methods can be used to identify structural domains.

**Computational Methods.** The amino acid sequence of a DNA binding domain isolated by a method described herein can be compared to a database of known sequences, e.g., an annotated database of protein sequences or an annotated database which includes entries for nucleic acid binding domains. In another implementation, databases of uncharacterized sequences, e.g., unannotated genomic, EST or full-length cDNA sequence; of characterized sequences, e.g., SwissProt or PDB; and of domains, e.g., Pfam, ProDom (Corpet et al. (2000) Nucleic Acids Res. 28:267-269), and SMART (Simple Modular Architecture Research Tool, Letunic et al. (2002) Nucleic Acids Res 30, 242-244) can provide a source of nucleic acid binding domain sequences. Nucleic acid sequence databases can be translated in all six reading frames for the purpose of comparison to a query amino acid sequence. Nucleic acid sequences that are flagged as encoding candidate nucleic acid binding domains can be amplified from an appropriate nucleic acid source, e.g., genomic DNA or cellular RNA. Such nucleic acid sequences can be cloned into an expression vector. The procedures for computer-based domain identification can be interfaced with an oligonucleotide synthesizer and robotic systems to produce nucleic acids encoding the domains in a high-throughput platform. Cloned nucleic acids encoding the candidate domains can also be stored in a host expression vector and shuttled easily into an expression vector, e.g., into a translational fusion vector with Zif268 fingers 1 and 2, either by restriction enzyme-mediated subcloning or by site-specific recombinase-mediated subcloning (see U.S. Patent No. 5,888,732). The high-throughput platform can be used to generate multiple microtitre plates containing nucleic acids encoding different candidate nucleic acid binding domains.

Detailed methods for the identification of domains from a starting sequence or a profile are well known in the art. See, for example, Prosite (Hofmann et al., (1999) Nucleic Acids Res. 27:215-219), FASTA, BLAST (Altschul et al., (1990) J. Mol. Biol. 215:403-10.), etc. A simple string search can be done to find amino acid sequences with identity to a query sequence or a query profile, e.g., using Perl to scan text files. Sequences so identified can be at least about 30%, 40%, 50%, 60%, 70%, 80%, 90%, or greater identical to an initial input sequence.

Domains similar to a query domain can be identified from a public database, e.g., using the XBLAST programs (version 2.0) of Altschul et al., (1990) J. Mol. Biol. 215:403-10. For example, BLAST protein searches can be performed with the XBLAST parameters as follows: score = 50, wordlength = 3. Gaps can be introduced into the query or searched sequence as described in Altschul et al., (1997) Nucleic Acids Res. 25(17):3389-3402. Default parameters for XBLAST and Gapped BLAST programs are available at National Center for Biotechnology Information (NCBI), National Institutes of Health, Bethesda MD.

The Prosite profiles PS00028 and PS50157 can be used to identify zinc finger domains. In a SWISSPROT release of 80,000 protein sequences, these profiles detected 3189 and 2316 zinc finger domains, respectively. Profiles can be constructed from a multiple sequence alignment of related proteins by a variety of different techniques. Gribskov and co-workers (Gribskov et al., (1990) Meth. Enzymol. 183:146-159) utilized a symbol comparison table to convert a multiple sequence alignment supplied with residue frequency distributions into weights for each position. See, for example, the PROSITE database and the work of Luethy et al., (1994) Protein Sci. 3:139-1465.

Hidden Markov Models (HMM's) representing a DNA binding domain of interest can be generated or obtained from a database of such models, e.g., the Pfam database, release 2.1. A database can be searched, e.g., using the default parameters, with the HMM in order to find additional domains (see, e.g., Bateman et al. (2002) Nucleic Acids Research 30:276-280). Alternatively, the user can optimize the parameters. A threshold score can be selected to filter the database of sequences such that sequences that score above the threshold are displayed as candidate domains. A description of the Pfam database can be found in Sonhammer et al., (1997) Proteins 28(3):405-420, and a detailed description of HMMs can be found, for example, in Gribskov et al., (1990) Meth. Enzymol. 183:146-159; Gribskov et al., (1987) Proc. Natl. Acad. Sci. USA 84:4355-4358; Krogh et al., (1994) J. Mol. Biol. 235:1501-1531; and Stultz et al., (1993) Protein Sci. 2:305-314.

The SMART database of HMM's (Simple Modular Architecture Research Tool, Schultz et al., (1998) Proc. Natl. Acad. Sci. USA 95:5857 and Schultz et al., (2000) Nucl. Acids Res 28:231) provides a catalog of zinc finger domains (ZnF_C2H2; ZnF_C2C2; ZnF_C2HC; ZnF_C3H1; ZnF_C4; ZnF_CHCC; ZnF_GATA; and ZnF_NFX) identified by profiling with the hidden Markov models of the HMMer2 search program (Durbin et al., (1998) Biological sequence analysis: probabilistic models of proteins and nucleic acids. Cambridge University Press).

**Hybridization-based Methods**. A collection of nucleic acids encoding various forms of a DNA binding domain can be analyzed to profile sequences encoding conserved amino- and carboxy-terminal boundary sequences. Degenerate oligonucleotides can be designed to hybridize to sequences encoding such conserved boundary sequences. Moreover, the efficacy of such degenerate oligonucleotides can be estimated by comparing their composition to the frequency of possible annealing sites in known genomic sequences. Multiple rounds of design can be used to optimize the degenerate oligonucleotides. For example, comparison of known Cys₂His₂ zinc fingers revealed a common sequence in the linker region between adjacent fingers in natural sequence (Agata et al., (1998) Gene 213:55-64). Such degenerate oligonucleotides are used to amplify a plurality of DNA binding domains. The amplified domains are inserted as test zinc finger domains into the hybrid nucleic acid, and subsequently assayed for binding to a target site by the methods described herein.

### Collections of Nucleic Acid Binding Domains

The method permits the screening of a collection of nucleic acids encoding DNA binding domains (for example, in the form of a plasmid, phagemid, or phage library) for functional nucleic acid binding properties. The collection can encode a diverse group of DNA binding domains, even domains of different structural folds. In one instance, the collection encodes domains of a single structural fold such as a zinc finger domain. Although the following methods are described in the context of zinc finger domains, one skilled in the art would be able to adapt them to other types of nucleic acid binding domains.

**Mutated Domains**. In still another instance, the collection is composed of nucleic acids encoding a structural domain that is assembled from a degenerate patterned library. For example, in the instance of zinc fingers, an alignment of known zinc fingers can be utilized to identify the optimal amino acids at each position. Alternatively, structural studies and mutagenesis experiments can be used to determine the preferred properties of amino acids at each position. Any nucleic acid binding domain can be used as a structural scaffold for introducing mutations. In particular, positions in close proximity to the nucleic acid binding interface or adjacent to a position so located can be targeted for mutagenesis. A mutated test zinc finger domain can be constrained at any mutated position to a subset of possible amino acids by using a patterned degenerate library. Degenerate codon sets can be used to encode the profile at each position. For example, codon sets are available that encode only hydrophobic residues, aliphatic residues, or hydrophilic residues. The library can be selected for full-length clones that encode folded polypeptides. Cho et al. ((2000) J. Mol. Biol. 297(2):309-19) provides a method for producing such degenerate libraries using degenerate oligonucleotides, and also provides a method of selecting library nucleic acids that encode full-length polypeptides. Such nucleic acids can be easily inserted into an expression plasmid using convenient restriction enzyme cleavage sites or transposase or recombinase recognition sites for the selection methods described herein.

Selection of the appropriate codons and the relative proportions of each nucleotide at a given position can be determined by simple examination of a table representing the genetic code, or by computational algorithms. For example, Cho *et al., supra,* describe a computer program that accepts a desired profile of protein sequences and outputs a preferred oligonucleotide design that encodes sequences of the profile. For example, the design may include degenerate positions for an oligonucleotide population.

**Isolation of a natural repertoire of domains**. A library of domains can be constructed from genomic DNA or cDNA of eukaryotic organisms such as humans. Multiple methods are available for doing this. For example, a computer search of available amino acid sequences can be used to identify the domains, as described above. A nucleic acid encoding each domain can be isolated and inserted into a vector appropriate for the expression in cells, e.g., a vector containing a promoter, an activation domain, and a selectable marker. In another example, degenerate oligonucleotides that hybridize to sequences encoding a conserved motif are used to amplify, e.g., by PCR, a large number of related domains containing the motif. For example, Kruppel-like Cys₂His₂ zinc fingers can be amplified by the method of Agata et al., (1998) Gene 213:55-64. This method also maintains the naturally occurring zinc finger domain linker peptide sequences, e.g., sequences with the pattern: Thr-Gly-(Glu/Gln)-(Lys/Arg)-Pro-(Tyr/Phe) (SEQ ID NO:78). Moreover, screening a collection limited to domains of interest, unlike screening a library of unselected genomic or cDNA sequences, significantly decreases library complexity and reduces the likelihood of missing a desirable sequence due to the inherent difficulty of completely screening large libraries.

The human genome contains numerous zinc finger domains, many of which are uncharacterized and unidentified. It is estimated that there are thousands of genes encoding proteins with zinc finger domains (Pellegrino and Berg, (1991) Proc. Natl. Acad. Sci. USA 88:671-675). These human zinc finger domains represent an extensive collection of diverse domains from which novel DNA-binding proteins can be constructed. If each zinc finger domain recognizes a unique 3- to 4-bp sequence, the total number of domains required to bind every possible 3- to 4-bp sequence is only 64 to 256 (4³ to 4⁴). It is possible that the natural repertoire of the human genome contains a sufficient number of unique zinc finger domains to span all possible recognition sites. These zinc finger domains are a valuable resource for constructing artificial chimeric DNA-binding proteins. Naturally occurring zinc finger domains, unlike artificial mutants derived from the human genome, have evolved under natural selective pressures and therefore may be naturally optimized for binding specific DNA sequences and *in vivo* function.

Human zinc finger domains are much less likely to induce an immune response when introduced into humans, e.g., in gene therapy applications.

### In vivo Selection of Zinc Finger Domains Possessing Specific DNA Binding Properties

Zinc finger domains with desired DNA recognition properties can be identified using the following *in vivo* screening system. A composite binding site of interest is inserted upstream of a reporter gene such that recruitment of a transcriptional activation domain to the composite binding site results in increased reporter gene transcription above a given level. An expression plasmid that encodes a hybrid protein consisting of a test zinc finger domain fused to a fixed DNA binding domain and a transcriptional activation domain is constructed.

The composite binding site includes at least two elements, a recruitment site and a target site. The system is engineered such that the fixed DNA binding domain recognizes the recruitment site. However, the binding affinity of the fixed DNA binding domain for the recruitment site is such that *in vivo* it alone is insufficient for transcriptional activation of the reporter gene. This can be verified by a control experiment.

For example, when expressed in cells, the fixed DNA binding domain (in the absence of a test zinc finger domain, or in the presence of a test zinc finger domain that is known to be nonfunctional or whose known DNA contacting residues have been replaced with an alternative amino acid such as alanine) should not be able to activate transcription of the reporter gene above a nominal level. Some leaky or low-level activation is tolerable, as the system can be sensitized by other means (e.g., by use of a competitive inhibitor of the reporter). The fixed DNA binding domain is expected not to bind stably to the recruitment site. For example, the fixed DNA binding domain can bind to the recruitment site with a dissociation constant (K_{d}) of at least approximately 0.1 nM, 1 nM, 1 µM, 10 µM, 100 µM, or greater. (In addition, the K_{d} can be less than 100 µM, or 10 µM). The K_{d} of the DNA binding domain for the target site can be measured *in vitro* by an electrophoretic mobility shift assay (EMSA) in the absence of a test zinc finger domain or in the absence of a test zinc finger domain with specificity for the second target site.

Thus, attachment of a functional test zinc finger domain that recognizes the target site, e.g., the variable site of the composite binding site, is necessary for the hybrid protein to bind stably to the composite binding site in cells, and thereby to activate the reporter gene. The binding preference of the test zinc finger domain for the target site results in an increase in reporter gene expression relative to the given level. For example, the fold increase of reporter gene expression obtained by dividing the observed level by the given level can be at least approximately 2, 4, 8, 20, 50, 100, 1000 fold or greater. When the test zinc finger domain recognizes the target site, the K_{d} of the transcription factor comprising the DNA binding domain and the test zinc finger domain is decreased, e.g., relative to a transcription factor lacking a test zinc finger domain with specificity for the target site. For example, the dissociation constant (K_{d}) of a transcription factor complexed to a target site for which it has specificity can be at or below approximately 50 nM, 10 nM, 1 nM, 0.1 nM, 0.01 nM or less. The K_{d} can be determined *in vitro* by EMSA.

The discovery that DNA binding specificity can be sensitively and accurately assayed by determining the ability of test zinc finger domains to augment the *in vivo* binding affinity of a fixed DNA binding domain has enabled the rapid isolation and characterization of novel zinc finger domains from the human genome.

Fixed DNA binding domains include modular domains isolated from naturally occurring DNA-binding proteins, e.g., a naturally occurring DNA-binding protein that has multiple domains or that is an oligomer: For example, an amino acid sequence that includes two known zinc fingers, e.g., fingers 1 and 2 of Zif268, can be used as the fixed DNA binding domain. A skilled artisan would be able to identify from the myriad of nucleic acid binding domains (e.g., a domain family described herein, such as a homeodomain, a helix-turn-helix domain, or a helix-loop-helix domain, or a nucleic acid binding domain well characterized in the art) a fixed DNA binding domain suitable for the system. Appropriate selection of a recruitment site that is recognized by the fixed DNA binding domain is also necessary. The recruitment site can be a subsite within the natural binding site for the naturally occurring DNA binding protein from which the fixed DNA binding domain is obtained. If necessary, mutations can be introduced either into the fixed domain or into the recruitment site, in order to sensitize the system.

Cells suitable for the *in vivo* screening system include both eukaryotic and prokaryotic cells. Exemplary eukaryotic cells include yeast cells, e.g., *Saccharomyces cerevisiae, Saccharomyces pombe,* and *Pichia pastoris* cells.

The yeast one-hybrid system, using *Saccharomyces cerevisiae,* was modified to select zinc finger domains using the aforementioned screening system. First, reporter plasmids that encode the *HIS3* reporter gene were prepared. The predetermined 4-bp target DNA sequences were connected to a truncated binding sequence to provide composite binding sequences for the DNA-binding domains, and each of the composite binding sequences was operably linked to the reporter gene on separate plasmids.

The hybrid nucleic acid sequence encodes a transcriptional activation domain linked to a DNA-binding domain comprising a truncated DNA-binding domain and a zinc finger domain.

The binding sites used herein are not necessarily contiguous, although contiguous sites are frequently used. Flexible and/or extensible linkers between nucleic acid binding domains can be used to construct proteins that recognize non-contiguous sites.

According to one aspect of the present invention, a polypeptide composed of finger 1 and finger 2 of Zif268 and devoid of finger 3 can be used as a fixed DNA-binding domain. (Among the three zinc finger domains of Zif268, finger 1 refers to the zinc finger domain located at the N-terminal end, finger 2, the zinc finger domain in the middle, and finger 3 the zinc finger domain at the C-terminal end.) Alternately, any two zinc finger domains whose binding site is characterized can be used as a fixed DNA-binding domain. Many novel examples are disclosed herein.

Other useful fixed DNA-binding domains may be derived from other zinc finger proteins, such as Sp1, CF2-II, YY1, Kruppel, WT1, Egr2, or POU-domain proteins, such as Oct1, Oct2, and Pit1. These are provided by way of example and the present invention is not limited thereto.

According to one particular example of the present invention, the base sequence of 5'-GGGCG-3', generated by deleting 4-bp from the 5' end of the optimal Zif268 recognition sequence (5'-GCG TGG GCG-3'), can be used as a recruitment site. Any target sequence of 3 to 4 bp can be linked to this recruitment site, to yield a composite binding sequence.

**Activation domains**. Transcriptional activation domains that may be used in the present invention include but are not limited to the Gal4 activation domain from yeast and the VP16 domain from herpes simplex virus. In bacteria, activation domain function can be emulated by fusing a domain that can recruit a wild-type RNA polymerase alpha subunit C-terminal domain or a mutant alpha subunit C-terminal domain, e.g., a C-terminal domain fused to a protein interaction domain.

**Repression domains.** If desired, a repression domain instead of an activation domain can be fused to the DNA binding domain. Examples of eukaryotic repression domains include ORANGE, groucho, and WRPW (Dawson et al., (1995) Mol. Cell Biol. 15:6923-31). When a repression domain is used, a toxic reporter gene and/or a non-selectable marker can be used to screen for decreased expression.

**Other Functional Domains.** A protein transduction domain can be fused to the DNA binding domain, e.g., of the chimeric zinc finger protein. Protein transduction domains result in uptake of the transduction domain and attached polypeptide into cells. One example of a protein transduction domain is the HIV tat protein.

**Reporter genes**. The reporter gene can be a selectable marker, e.g., a gene that confers drug resistance or an auxotrophic marker. Examples of drug resistance genes include *S*. *cerevisiae* cyclohexamide resistance *(CYH), S. cerevisiae* canavanine resistance gene *(CAN1),* and the hygromycin resistance gene. *S. cerevisiae* auxotrophic markers include the *URA3, HIS3, LEU2, ADE2* and *TRP1* genes. When an auxotrophic marker is the reporter gene, cells that lack a functional copy of the auxotrophic gene and so the ability to produce a particular metabolite are utilized. Selection for constructs encoding test zinc finger domains that bind a target site is achieved by maintaining the cells in medium lacking the metabolite. For example, the *HIS3* gene can be used as a selectable marker in combination with a *his3⁻* yeast strain. After introduction of constructs encoding the hybrid transcription factors, the cells are grown in the absence of histidine. Selectable markers for use in mammalian cells, such as thymidine kinase, neomycin resistance, and HPRT, are also well known to the skilled artisan.

Alternatively, the reporter gene encodes a protein whose presence can be easily detected and/or quantified. Exemplary reporter genes include *lacZ,* chloramphenicol acetyl transferase (CAT), luciferase, green fluorescent protein (GFP), beta-glucuronidase (GUS), blue fluorescent protein (BFP), and derivatives of GFP, e.g., with altered or enhanced fluorescent properties (Clontech Laboratories, Inc. CA). Colonies of cells expressing lacZ can be easily detected by growing the colonies on plates containing the colorimetric substrate X-gal. GFP expression can be detected by monitoring fluorescence emission upon excitation. Individual GFP expressing cells can be identified and isolated using fluorescence activated cell sorting (FACS).

The system can be constructed with two reporter genes, e.g., a selectable reporter gene and a non-selectable reporter gene. The selectable marker facilitates rapid identification of the domain of interest, as under the appropriate growth conditions, only cells bearing the domain of interest grow. The non-selectable reporter provides a means of verification, e.g., to distinguish false-positives, and a means of quantifying the extent of binding. The two reporters can be integrated at separate locations in the genome, integrated in tandem in the genome, contained on the same extrachromosomal element (e.g., plasmid) or contained on separate extrachromosomal elements.

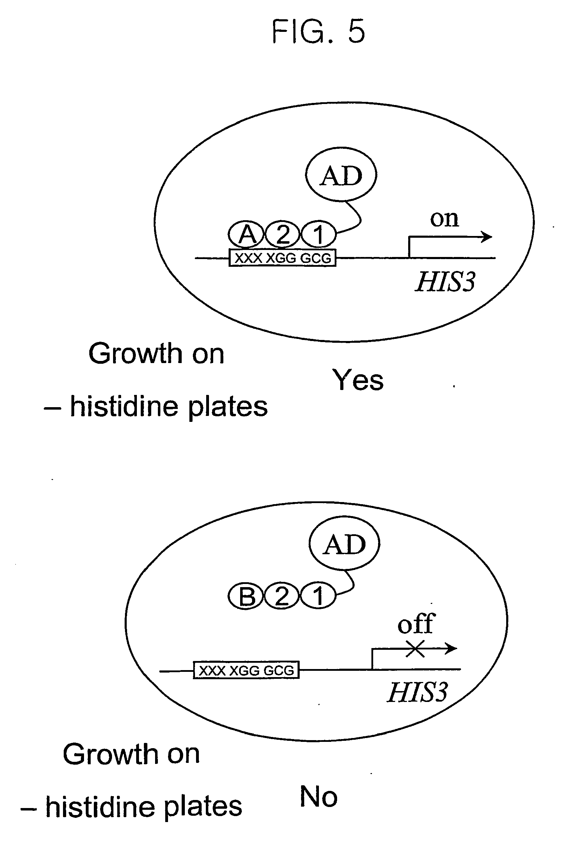

Fig. 5 illustrates the principle of the modified one-hybrid system used to select desired zinc finger domains. The DNA-binding domain of the hybrid transcription factor is composed of (a) a truncated DNA-binding domain consisting of finger 1 and finger 2 of Zif268 and (b) zinc finger domain A or B. The base sequence of the binding site located at the promoter region of the reporter gene is a composite binding sequence (5'-XXXXGGGCG-3'), which consists of a 4-bp target sequence (nucleotides 1 to 4, 5'-XXXX-3'), and a truncated binding sequence (nucleotides 5 to 9, 5'-GGGCG-3').

If the test zinc finger domain (A in Fig. 5) in the hybrid transcription factor recognizes the target sequence, the hybrid transcription factor can bind the composite binding sequence stably. This stable binding leads to expression of the reporter gene through the action of the activation domain (AD in Fig. 5) of the hybrid transcription factor. As a result, when *HIS3* is used as a reporter gene, the transformed yeast grows in medium devoid of histidine. Alternatively, when *lacZ* is used as a reporter gene, the transformed yeast grows as a blue colony in a medium containing X-gal, a substrate of the *lacZ* protein. However, if the zinc finger domain (B in Fig. 5) of the hybrid transcription factor fails to recognize the target sequence, expression of the reporter gene is not induced. As a result, the transformed yeast cannot grow in the medium devoid of histidine (when *HIS3* is used as a reporter gene) or grows as a white colony in a medium containing X-gal (when *lacZ* is used as a reporter gene).

The selection method using this modified one-hybrid system is advantageous because zinc finger domains selected by virtue of this procedure are demonstrated to function in the cellular milieu. Thus, the domains are presumably able to fold, enter the nucleus, and withstand intracellular proteases and other potentially damaging intracellular agents. Furthermore, the modified one-hybrid system disclosed herein allows the isolation of desired zinc finger domains quickly and easily. The modified one-hybrid system requires only a single round of transformation of yeast cells to isolate the desired zinc finger domains.

The selection method described herein can be utilized to identify a zinc finger domain from a genome e.g., a genome of a plant or animal species (e.g., a mammal, e.g., a human). The method can also be utilized to identify a zinc finger domain from a library of mutant zinc finger domains prepared, for example, by random mutagenesis. In addition, the two methods can be used in conjunction. For example, if a zinc finger domain cannot be isolated from the human genome for a particular 3-bp or 4-bp DNA sequence, a library of zinc finger domains prepared by random or directed mutagenesis can be screened for such a domain.

Although the modified one-hybrid system in yeast is a preferred means to select zinc finger domains that recognize and bind the given target sequences, it will be apparent to a person skilled in the art that systems other than yeast one-hybrid selection can be used. For example, phage display selection may be used to screen a library of naturally occurring zinc finger domains derived from a genome of a eukaryotic organism.

The present invention encompasses the use of the one-hybrid method in a variety of cultured cells. For example, a reporter gene operably linked to target sequences may be introduced into prokaryotic or animal or plant cells in culture, and the cultured cells may then be transfected with plasmids, phages, or viruses encoding a library of zinc finger domains. Desired zinc finger domains recognizing target sequences may then be obtained from the isolated cells in which the reporter gene is activated.

The examples disclosed below demonstrate that the method can identify zinc finger domains for binding sites of interest. A library of hybrid transcription factors with a variety of zinc finger domains positioned at finger 3 was prepared. Of the novel zinc finger domains (e.g., HSNK, QSTV, and VSTR zinc fingers; see below) selected from the library, none is naturally located at the C-terminus in its corresponding parent zinc finger protein. This clearly demonstrates that zinc finger domains are modular and that novel DNA-binding domains can be constructed by mixing and matching appropriate zinc finger domains.

The zinc finger domains selected via the method of the present invention can be used as building blocks to make new DNA-binding proteins by appropriate rearrangement and recombination. For example, a novel DNA-binding protein recognizing the promoter region of human *CCR5,* a coreceptor of HIV-1, can be constructed as follows. The promoter region of human *CCR5* contains the following 10-bp sequence: 5'-AGG GTG GAG T-3' (SEQ ID NO:4). Using the modified one-hybrid system disclosed herein, one should be able to isolate three zinc finger domains, each of which specifically recognizes one of the following 4-bp target sequences; 5'-AGGG-3', 5'-GTGG-3', and 5'-GAGT-3'. These target sequences are overlapping 4-bp segments of the *CCR5* target sequence. These three zinc finger domains can be connected with appropriate linkers and attached to a regulatory domain such as the VP16 domain and the GAL4 domain or repression domains such as the KRAB domain in order to generate novel transcription factors that specifically bind to the *CCR5* promoter. Similarly zinc fingers can be designed to recognize the following sequences:

| | | |
|---|---|---|
| HIV-1 LTR (-124/-115): | 5'-GAC ATC GAG C-3' | (SEQ ID NO:1) |
| HIV-1 LTR (-23/-14): | 5'-GCA GCT GCT T-3' | (SEQ ID NO:2) |
| HIV-1 LTR (-95/-86): | 5'-GCT GGG GAC T-3' | (SEQ ID NO:3) |
| Human CCR5 (-70/-79): | 5'-AGG GTG GAG T-3' | (SEQ ID NO:4) |
| Human CCR5 (+7/+16): | 5'-GCT GAG ACA T-3' | (SEQ ID NO:5) |

These zinc finger proteins could be used in therapy to help prevent proliferation of HIV-1.

### High Throughput Screening

The following method allows rapid measurement of the relative *in vivo* binding affinity for each domain in a collection for multiple possible DNA-binding sites or even all possible DNA-binding sites. A large collection of nucleic acids encoding nucleic acid binding domains is generated. Each nucleic acid binding domain is encoded as the test zinc finger domain in a hybrid nucleic acid construct, and expressed in a yeast strain of one mating type. Thus, a first set of yeast strains expressing all available or desired domains is generated. A second set of yeast strains containing reporter constructs with putative target sites for the domains in the reporter construct is constructed in the opposite mating type. The method requires performing many or all of the possible pairwise matings in order to create a matrix of fused cells, each having a different test zinc finger domain and a different target site reporter construct. Each fused cell is assayed for reporter gene expression. The method thereby rapidly and effortlessly determines the binding preferences of the tested domains.

A collection of domains is identified, e.g., by searching a genomics database for putative domains that fit a given profile. The collection can include, for example, ten to twenty domains, or all the identified domains, possibly thousands or more. Nucleic acids encoding the domains identified from the database are synthesized de novo or amplified from a sample of genomic DNA using oligonucleotides. Manual and automated methods for designing such synthetic oligonucleotides are routine in the art. Nucleic acids encoding additional domains can be similarly synthesized or amplified with degenerate primers. Nucleic acids encoding the domains of the collection are cloned into the yeast expression plasmid described above, thus creating fusion proteins of the domains and the first two fingers of Zif268 and a transcription activation domain. The amplification and cloning steps can be done in a microtitre plate format in order to clone nucleic acids encoding the multiple domains.

Alternatively, a recombinational cloning method can be used to rapidly insert multiple amplified nucleic acids encoding the domains into the yeast expression vector. This method, which is described in U.S. Patent No. 5,888,732 and the "Gateway" manual (Life Technologies-Invitrogen, CA, USA), entails including customized sites for a site-specific recombinase at the ends of the amplification primers. The expression vector contains an additional site or sites at the position for insertion of amplified nucleic acid encoding the domain. These sites are designed to lack stop codons. Addition of the amplification product, the expression vector, and the site-specific recombinase to the recombination reaction results in insertion of the amplified sequence into the vector. Additional features, e.g., the displacement of a toxic gene upon successful insertion, make this method highly efficient and suitable for high throughput cloning.

Restriction enzyme-mediated and/or recombination cloning can be used to insert nucleic acids encoding each of the identified domains into an expression vector. The vectors can be propagated in bacteria, and frozen in indexed microtitre plates, such that each well contains a cell harboring a nucleic acid encoding one of the different, unique DNA-binding domains.

Isolated plasmid DNA is obtained for each domain and transformed into a yeast cell, e.g., a *Saccharomyces cerevisiae MAT**a*** cell. As the expression vector contains a selectable marker, the transformed cells are grown in minimal medium under nutritional conditions selecting for the marker. Such cells can also be frozen and stored, e.g., in microtitre plates, for later use.

A second set of yeast strains is constructed, e.g., in a *Saccharomyces cerevisiae MAT**a*** cell. This set of yeast strains contains a variety of different reporter vectors. Each yeast strain bearing an expression vector with a unique DNA-binding domain is then mated to each yeast strain of the reporter gene set. As these two strains are from opposite mating types and are engineered to have different auxotrophies, diploids can easily be selected. Such diploids have both the reporter and the expression plasmids. The cells are also maintained under nutritional conditions that select for both the reporter and the expression plasmids. Uetz et al. (2000) Nature 403:623-7 describe a complete two-hybrid map of all yeast proteins by generating such a matrix of yeast matings.

Reporter gene expression can be detected in a high-volume format, e.g., in microtitre plates. For example, when using GFP as the reporter, a plate containing the matrix of mated cells can be scanned for fluorescence.

### Design of Novel DNA-Binding Proteins

A new DNA-binding protein can be rationally constructed to recognize a target 9-bp or longer DNA sequence by mixing and matching appropriate zinc finger domains. The modular structure of zinc finger domains facilitates their rearrangement to construct new DNA-binding proteins. As shown in Fig. 1a, zinc finger domains in the naturally-occurring Zif268 protein are positioned tandemly along the DNA double helix. Each domain independently recognizes a different 3-4 basepair DNA segment.

**A Database of Zinc Finger Domains.** The one-hybrid selection system described above can be utilized to identify one or more zinc finger domains for each possible 3- or 4-basepair binding site or a representative number of such binding sites. The results of this process can be accumulated as a series of associations between a zinc finger domain and its preferred 3- or 4-basepair binding site or sites. Examples of such associations are provided in Tables 3 to 6.

The results can also be stored in a machine as a database, e.g., a relational database, spreadsheet, or text file. Each record of such a database associates a representation of a zinc finger domain and a string indicating the sequence of the one or more preferred binding sites of the domain. The database record can include an indication of the relative affinity of the zinc finger domains that bind each site. In some implementations, the database record can also include information that indicates the physical location of the nucleic acid encoding the particular zinc finger domain. Such a physical location can be, for example, a particular well of a microtitre plate stored in a freezer.

The database can be configured so that it can be queried or filtered, e.g., using a SQL operating environment, a scripting language (such as PERL or a Microsoft Excel® macro), or a programming language. Such a database would enable a user to identify one or more zinc finger domains that recognizes a particular 3- or 4-basepair binding site. Database and other information such as can be stored on a database server can also be configured to communicate with each device using commands and other signals that are interpretable by the device. The computer-based aspects of the system can be implemented in digital electronic circuitry, or in computer hardware, firmware, software, or in combinations thereof. An apparatus of the invention, e.g., the database server, can be implemented in a computer program product tangibly embodied in a machine-readable storage device for execution by a programmable processor; and method actions can be performed by a programmable processor executing a program of instructions to perform functions of the invention by operating on input data and generating output. One non-limiting example of an execution environment includes computers running Windows XP or Windows NT 4.0 (Microsoft) or better or Solaris 2.6 or better (Sun Microsystems) operating systems.

The zinc finger domains can also be tested in the context of multiple different fusion proteins to verify their specificity. Moreover, particular binding sites for which a paucity of domains is available can be the target of additional selection screens. Libraries for such selections can be prepared by mutagenizing a zinc finger domain that binds a similar yet distinct site. A complete matrix of zinc finger domains for each possible binding site is not essential, as the domains can be staggered relative to the target binding site in order to best utilize the domains available. Such staggering can be accomplished both by parsing the binding site in the most useful 3 or 4 basepair binding sites, and also by varying the linker length between zinc finger domains. In order to incorporate both selectivity and high affinity into the design polypeptide, zinc finger domains that have high specificity for a desired site can be flanked by other domains that bind with higher affinity, but lesser specificity. The *in vivo* screening method described herein can used to test the *in vivo* function, affinity, and specificity of an artificially assembled zinc finger protein and derivatives thereof. Likewise, the method can be used to optimize such assembled proteins, e.g., by creating libraries of varied linker composition, zinc finger domain modules, zinc finger domain compositions, and so forth.

**Parsing a target site.** The target 9-bp or longer DNA sequence is parsed into 3- or 4-bp segments. Zinc finger domains are identified (e.g., from a database described above) that recognize each parsed 3- or 4-bp segment. Longer target sequences, e.g., 20 bp to 500 bp sequences, are also suitable targets as 9 bp, 12 bp, and 15 bp subsequences can be identified within them. In particular, subsequences amenable for parsing into sites well represented in the database can serve as initial design targets.

A scoring regime can be used to estimate the probability that a particular designed chimeric zinc finger protein would recognize the target site in the cell. The scores can be a function of each component finger's affinity for its preferred subsites, its specificity, and its success in previously designed proteins.

**Computer Programs.** Computer systems and software can be used to access a machine-readable database described above, parse a target site, and output one or more chimeric zinc finger protein designs.

The techniques may be implemented in programs executing on programmable machines such as mobile or stationary computers, and similar devices that each include a processor, a storage medium readable by the processor, and one or more output devices. Each program may be implemented in a high level procedural or object oriented programming language to communicate with a machine system. Some merely illustrative examples of computer languages include C, C++, Java, Fortran, and Visual Basic.

Each such program may be stored on a storage medium or device, e.g., compact disc read only memory (CD-ROM), hard disk, magnetic diskette, or similar medium or device, that is readable by a general or special purpose programmable machine for configuring and operating the machine when the storage medium or device is read by the computer to perform the procedures described in this document. The system may also be implemented as a machine-readable storage medium, configured with a program, where the storage medium so configured causes a machine to operate in a specific and predefined manner.

The computer system can be connected to an internal or external network. For example, the computer system can receive requests from a remotely located client system, e.g., using HTTP, HTTPS, or XML protocols. The requests can be an identifier for a known target gene or a string representing the sequence of a target nucleic acid. In the former case, the computer system can access a sequence database such as GenBank to retrieve the nucleic acid sequence of regulatory regions of the target gene. The sequence of the regulatory region or the directly-received target nucleic acid sequence is then parsed into subsites, and chimeric zinc finger proteins are designed, e.g., as described above.

The system can communicate the results to the remotely located client. Alternatively, the system can control a robot to physically retrieve nucleic acid encoding the designed chimeric zinc finger proteins. In this implementation, a library of nucleic acids encoding chimeric zinc finger proteins is constructed and stored, e.g., as frozen purified DNA or frozen bacterial strains harboring the nucleic acids. The robot responds to signals from the computer system by accessing specified addresses of the library. The retrieved nucleic acids can then be processed, packaged and delivered to the client. Alternatively, the retrieved nucleic acids can be introduced into cells and assayed. The computer system can then communicate the results of the assay to the client across the network.

**Constructing a Protein from Selected Modules.** Once a chimeric polypeptide sequence containing multiple zinc finger domains is designed, a nucleic acid sequence encoding the designed polypeptide sequence can be synthesized. Methods for constructing synthetic genes are routine in the art. Such methods include gene construction from custom synthesized oligonucleotides, PCR mediated cloning, and mega-primer PCR. Example 66, below, provides a method of serially ligating nucleic acids encoding selected zinc finger domains to form a nucleic acid encoding a chimeric polypeptide. Additional sequences can be joined to the nucleic acid encoding the designed polypeptide sequence. The additional sequence can provide regulatory functions or a sequence coding for an amino acid sequence with a desired function. Examples of such additional sequences are described herein.

### Constructing Libraries of Chimeric Zinc Finger Proteins

Multiple nucleic acid sequences encoding chimeric zinc finger proteins can be synthesized, e.g., to form a library. The library of nucleic acids encoding diverse chimeric zinc finger proteins can be formed by serial ligation, e.g., as described in Example 67. The library can be constructed such that each nucleic acid encodes a protein that has at least three, four, or five zinc finger domains. In some implementations, particularly for large libraries, each zinc finger position can be designed to randomly include any one of a set of zinc finger domains. The set of zinc finger domains can be selected to represent domains with a range of specificities, e.g., covering 30, 40, 50 or more of the 64 possible 3-basepair subsites. The set can include at least about 12, 15, 20, 25, 30, 40 or 50 different zinc finger domains. Some or all of these domains can be domains isolated from naturally occurring proteins.

One exemplary library includes nucleic acids that encode a chimeric zinc finger protein having 3 fingers and 30 possible domains at each finger. In its fully represented form, this library includes 27,000 sequences (i.e., the result of 30³). The library can be constructed by serial ligation in which a pool of nucleic acids encoding all 30 possible domains is added at each step. The final library can be stored as a pool.

Alternatively, individual members can be isolated, stored at an addressable location (e.g., arrayed), and sequenced. After high throughput sequencing of 40 to 50 thousand constructed library members, missing chimeric combinations can be individually assembled in order to obtain complete coverage. Once arrayed, e.g., in microtitre plates, each individual member can be recovered later for further analysis or for a particular application. In particular, each individual member can be validated by determining if it can repress transcription *in vivo* using the p1G reporter assay described in Example 68. If validated, the library member can be profiled using nucleic acid microarrays to determine its ability to regulate endogenous genes (see "Profiling Regulatory Properties of a Chimeric Zinc Finger Protein," below).

Small libraries, e.g., having about 6 to 200 or 50 to 2000 members can be used to identify an optimal chimeric protein that binds a target site. These libraries can be designed by the judicious selection of combinations of nucleic acids encoding particular zinc finger domains for each positioned zinc finger in the resultant chimeric polypeptide. For example, the nucleic acids that encode a particular position can be selected to vary such that they encode different zinc finger domains whose recognition specificity is suitable for that position.

These small tailored libraries can be synthesized by serial ligation or by pooling specific library members from a prefabricated and arrayed large library. Subsequent steps (e.g., sexual PCR and "DNA Shuffling™" (Maxygen, Inc., CA)) can be used to introduce additional diversity.

### Screening Libraries of Chimeric Zinc Finger Proteins

The libraries can be designed with a particular intended screening application, in which case the parental vector can be engineered to include necessary regulatory and functional sequences. In one embodiment, the library is designed such that the nucleic acid encoding the chimeric zinc finger protein is flanked by site-specific recombination sites. Recombination-mediated cloning, e.g., as described in U.S. Patent No. 5,888,732 and the "Gateway" manual (Life Technologies-Invitrogen, CA, USA), then enables each sequence to be excised from a parent vector and inserted into an application-specific vector. Thus, once a complete library is constructed in the parent vector, it can subjected to diverse screening and selective procedures.

Library members (from a small or large library) can be screened *in vivo* to determine if it can regulate a target gene of interest in a cell. The cell can be cultured or within a subject. The target gene can be a reporter construct that includes a regulatory region of interest operably linked to a heterologous reporter gene, e.g., as described in Example 64. Alternatively, the target gene can be an endogenous gene. The effect of one or more proteins encoded by library members on the regulation of the endogenous gene in its normal chromosomal milieu is assessed. The screening can also include assessing if the chimeric protein encoded by the library member alters the transcription of other genes. Nucleic acid arrays can be used to monitor the expression of large numbers of such genes as described below.

The library can also be screened using a display format in order to biochemically query the chimeric proteins encoded by library members. For example, the polypeptides encoded by the library can be displayed on the surface of bacteriophages, e.g., as described in U.S. Patent No. 5,223,409 and Rebar et al., (1996) Methods Enzymol. 267:129-49. The library can also be displayed by covalently linking each nucleic acid of the library to the polypeptide that it encodes, e.g., using the method described in WO 00/32823. Individual library members with a particular binding property can be isolated by contacting the display library to a target DNA site that is fixed to a solid support, washing the support, and recovering the bound library members. This method can be adapted to identify chimeric polypeptides that bind to other ligands, e.g., a target RNA site or a target protein.

In one embodiment, the chimeric protein encoded by each library member is produced and isolated at an address of a planar array. Methods for producing polypeptide arrays are described, e.g., in De Wildt et al., (2000) Nature Biotech. 18:989-994; Lueking et al., (1999) Anal. Biochem. 270:103-111; Ge, H. (2000) Nucleic Acids Res. 28:e3, I-VII; MacBeath and Schreiber, (2000) Science 289, 1760-1763; Haab et al., (2001) Genome Biology 2(2):research0004.1; and WO 99/51773A1. Such an array can be used to identify library members that bind a particular target DNA site. DNA including the target site is labeled and contacted to the array. The amount of label at each address of the array is quantitated to identify library members that binding to the target site. The assay can include non-specific DNA or a competitor DNA in order to increase the stringency of the selection. This method can be adapted to identify chimeric proteins that bind to targets other than DNA, e.g., by using a labeled target RNA or a labeled target protein.

The array of zinc finger proteins can also be used to profile a complex nucleic acid sample. The sample is labeled and contacted to the array. Then, binding to each address is quantified in order to generate a profile for the sample. The profile can be compared to reference profiles in order to characterize the sample.

### Profiling Regulatory Properties of a Chimeric Zinc Finger Protein

A chimeric zinc finger protein can be characterized to determine its ability to regulate an endogenous gene of a cell, e.g., a mammalian cell. Nucleic acid encoding the chimeric zinc finger protein is first fused to a repression or activation domain, and then introduced into a cell of interest. After appropriate incubation and induction of expression of the coding nucleic acid, mRNA is harvested from the cell and analyzed using a nucleic acid microarray.

Nucleic acid microarrays can be fabricated by a variety of methods, e.g., photolithographic methods (see, e.g., U.S. Patent No. 5,510,270;), mechanical methods (e.g., directed-flow methods as described in U.S. Patent No. 5,384,261), and pin based methods (e.g., as described in U.S. Pat. No. 5,288,514). The array is synthesized with a unique capture probe at each address, each capture probe being appropriate to detect a nucleic acid for a particular expressed gene.

The mRNA can be isolated by routine methods, e.g., including DNase treatment to remove genomic DNA and hybridization to an oligo-dT coupled solid substrate (e.g., as described in Current Protocols in Molecular Biology, John Wiley & Sons, N.Y). The substrate is washed, and the mRNA is eluted. The isolated mRNA is then reversed transcribed and optionally amplified, e.g., by rtPCR, e.g., as described in (U.S. Patent No. 4,683,202). The nucleic acid can be labeled during amplification or reverse transcription, e.g., by the incorporation of a labeled nucleotide. Examples of preferred labels include fluorescent labels, e.g., red-fluorescent dye Cy5 (Amersham) or green-fluorescent dye Cy3 (Amersham). Alternatively, the nucleic acid can be labeled with biotin, and detected after hybridization with labeled streptavidin, e.g., streptavidin-phycoerythrin (Molecular Probes).

The labeled nucleic acid is then contacted to the array. In addition, a control nucleic acid or a reference nucleic acid can be contacted to the same array. The control nucleic acid or reference nucleic acid can be labeled with a label other than the sample nucleic acid, e.g., one with a different emission maximum. Labeled nucleic acids are contacted to an array under hybridization conditions. The array is washed, and then imaged to detect fluorescence at each address of the array.

A general scheme for producing and evaluating profiles is includes detecting hybridization at each address of the array. The extent of hybridization at an address is represented by a numerical value and stored, e.g., in a vector, a one-dimensional matrix, or one-dimensional array. The vector x has a value for each address of the array. For example, a numerical value for the extent of hybridization at a particular address is stored in variable xₐ. The numerical value can be adjusted, e.g., for local background levels, sample amount, and other variations. Nucleic acid is also prepared from a reference sample and hybridized to the same or a different array. The vector y is construct identically to vector x. The sample expression profile and the reference profile can be compared, e.g., using a mathematical equation that is a function of the two vectors. The comparison can be evaluated as a scalar value, e.g., a score representing similarity of the two profiles. Either or both vectors can be transformed by a matrix in order to add weighting values to different genes detected by the array.

The expression data can be stored in a database, e.g., a relational database such as a SQL database (e.g., Oracle or Sybase database environments). The database can have multiple tables. For example, raw expression data can be stored in one table, wherein each column corresponds to a gene being assayed, e.g., an address or an array, and each row corresponds to a sample. A separate table can store identifiers and sample information, e.g., the batch number of the array used, date, and other quality control information.

Genes that are similarly regulated can be identified by clustering expression data to identify coregulated genes. Such cluster may be indicative of a set of genes coordinately regulated by the chimeric zinc finger protein. Genes can be clustered using hierarchical clustering (see, e.g., Sokal and Michener (1958) *Univ. Kans. Sci. Bull.* 38:1409), Bayesian clustering, k-means clustering, and self-organizing maps (see, Tamayo et al. (1999) Proc. Natl. Acad. Sci. USA 96:2907).

The similarity of a sample expression profile to a reference expression profile (e.g., a control cell) can also be determined, e.g., by comparing the log of the expression level of the sample to the log of the predictor or reference expression value and adjusting the comparison by the weighting factor for all genes of predictive value in the profile.

### Additional Features for Designed Transcription Factors

**Peptide Linkers.** DNA binding domains can be connected by a variety of linkers. The utility and design of linkers are well known in the art. A particularly useful linker is a peptide linker that is encoded by nucleic acid. Thus, one can construct a synthetic gene that encodes a first DNA binding domain, the peptide linker, and a second DNA binding domain. This design can be repeated in order to construct large, synthetic, multi-domain DNA binding proteins. PCT WO 99/45132 and Kim and Pabo ((1998) Proc. Natl. Acad. Sci. USA 95:2812-7) describe the design of peptide linkers suitable for joining zinc finger domains.

Additional peptide linkers are available that form random coil, α-helical or β-pleated tertiary structures. Polypeptides that form suitable flexible linkers are well known in the art (see, e.g., Robinson and Sauer (1998) Proc Natl Acad Sci U S A. 95:5929-34). Flexible linkers typically include glycine, because this amino acid, which lacks a side chain, is unique in its rotational freedom. Serine or threonine can be interspersed in the linker to increase hydrophilicity. In additional, amino acids capable of interacting with the phosphate backbone of DNA can be utilized in order to increase binding affinity. Judicious use of such amino acids allows for balancing increases in affinity with loss of sequence specificity. If a rigid extension is desirable as a linker, α-helical linkers, such as the helical linker described in Pantoliano et al. (1991) Biochem. 30:10117-10125, can be used. Linkers can also be designed by computer modeling (see, e.g., U.S. Pat. No. 4,946,778). Software for molecular modeling is commercially available (e.g., from Molecular Simulations, Inc., San Diego, CA). The linker is optionally optimized, e.g., to reduce antigenicity and/or to increase stability, using standard mutagenesis techniques and appropriate biophysical tests as practiced in the art of protein engineering, and functional assays as described herein.

For implementations utilizing zinc finger domains, the peptide that occurs naturally between zinc fingers can be used as a linker to join fingers together. A typical such naturally occurring linker is: Thr-Gly-(Glu or Gln)-(Lys or Arg)-Pro-(Tyr or Phe) (SEQ ID NO:78) (Agata *et al.*, *supra*).

**Dimerization Domains.** An alternative method of linking DNA binding domains is the use of dimerization domains, especially heterodimerization domains (see, e.g., Pomerantz et al (1998) Biochemistry 37:965-970). In this implementation, DNA binding domains are present in separate polypeptide chains. For example, a first polypeptide encodes DNA binding domain A, linker, and domain B, while a second polypeptide encodes domain C, linker, and domain D. An artisan can select a dimerization domain from the many well-characterized dimerization domains. Domains that favor heterodimerization can be used if homodimers are not desired. A particularly adaptable dimerization domain is the coiled-coil motif, e.g., a dimeric parallel or anti-parallel coiled-coil. Coiled-coil sequences that preferentially form heterodimers are also available (Lumb and Kim, (1995) Biochemistry 34:8642-8648). Another species of dimerization domain is one in which dimerization is triggered by a small molecule or by a signaling event. For example, a dimeric form of FK506 can be used to dimerize two FK506 binding protein (FKBP) domains. Such dimerization domains can be utilized to provide additional levels of regulation.

### Functional Assays and Uses

In addition to biochemical assays, the function of a nucleic acid binding domain or a protein designed by a method described herein, e.g., by modular assembly, can be assayed or used *in vivo.* For example, domains can be selected to bind to a target site, e.g., to a promoter site of a gene required for cell proliferation. By modular assembly, a protein can be designed that includes (1) the selected domains that respectively bind to subsites spanning the target promoter site, and (2) a DNA repression domain, e.g., a WRPW domain.

A nucleic acid sequence encoding a designed protein can be cloned into an expression vector, e.g., an inducible expression vector as described in Kang and Kim, (2000) J Biol Chem 275:8742. The inducible expression vector can include an inducible promoter or regulatory sequence. Non-limiting examples of inducible promoters include steroid-hormone responsive promoters (e.g., ecdysone-responsive, estrogen-responsive, and glutacorticoid-responsive promoters), the tetracyclin "Tet-On" and "Tet-Off" systems, and metal-responsive promoters. The construct can be transfected into tissue culture cells or into embryonic stem cells to generate a transgenic organism as a model subject. The efficacy of the designed protein can be determined by inducing expression of the protein and assaying cell proliferation of the tissue culture cell or assaying for developmental changes and/or tumor growth in a transgenic animal model. In addition, the level of expression of the gene being targeted can be assayed by routine methods to detect mRNA, e.g., RT-PCR or Northern blots. A more complete diagnostic includes purifying mRNA from cells expressing and not expressing the designed protein. The two pools of mRNA are used to probe a microarray containing probes to a large collection of genes, e.g., a collection of genes relevant to the condition of interest (e.g., cancer) or a collection of genes identified in the organism's genome. Such an assay is particularly valuable for determining the specificity of the designed protein. If the protein binds with high affinity but little specificity, it may cause pleiotropic and undesirable effects by affecting expression of genes in addition to the contemplated target. Such effects are revealed by a global analysis of transcripts.

In addition, the designed protein can be produced in a subject cell or subject organism in order to regulate an endogenous gene. The designed protein is configured, as described above, to bind to a region of the endogenous gene and to provide a transcriptional activation or repression function. As described in Kang and Kim (*supra*), the expression of a nucleic acid encoding the designed protein can be operably linked to an inducible promoter. By modulating the concentration of the inducer for the promoter, the expression of the endogenous gene can be regulated in a concentration dependent manner.

### Assaying binding site preference

The binding site preference of each domain can be verified by a biochemical assay such as EMSA, DNase footprinting, surface plasmon resonance, or column binding. The substrate for binding can be a synthetic oligonucleotide encompassing the target site. The assay can also include non-specific DNA as a competitor, or specific DNA sequences as a competitor. Specific competitor DNAs can include the recognition site with one, two, or three nucleotide mutations. Thus, a biochemical assay can be used to measure not only the affinity of a domain for a given site, but also its affinity to the site relative to other sites. Rebar and Pabo, (1994) Science 263:671-673 describe a method of obtaining apparent K_{d} constants for zinc finger domains from EMSA.

The present invention will be described in more detail through the following practical examples. However, it should be noted that these examples are not intended to limit the scope of the present invention.

### Example 1: Construction of Plasmids for Hybrid Transcription Factor Expression.

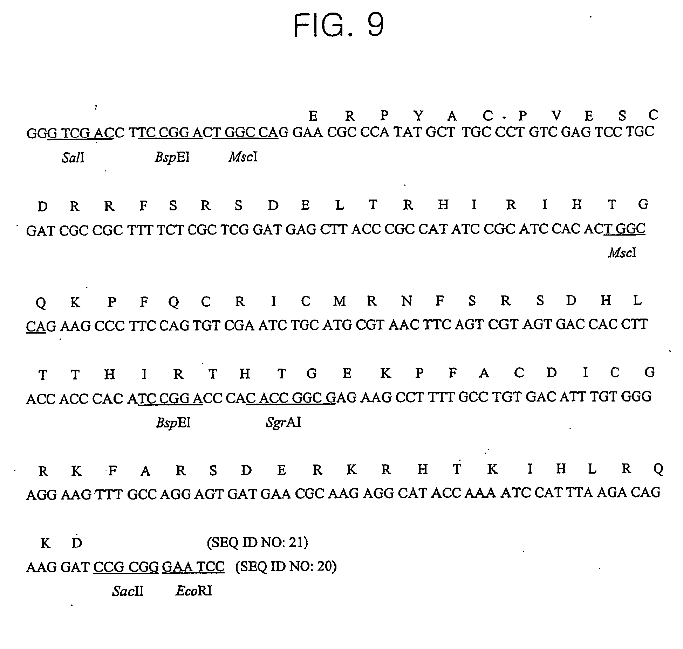

An expression plasmid expressing a zinc finger transcription factor was prepared by modification of pPC86 (Chevray and Nathans, (1991) Proc. Natl. Acad. Sci. USA 89:5789-5793). Manipulations of DNA were performed as described in Ausubel et al. (Current Protocols in Molecular Biology (1998), John Wiley and Sons, Inc.). A DNA fragment encoding Zif268 zinc finger protein was inserted between the *Sal*I and *EcoRI* recognition sites of pPC86 to generate pPCFM-Zif. The result of this cloning step is a translational fusion protein encoding the yeast Gal4 activation domain followed by the three Zif268 zinc fingers. Transformation of pPCFM-Zif into yeast cells results in expression of a hybrid transcription factor comprising the yeast Gal4 activation domain and the Zif268 zinc fingers. The DNA sequence encoding the Zif268 zinc finger protein as cloned in pPCFM-Zif is shown in Fig. 9.

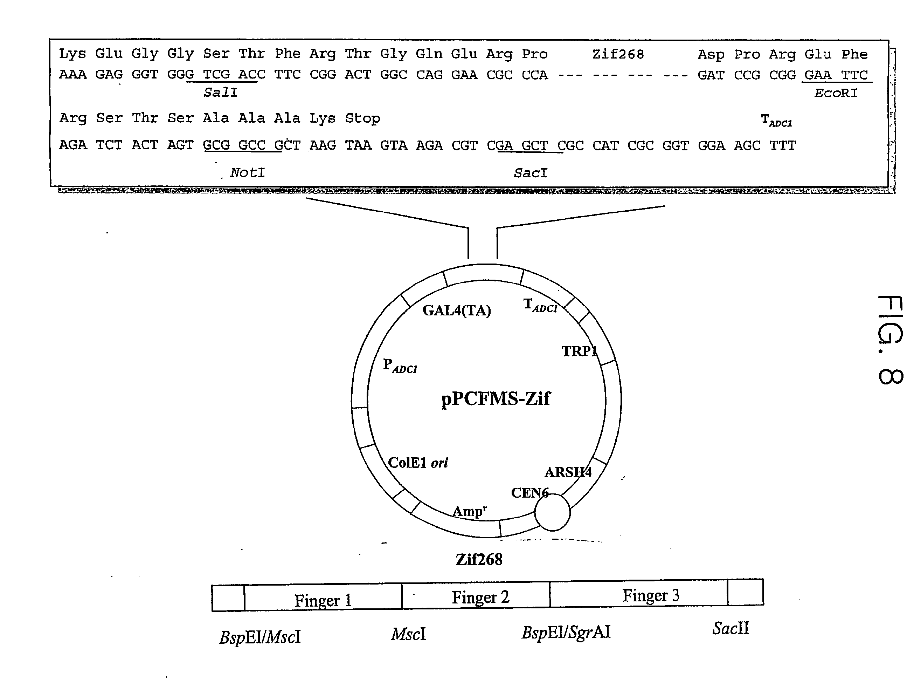

The plasmid pPCFMS-Zif was utilized as a vector for constructing libraries of zinc finger domains (Fig. 8). pPCFMS-Zif was constructed by insertion of an oligonucleotide cassette containing a stop codon and a *Pst*I recognition site in front of the finger 3 coding region of pPCFM-Zif. The oligonucleotide cassette was formed by annealing two synthetic oligonucleotides: 5'-TGCCTGCAGCATTTGTGGGAGGAAGTTTG-3' (SEQ ID NO:79); and 5'-ATGCTGCAGGCTTAAGGCTTCTCGCCGGTG-3'(SEQ ID NO:80). The insertion of a stop codon prevents the generation of library plasmids encoding finger 3 of Zif268.

The plasmid was used as a vector for the generation of zinc finger domain libraries as described in "Example 2" below.

In addition, gap repair cloning of DNA sequences encoding individual zinc finger domains was carried out as described in Hudson et al., ((1997) Genome Research 7:1169-1173) with minor modification.

To clone an individual zinc finger domain, two overlapping oligonucleotides were synthesized. Each oligonucleotide included a 21-nucleotide-long common tail at its 5' end for second round PCR (rePCR) and a specific sequence that annealed to the nucleic acid encoding the individual zinc finger domain. The sequences of the forward and back primers were 5'-ACCCACACTGGCCAGAAACCCN₄₈₋₅₁ - 3' (SEQ ID NO:108) and 5'-GATCTGAATTCATTCACCGGTN₄₂₋₄₅ - 3' (SEQ ID NO:109), respectively, where N₄₃₋₅₁ and N₄₂₋₄₅ correspond to the customized sequence for annealing to the nucleic acid encoding the zinc finger domain. Double stranded DNA was prepared by amplifying template nucleic acid with an equimolar mixture of two oligonucleotides. PCR conditions consisted of a first cycle at 94°C for 3 minutes followed by 5 cycles of 94°C for 1 minutes, 50°C for 1 minutes , and 72°C for 30 seconds.

The double stranded DNA encoding each zinc finger domain was then used as a template in second round PCR. The rePCR primers had two regions, one region that is identical to yeast vector pPCFM-Zif and a second region that is identical to the 21-nucleotide-long common tail sequence described above. The sequence of forward primer was 5'-TGTCGAATCTGCATGCGTAACTTCAGTCGTAGTGACCACCTTACCACC CAC ATCCGGACCCACACTGGCCAGAAACCC-3' (SEQ ID NO:138) and that of reverse primer was 5'-GGTGGCGGCCGTTACTTACTTAGAGCTCGACGTCTTACTTACTTAGC GGCCGCACTAGTAGATCTGAATTCATTCACCGGT - 3' (SEQ ID NO:139). The reaction mixture contained 2.5 pmoles of each primer, 1.5mM Mg²⁺, 2 units of *Taq* polymerase and 0.01 units of *Pfu* polymerase in 25 ul. Reactions were carried out at 94°C for 3 min, then cycled through 20 cycles of 94°C for 1 min, 65°C for 1min, and 72°C for 30 sec or at 94°C for 3 min, then cycled through 25 cycles of 94°C for 30 seconds and 72°C for 30 seconds.

Gap repair cloning was performed by transforming the mixture of rePCR products and linearized pPCFM-Zif vector that had been digested with *Msc*I and *Eco*RI into yeast YW1 cells. The region identical to the yeast vector pPCFM-Zif allows for homologous recombination with the vector in the yeast cells. All constructs thus formed were confirmed by DNA sequencing.

### Example 2: Construction of a Library for Assaying an Individual Zinc Finger Domain

A plasmid library of naturally occurring zinc finger domains was prepared by cloning zinc finger domains from the human genome. DNA segments encoding zinc finger domains were amplified from template human genomic DNA (purchased from Promega Corporation, Madison, WI, USA) using PCR and degenerate oligonucleotide primers. The DNA sequences of the degenerate PCR primers used to clone human zinc finger domains were as follows; 5'-GCGTCCGGACNCAYACNGGNSARA -3' (SEQ ID NO:81) and 5'-CGGAATTCANNBRWANGGYYTYTC -3' (SEQ ID NO:82), wherein R represent G and A; B represents G, C, and T; S represents G and C; W represents A and T; Y represents C and T; and N represents A, C, G, and T.

The degenerate PCR primers anneal to nucleic acid sequences coding for an amino acid profile, His-Thr-Gly-(Glu or Gln)-(Lys or Arg)-Pro-(Tyr or Phe) (SEQ ID NO:83), that is found at the junction between zinc finger domains in many naturally occurring zinc finger proteins (Agata et al. (1998) Gene 213:55-64).

The buffer composition of the PCR reaction was 50 mM KC1, 3 mM MgCl₂, 10 mM Tris pH 8.3. Taq DNA polymerase was added and the reaction mixture was incubated at 94°C for 30 seconds, at 42°C for 60 seconds, and then at 72°C for 30 seconds. This cycle was repeated 35 times, and was followed by a final incubation at 72°C for 10 minutes.

The PCR products were cloned into pPCFMS-Zif as follows: The PCR products were electrophoresed, and the DNA segments corresponding to about 120 bp were isolated. After digestion with *Bsp*EI and *Eco*RI, the 120-bp DNA segments were ligated into pPCFMS-Zif. As a result, the DNA-binding domain of the hybrid transcription factor encoded by this plasmid library consists of finger 1 and finger 2 of Zif268 and a zinc finger domain derived from the human genome. The plasmid library was prepared from a total of 10⁶ *Escherichia coli* transformants. This library construction scheme retains the naturally occurring linker sequence found between zinc finger domains.

### Example 3: Construction of a Library for Assaying an Individual Zinc Finger Domain

A library of mutant zinc finger domains was prepared by random mutagenesis. Finger 3 of Zif268 was used as a polypeptide framework. Random mutations were introduced at positions -1, 2, 3, 4, 5, and 6 along the α-helix, corresponding respectively to the arginine at position 73, aspartic acid at position 75, glutamic acid at position 76, arginine at position 77, lysine at position 78, and arginine at position 79 of SEQ ID NO:21 (within finger 3 of Zif268).

At each of the nucleic acid sequence positions encoding these amino acids, a randomized codon, 5'-(G/A/C) (G/A/C/T) (G/C)-3', was introduced. This randomized codon encodes any one of 16 amino acids (excluding four amino acids: tryptophan, tyrosine, cysteine and phenylalanine). Also excluded are all three possible stop codons. The randomized codons were introduced with an oligonucleotide cassette constructed from two oligonucleotides: 5'-CTCCCCGCGGTTCGCCGGTGTGGATTCTGATATGSNBSNBAAGS NBSNBSNBSNBTGAGAATCTTCTATCACAAG-3' (SEQ ID NO:85), wherein B represents G, T, and C; S represents G and C; and N represents A, G, C, and T.

After annealing these two oligonucleotides, the DNA duplex cassette was synthesized by reaction with Klenow polymerase for 30 minutes. After digestion with *AvaI* and *SacII*, the DNA duplex was ligated into pPCFMS-Zif digested with SgrAI and SacII. Plasmids were isolated from about 10⁹ *E. coli* transformants.

### Example 4: Construction of Reporter Plasmids

Reporter plasmids including the yeast *HIS3* gene were prepared by modification of pRS315His (Wang and Reed (1993) Nature 364:121-126). The reporter plasmids also contain the *LEU2* marker under its natural promoter for the purpose of selecting transformants bearing the plasmid. First, the *Sal*I recognition site in pRS315His was removed by ligating the small fragment of pRS315His after digestion with *Sal*I and *Bam*HI and the large fragment of pRS315His after digestion with *Bam*HI and *Xho*I to make pRS315HisΔSal. Next, a new *Stal*I recognition site was created within the promoter region of the *HIS3* gene by inserting an oligonucleotide duplex into pRS315HisΔSal between the *Bam*HI and *Sma*I site. The sequences of the two oligonucleotides that were annealed to produce the inserted duplex are
5'-CTAGACCCGGGAATTCGTCGACG-3' (SEQ ID NO:86); and
5'-GATCCGTCGACGAATTCCCGGGT-3' (SEQ ID NO:87). The resulting plasmid was named pRS315HisMCS.

Multiple reporter plasmids were constructed by inserting desired composite sequences into pRS315HisMCS. The composite sequences are inserted as a tandem array containing four copies of the composite sequence. The target sequences were derived from 10-bp DNA sequences found in the LTR region of HIV-1:
5'-GAC ATC GAG C-3' (SEQ ID NO:1) HIV-1 LTR (-124/-115)
5'-GCA GCT GCT T-3' (SEQ ID NO:2) HIV-1 LTR (-23/-14)
5'-GCT GGG GAC T-3' (SEQ ID NO:3) HIV-1 LTR (-95/-86))
and in the promoter of human *CCR5* gene:
5'-AGG GTG GAG T-3' (SEQ ID human Curs (-70/-79) NO:4)
5'-GCT GAG ACA T-3' (SEQ ID human CCR5 (+7/+16)). NO:5)

Each of these 10-bp DNA sequence can be parsed into component 4-bp target sites in order to identify a zinc finger domain that recognizes each region of the site. Using the modular assembly method, such zinc finger domains can be coupled to produce a DNA binding protein that recognizes the site *in vivo.*

The underlined portions above depict examples of 4-bp target sequences. Each of these 4-bp target sequences was connected to the 5-bp recruitment sequence, 5'-GGGCG-3', that is recognized by finger 1 and finger 2 of Zif268. The resulting 9-bp sequences constitute composite binding sequences. Each composite binding sequence has the following format:
5'-XXXXGGGCG-3', where XXXX is the 4-bp target sequence and the adjacent 5'-GGGCG-3' is the recruitment sequence.

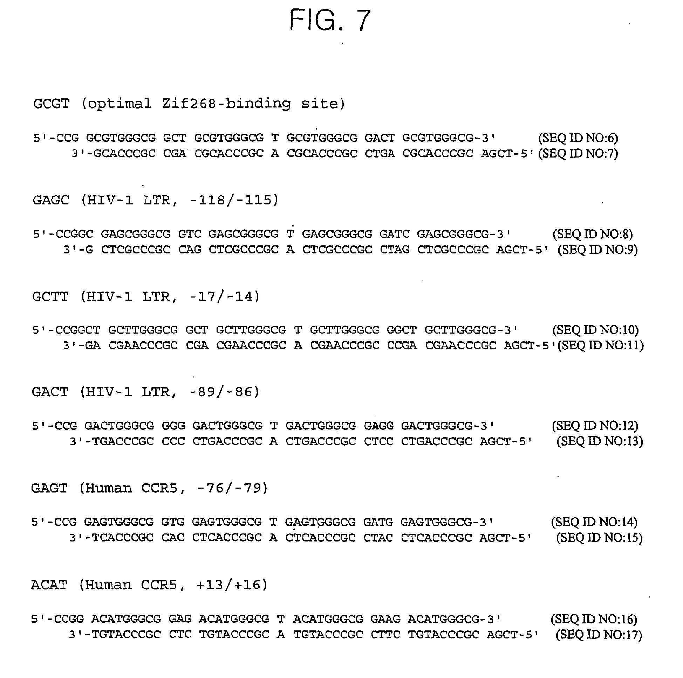

Fig. 7 recites the DNA sequences of the inserted tandem arrays of composite binding sites, each of which was operably linked to the reporter gene in pRS315HisMCS. Each tandem array contains 4 copies of a composite binding sequence. For each binding site, two oligonucleotides were synthesized, annealed and ligated into pRS315HisMCS restricted with *Sal*I and *Xma*I site to make a reporter plasmid.

### Example 5: Construction of reporter plasmids

A set of reporter plasmids that includes a pair of reporters (one having *lacZ*, the other having *HIS3*) for each 3 basepair subsite was constructed as follows: Reporter plasmids were constructed by inserting the desired target sequences into pRS315HisMCS and pLacZi. For each 3 basepair target site, two oligonucleotides were synthesized, annealed, and inserted into the *Sal*I and *Xma*I site of pRS315HisMCS and of pLacZi to make reporter plasmids. The DNA sequences of the oligonucleotides were as follows: 5'- CCGGT NNNTGGGCG TAC NNNTGGGCG TCA NNNTGGGCG -3' (SEQ ID NO:88) and 5'- TCGA CGCCCANNN TGA CGCCCANNN GTA CGCCCANNN A -3' (SEQ ID NO:89). Total 64 pairs of oligonucleotides were synthesized and inserted into the two reporter plasmids.

### Example 6: Selection of Zinc Finger Domains with Desired DNA-Binding Specificity

To select zinc finger domains that specifically bind given target sequences, yeast cells were transformed first with a reporter plasmid and then a library of hybrid plasmids encoding hybrid transcription factors. Yeast transformation and screening procedures were carried out as described in Ausubel et al. (Current Protocols in Molecular Biology (1998), John Wiley and Sons, Inc.). Yeast strain yWAM2 (*MAT**a**(alpha) Δgal4 Δgal80 URA3::GAL1-lacZ lys2801 his3-Δ200 trp1-Δ63 leu2 ade2-101CYH2)* was used.

In one instance, yeast cells were first transformed with a reporter plasmid containing the composite binding sequence 5'-GAGCGGGCG-3' (the 4-bp target sequence is underlined), which was operably linked to the reporter gene. Then, the plasmid library of mutant zinc finger domains prepared by random mutagenesis was introduced into the transformed yeast cells. About 10⁶ colonies were obtained in medium lacking both leucine and tryptophan. Because the reporter plasmid and the zinc finger domain expression plasmids contain yeast *LEU2* and *TRP1* genes, respectively, as a marker, yeast cells were grown in medium lacking both leucine and tryptophan in order to select for cells that contain both the reporter and the zinc finger domain expression plasmid.

In one implementation, the library of zinc finger domains derived from the human genome was transformed into cells bearing the reporter plasmids. The transformation was performed on five different host cell strains, each strain containing one of five different target sequences operably linked to the reporter gene. About 10⁵ colonies were obtained per transformation in medium lacking both leucine and tryptophan. Transformants were grown on petri plates containing synthetic medium lacking leucine and tryptophan. After incubation, transformed cells were collected by applying a 10% sterile glycerol solution to the plates, scraping the colonies into the solution, and retrieving the solution. Cells were stored as frozen aliquots in the glycerol solution. A single aliquot was spread onto medium lacking leucine, tryptophan and histidine. 3-aminotriazole (AT) was added to the growth medium at the final concentrations of 0, 0.03, 0.1 and 0.3 mM. AT is a competitive inhibitor of His3 and titrates the sensitivity of the *HIS3* selection system. AT suppressed the basal activity of His3. Such basal activity can arise from leaky expression of the *HIS3* gene on the reporter plasmid. Out of about 10⁷ yeast cells spread on medium, on the order of hundreds of colonies grew in the selective medium lacking AT. The number of colonies gradually decreased as the concentration of AT increased. On the order of tens of colonies grew in the selective medium containing 0.3 mM of AT. Several colonies were randomly picked from the medium lacking AT and from the medium containing 0.3 mM of AT. Plasmids were isolated from yeast cells and transformed into *Escherichia coli* strain KC8 (*pyrF leuB600 trpC hisB463*). The plasmids encoding zinc finger transcription factor were isolated, and the DNA sequences of selected zinc finger domains were determined.

The amino acid sequence of each selected zinc finger domain was deduced from the DNA sequence. Each zinc finger domain was named after the four amino acid residues at base-contacting positions, namely positions -1, 2, 3, and 6 along the alpha-helix. The results are shown in Table 1. Identified zinc finger domains are named by the four amino acids found at base-contacting positions. Analysis of the sequences showed that in some cases the same zinc finger domain was obtained repeatedly. The numbers in the parenthesis in Table 1 represent how many times the same zinc finger domains have been obtained. For example, two zinc fingers having CSNR at the four base contacting positions were identified as binding the GAGC nucleic acid site (see column 3, "GAGC/human genome").

**Table 1**

| | | | | | | |
|---|---|---|---|---|---|---|
| Target Sequence | GAGC | GAGC | GCTT | GACT | GAGT | ACAT |
| origin of zinc finger domain library | random mutagenesis | human genome | human genome | human genome | human genome | human genome |
| amino acid | KTNR(2) | RTNR(2) | VSTR(9) | HSNK(2) | RDER(2) | QSTV(3) |
| residues at base | RTTR | RTNR | | CSNR(7) | SSNR(5) | |
| contacting | RPNR | CSNR(2) | | | | |
| positions* | HSNR | SSNR(3) | | | | |
| | RLKP | RSTV | | | | |
| | TRQR | SSGE | | | | |
| | TALH | | | | | |
| | RQKA | | | | | |
| | PARV | | | | | |
| | RTFR | | | | | |
| | RNNR | | | | | |
| | DPLH | | | | | |
| | RGNR | | | | | |

| | | | | | | |
|---|---|---|---|---|---|---|
| * The four-letter identifiers in the six columns to the right are the descriptors of the zinc finger domains isolated for each target sequence. Although these names are indicative of the amino acid residues at base contacting positions, they are not sequences of polypeptides. | | | | | | |

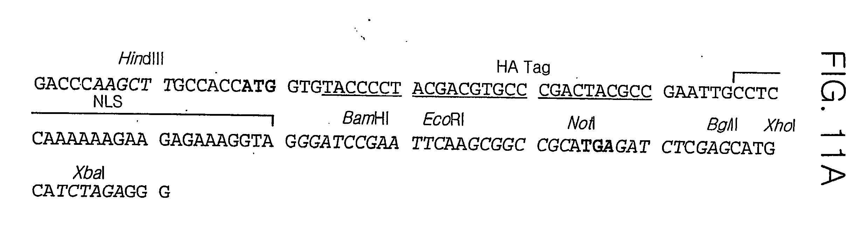

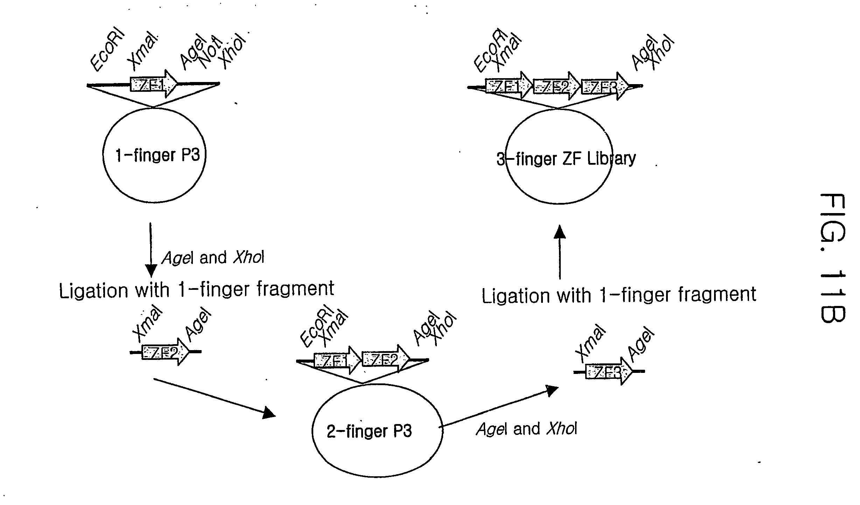

The full DNA sequences encoding selected human zinc finger domains and their translated amino acid sequences are shown in Fig. 11. The DNA sequence that is complementary to the degenerate PCR primers used to amplify DNA segments encoding zinc finger domains in the human genome is underlined. This sequence may differ from the original base sequence of reported human genome sequence due to either allelic differences or alterations introducing during amplification.

Most human zinc finger domains identified by screening in accordance with the present invention either were novel polypeptides or corresponded to anonymous open reading frames. For example, zinc finger domains designated as HSNK (contained in the sequence reported in GenBank accession number AF155100) and VSTR (contained in the sequence reported in GenBank accession number AF02577) are found in proteins whose function is as yet unknown. The results described herein not only indicate that these zinc finger domains are able to function as sequence-specific DNA-binding domains, but also document their preferred binding site preference in the context of chimeric proteins.

In addition, the present invention reveals that zinc finger domains obtained from the human genome can be used as modular building blocks to construct novel DNA-binding proteins. Human zinc finger domains of the present invention were obtained as a result of their functionality *in vivo* when connected to the C-terminus of finger 1 and finger 2 of Zif268. Thus, the identified zinc finger domains can recognize specific sequence in an artificial context, and are suitable as modular building blocks for designing synthetic transcription factors.

### Example 7: Pairwise Mating

To facilitate identification of zinc finger domains that bind to each 3 basepair target site, yeast mating was used to eliminate the need for repetitively transforming yeast cells and to search for positive binders to each of the 64 reporter constructs with a single transformation. Two yeast strains, YWI (*MATά* mating type) and YPH499 (*MATα* mating type), were used. YW1 was derived from yWAM2 by selecting a clone resistant to 5-fluoroorotic acid (FOA) in order to generate a *ura3-* derivative of yWAM2.

The plasmid library of zinc finger domains were introduced into the YW1 cells by yeast transformation. Cells from approximately 10⁶ independently transformed colonies were collected by scraping plates with a 10% glycerol solution. The solution was frozen in aliquots. Each pair of 64 reporter plasmids (derived from pLacZi or pRS315His) also was cotransfected into yeast strain YPH499. Transformants containing both reporter plasmids were harvested and frozen.

After thawing, the yeast cells were grown on minimal media to mid-log phase. The two cell types were then mixed and allowed to mate in YPD for 5 h. Diploid cells were selected on minimal media containing X-gal and AT (1 mM) but lacking tryptophan, leucine, uracil, and histidine. After several days, blue colonies that grew on the selective plate were isolated. The plasmids encoding zinc finger domains were isolated from blue colonies, and the DNA sequences of the selected zinc finger domains were determined.

The nucleic acids isolated from the blue colonies were individually retransformed into YW1 cells. For each isolated nucleic acid, retransformed YW1 cells were mated to YPH499 cells containing each of the 64 LacZ reporter plasmids in a 96-well plate, and then spread onto minimal media containing X-gal but lacking tryptophan and uracil. The DNA binding affinities and specificities of a zinc finger domain for 64 target sequences were determined by the intensity of blue color. Control experiments with the Zif268 zinc finger domains indicated that positive interactions between a zinc finger domain and a binding site yielded dark to pale blue colonies, (whose blue intensity is proportional to the binding affinity) and that negative interactions yielded white colonies.

### Example 8: Comparison of Identified Zinc Finger Domains with an Interaction Code

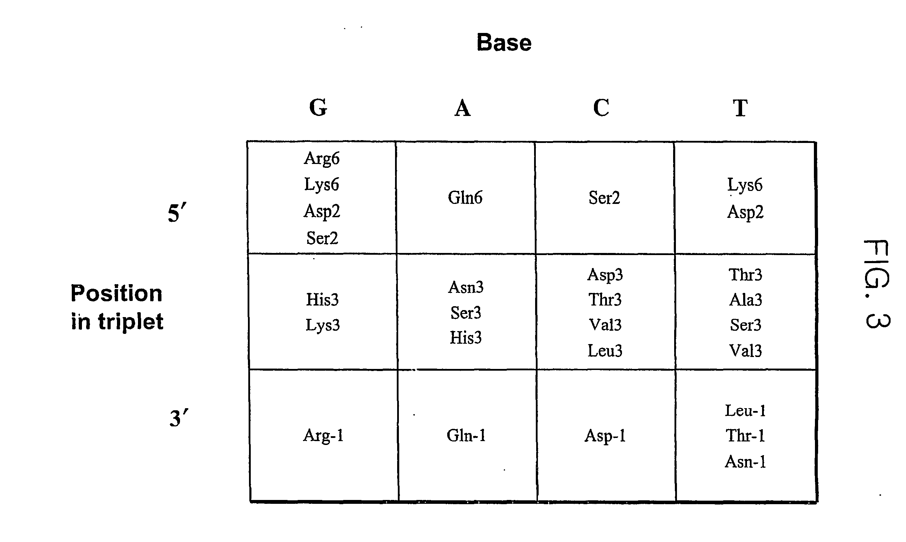

The amino acid residues of selected zinc finger domains at the critical base-contacting positions were compared with those anticipated from the zinc finger domain-DNA interaction code (Fig. 3). Most of zinc finger domains showed expected patterns, i.e. the amino acid residues at the critical positions match well those predicted from the code.

For example, the consensus amino acid residues in zinc finger domains selected from the library generated by random mutagenesis were R (Arg; 7 out of 14) or K (Lys; 2 out of 14) at position -1, N (Asp; 6 out of 14) at position 3, and R (9 out of 14) at position 6 (Table 1). These zinc finger domains were selected with the GAGC plasmid. (The reporter plasmid in which the composite binding sequence, 5'-GAGCGGGCG-3', is operably linked to the reporter gene is referred to as the GAGC plasmid. Likewise, the other reporter plasmids in which the sequence, 5'-XXXXGGGCG-3', is operably linked to the reporter gene are referred to as the XXXX plasmids.) These amino acid residues at critical base-contacting positions exactly match those expected from the code.

It is also known that amino acid residues at position 2 usually play only a minor role in base recognition (Pavletich and Pabo (1991) Science 252, 809-817). In some cases, however, position 2 may be more influential.

The amino acid residues in zinc finger domains obtained from the human genome also match those expected from the code quite well. For example, the consensus amino acid residues at position -1, 3, and 6 in the zinc finger domains obtained with the GAGC plasmid were R, N, and R, respectively (Table 1, column 3). These amino acids are exactly those anticipated from the code.

The amino acid residues at position -1, 3, and 6 in the zinc finger domain obtained with the GCTT plasmid were V, T, and R, respectively (Table 1, column 4). The T and R residues are exactly those expected from the code. The amino acid residues predicted from the code at position -1 that would interact with the base T (underlined) of the GCTT site are L, T or N. The VSTR zinc finger domain, which was selected with the GCTT plasmid, contained V (valine), a hydrophobic amino acid similar to L (leucine) at this position.

Overall, the amino acid residues in selected zinc finger domains match those predicted from the code at least at two positions out of the three critical positions. The amino acid residues in selected zinc finger domains that are expected from the code are underlined in Table 1. These results strongly suggest that the *in vivo* selection system disclosed herein functions as expected. However, as the *in vivo* selection and assay systems measure actual function of the zinc finger proteins in a cell, they may identify fingers with useful functions and DNA binding specificities that do not conform to theoretical expectations (e.g., the relationship depicted in FIG. 3).

### Example 9: Retransformation and Cross-transformation

To rule out the possibility of false positive results and to investigate the sequence specificity of the zinc finger protein described above, retransformation and cross-transformation of yeast cells were carried out using the isolated plasmids.

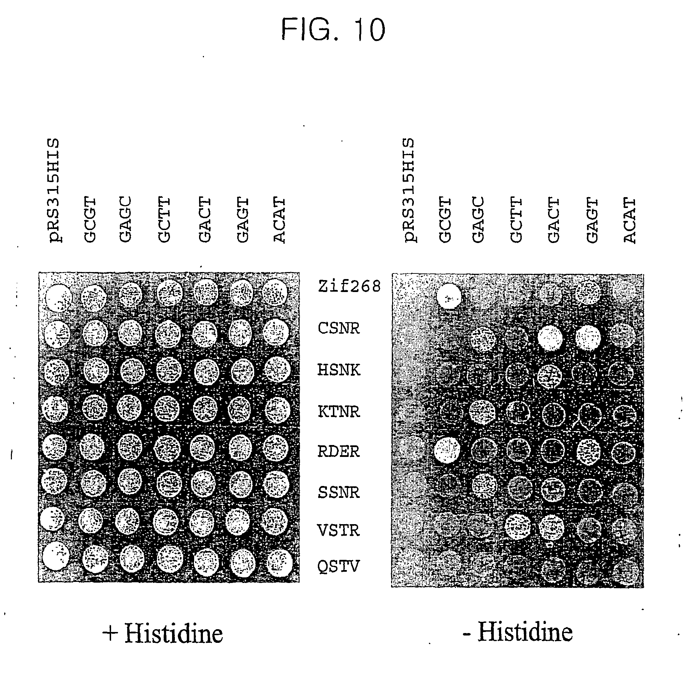

Yeast cells were first co-transformed with a reporter plasmid and a hybrid plasmid encoding a zinc finger domain. Yeast transformants were inoculated into minimal medium lacking leucine and tryptophan and incubated for 36 hours. About 1,000 cells in the growth medium were spotted directly onto solid medium lacking leucine, tryptophan, and histidine (designated as - histidine in Fig. 10) and onto solid medium lacking leucine and tryptophan (designated as + histidine in Fig. 10). These cells were then incubated for 50 hours at 30°C. The results are shown in Fig. 10.

It is expected that colonies can grow in the medium lacking histidine when the zinc finger moiety of the hybrid transcription factor binds the composite binding sequence, allowing the hybrid transcription factor to activate expression of the *HIS3* reporter gene. Colonies cannot grow in the medium lacking histidine when the zinc finger moiety of the transcription factor does not bind the composite binding sequence.

As shown in Fig. 10, the isolated zinc finger domains were capable of binding corresponding target sequences and showed sequence specificity markedly different from that of Zif268. Zif268 showed higher activity with the GCGT plasmid than with the other five plasmids, and relatively high activity with the GAGT plasmid. No colonies were formed by strains having reporters containing other binding sites and expressing the Zif268 protein.

The KTNR zinc finger domain isolated from the random mutant library was originally selected with the GAGC reporter plasmid. As expected, colonies were formed only with the GAGC plasmid. Zinc finger domains obtained from the library derived from the human genome also showed expected specificity. For example, HSNK, which had been selected with the GACT plasmid, allowed cell growth only with the GACT plasmid when retransformed into yeast cells. VSTR, which had been selected with the GCTT plasmid, showed the highest activity with the GCTT plasmid. RDER, which was selected with the GAGT plasmid, has the same amino acid residues at the four base-contacting positions as does finger 3 of Zif268. As expected, this zinc finger domain showed sequence specificity similar to that of finger 3. SSNR, selected with the GAGC and GAGT plasmids, allowed cell growth on histidine-deficient medium with the GAGC plasmid but not with the GAGT plasmid. QSTV, obtained with the ACAT plasmid, did not allow cell growth with any of the plasmids tested in this assay. However, this zinc finger domain was able to bind to the ACAT sequence tightly *in vitro* as demonstrated below.

### Example 10: Gel shift assays

Zinc finger proteins containing zinc finger domains selected using the modified one-hybrid system were expressed in *E. coli*, purified, and used in gel shift assays. The DNA segments encoding zinc finger proteins in the hybrid plasmids were isolated by digestion with *Stal*I and *Not*I and inserted into pGEX-4T2 (Pharmacia Biotech) between the *Sal*I and *Not*I sites. Zinc finger proteins were expressed in *E. coli* strain BL21 as fusion proteins connected to GST (Glutathione-S-transferase). The fusion proteins were purified using glutathione affinity chromatography (Pharmacia Biotech, Piscataway, NJ) and then digested with thrombin, which cleaves the connecting site between the GST moiety and zinc finger proteins. Purified zinc finger proteins contained finger 1 and finger 2 of Zif268 and selected zinc finger domains at the C-terminus.

The following probe DNAs were synthesized, annealed, labeled with ³²P using T4 polynucleotide kinase, and used in gel shift assays.
GCGT; 5'-CCGGGTCGCGCGTGGGCGGTACCG-3' (SEQ ID NO:90)
3'-CAGCGCGCACCCGCCATGGCAGCT-5' (SEQ ID NO:91)
GAGC; 5'-CCGGGTCGCGAGCGGGCGGTACCG-3' (SEQ ID NO:92)
3'-CAGCGCTCGCCCGCCATGGCAGCT-5' (SEQ ID NO:93)
GCTT; 5'-CCGGGTCGTGCTTGGGCGGTACCG-3' (SEQ ID NO:94)
3'-CAGCACGAACCCGCCATGGCAGCT-5' (SEQ ID NO:95)
GACT; 5'-CCGGGTCGGGACTGGGCGGTACCG-3' (SEQ ID NO:96)
3'-CAGCCCTGACCCGCCATGGCAGCT-5' (SEQ ID NO:97)
GAGT; 5'-CCGGGTCGGGAGTGGGCGGTACCG-3' (SEQ ID NO:98)
3'-CAGCCCTCACCCGCCATGGCAGCT-5' (SEQ ID NO:99)
ACAT; 5'-CCGGGTCGGACATGGGCGGTACCG-3' (SEQ ID NO:100)
3'-CAGCCTGTACCCGCCATGGCAGCT-5' (SEQ ID NO:101)

Various amounts of a zinc finger protein were incubated with a labeled probe DNA for one hour at room temperature in 20 mM Tris pH 7.7, 120 mM NaCl, 5 mM MgCl₂, 20 µM ZnSO₄, 10% glycerol, 0.1% Nonidet P-40, 5 mM DTT, and 0.10 mg/mL BSA (bovine serum albumin), and then the reaction mixtures were subjected to gel electrophoresis. The radioactive signals were quantitated by PhosphorImager™ analysis (Molecular Dynamics), and dissociation constants (*K*_{d}) were determined as described (Rebar and Pabo (1994) Science 263:671-673). The results are described in Table 2. All the constants were determined in at least two separate experiments, and the standard error of the mean is indicated. Cell growth of yeast transformants on histidine-deficient minimal medium (Fig. 10) is also indicated in Table 2.

**Table 2**

| Zinc finger protein | Probe DNA | Dissociation Constant (nM) | Growth of Yeast |
|---|---|---|---|
| Zif268 | GCTT | 2.1 ±0.3 | - |
| | GCGT | 0.024 ±0.004 | +++ |
| | GAGT | 0.17 ±0.04 | ++ |
| | GAGC | 2.3 ±0.9 | - |
| | GACT | 4.9 ±0.6 | - |
| | ACAT | 1.3. ±0.3 | - |
| KTNR | GCGT | 5.5 ±0.7 | - |
| | GAGC | 0.17 ±0.01 | ++ |
| | GACT | 30 ±1 | - |
| CSNR | GCGT | 2.7 ±0.3 | - |
| | GAGT | 0.46 ±0.04 | +++ |
| | GAGC | 1.2 ±0.1 | ++ |
| | GACT | 0.17 ±0.01 | +++ |
| HSNK | GCGT | 42 ±14 | - |
| | GAGT | 3.5 ±0.1 | - |
| | GACT | 0.32 ±0.08 | ++ |
| RDER | GCGT | 0.027 ±0.002 | +++ |
| | GAGT | 0.18 ±0.01 | ++ |
| | GACT | 28 ±9 | - |
| SSNR | GCGT | 3.8 ±1.3 | - |
| | GAGC | 0.45 ±0.09 | ++ |
| | GACT | 0.61 ±0.21 | + |
| VSTR | GCTT | 0.53 ±0.07 | ++ |
| | GCGT | 0.76 ±0.22 | - |
| | GAGT | 1.4 ±0.2 | - |
| QSTV | GCTT | 29 ±3 | - |
| | GCGT | 9.8 ±3.4 | - |
| | ACAT | 2.3 ±0.4 | - |

| | | | |
|---|---|---|---|
| * +++, 20 to 100% growth; ++, 5 to 20% growth; +, 1-5% growth; -, < 1% growth. | | | |

Zinc finger proteins that allowed cell growth on histidine-deficient plates bound the corresponding probe DNAs tightly. For example, the Zif268 protein used as a control allowed cell growth with the GCGT and GAGT reporter plasmids, and the dissociation constants measured *in vitro* using corresponding probe DNAs were 0.024 nM and 0.17 nM, respectively. In contrast, the Zif268 protein did not allow cell growth with other plasmids, and the dissociation constants measured using corresponding probe DNAs were higher than 1 nM.

Zinc finger proteins containing novel zinc finger domains also showed similar results. For example, the KTNR protein showed strong affinity for the GAGC probe DNA, with a dissociation constant of 0.17 nM, but not for the GCGT or GACT probe DNA, with dissociation constants of 5.5 nM or 30 nM, respectively. This protein allowed cell growth only with the GAGC plasmid. The HSNK protein was able to bind the GACT probe DNA tightly (*K*_{d} = 0.32 nM) but not the GCGT or GAGT probe DNA; as would be expected, the HSNK protein allowed cell growth only with the GACT plasmid.

The QSTV protein, which was selected with the ACAT reporter plasmid, was not able to promote cell growth with any of the other reporter plasmids when retransformed into yeast. Gel shift assays demonstrated that this protein bound the ACAT probe DNA more tightly than it did the other probe DNAs. That is, QSTV bound the ACAT probe DNA 13 times or 4.3 times stronger than it did the GCTT or GCGT probe DNA respectively.

In general, when a zinc finger protein, e.g., having three zinc finger domains, binds a DNA sequence with a dissociation constant lower than 1 nM, it allows cell growth, whereas when a zinc finger protein binds a DNA sequence with a dissociation constant higher than 1 nM, it does not allow cell growth. Zinc finger proteins that bind with a dissociation constant of greater than 1 nM but less than 5 nM can also be useful, e.g., in the context of a chimeric zinc finger protein having four zinc finger domains.

### Example 11: TGZFD-001 "CSNRL"

TG-ZFD-001 "CSNR1" was identified by *in vivo* screening from human genomic sequence. Its amino acid sequence is YKCKQCGKAFGCPSNLRRHGRTH (SEQ ID NO:23). It is encoded by the human nucleic acid sequence:

As a polypeptide fusion to fingers 1 and 2 of Zif268, TG-ZFD-001 "CSNR1" demonstrates recognition specificity for the 3-bp target sequence sequences GAA, GAC, and GAG. Its binding site preference is GAA > GAC > GAG > GCG as determined by *in vivo* screening results and EMSA. In EMSA, the TG-ZFD-001 "CSNR" fusion to fingers 1 and 2 of Zif268 and the GST purification handles has an apparent K_{d} of 0.17 nM for the GAC containing site, 0.46 nM for the GAG containing site, and 2.7 nM for the GCG containing site.

TG-ZFD-001 "CSNR" can be used as a module to construct a chimeric DNA binding protein comprising multiple zinc finger domains, e.g., for the purpose of recognizing a DNA site containing the sequence GAA, GAC, or GAG.

### Example 12: TG-ZFD-002 "HSNK"

TG-ZFD-002 "HSNK" was identified by *in vivo* screening from human genomic sequence. Its amino acid sequence is: YKCKECGKAFNHSSNFNKHHRIH (SEQ ID NO:25). It is encoded by the human nucleic acid sequence:

As a polypeptide fusion to fingers 1 and 2 of Zif268, TG-ZFD-002 "HSNK" demonstrates recognition specificity for the 3-bp target sequence GAC. Its binding site preference is GAC > GAG > GCG as determined by *in vivo* screening results and EMSA. In EMSA, the TG-ZFD-002 "HSNK" fusion to fingers 1 and 2 of Zif268 and the GST purification handles has an apparent K_{d} of 0.32 nM for the GAC containing site, 3.5 nM for the GAG containing site, and 42 nM for the GCG containing site.

TG-ZFD-002 "HSNK" can be used as a module to construct a chimeric DNA binding protein comprising multiple zinc finger domains, e.g., for the purpose of recognizing a DNA site containing the sequence GAC.

### Example 13: TG-ZFD-003 "SSNR"

TG-ZFD-003 "SSNR" was identified by *in vivo* screening from human genomic sequence. Its amino acid sequence is: YECKECGKAFSSGSNFTRHQRIH (SEQ ID NO:27). It is encoded by the human nucleic acid sequence:

As a polypeptide fusion to fingers 1 and 2 of Zif268, TG-ZFD-003 "SSNR" demonstrates recognition specificity for the 3-bp target sequence GAG. Its binding site preference is GAG > GAC > GCG as determined by *in vivo* screening results and EMSA. In EMSA, the TG-ZFD-003 "SSNR" fusion to fingers 1 and 2 of Zif268 and the GST purification handles has an apparent K_{d} of 0.45 nM for the GAG containing site, 0.61 nM for the GAC containing site, and 3.8 nM for the GCG containing site.

TG-ZFD-003 "SSNR" can be used as a module to construct a chimeric DNA binding protein comprising multiple zinc finger domains, e.g., for the purpose of recognizing a DNA site containing the sequence GAG, or GAC.

### Example 14: TGZFD-004 "RDER1"

TG-ZFD-004 "RDER1" was identified by *in vivo* screening from human genomic sequence. Its amino acid sequence is: YVCDVEGCTWKFARSDELNRHKKRH (SEQ ID NO:29). It is encoded by the human nucleic acid sequence:

As a polypeptide fusion to fingers 1 and 2 of Zif268, TG-ZFD-004 "RDER1" demonstrates recognition specificity for the 3-bp target sequence GCG. Its binding site preference is GCG > GTG, GAG > GAC as determined by *in vivo* screening results and EMSA. In EMSA, the TG-ZFD-004 "RDER1" fusion to fingers 1 and 2 of Zif268 and the GST purification handles has an apparent K_{d} of 0.027 nM for the GCG containing site, 0.18 nM for GAG containing site, and 28 nM for the GAC containing site.

TG-ZFD-004 "RDER1" can be used as a module to construct a chimeric DNA binding protein comprising multiple zinc finger domains, e.g., for the purpose of recognizing a DNA site containing the sequence GCG, GTG or GAG.

### Example 15: TGZFD-005 "QSTV"

TG-ZFD-005 "QSTV" was identified by *in vivo* screening from human genomic sequence. Its amino acid sequence is: YECNECGKAFAQNSTLRVHQRIH (SEQ ID NO:31). It is encoded by the human nucleic acid sequence:

As a polypeptide fusion to fingers 1 and 2 of Zif268, TG-ZFD-005 "QSTV" demonstrates recognition specificity for the 3-bp target sequence ACA. Its binding site preference is ACA > GCG > GCT as determined by EMSA. In EMSA, the TG-ZFD-005 "QSTV" fusion to fingers 1 and 2 of Zif268 and the GST purification handles has an apparent K_{d} of 2.3 nM for the ACA containing site, 9.8 nM for the GCG containing site, and 29 nM for the GCT containing site.

TG-ZFD-005 "QSTV" can be used as a module to construct a chimeric DNA binding protein comprising multiple zinc finger domains, e.g., for the purpose of recognizing a DNA site containing the sequence ACA.

### Example 16: TGZFD-006 "VSTR"

TG-ZFD-006 "VSTR" was identified by *in vivo* screening from human genomic sequence. Its amino acid sequence is: YECNYCGKTFSVSSTLIRHQRIH (SEQ ID NO:33). It is encoded by the human nucleic acid sequence:

As a polypeptide fusion to fingers 1 and 2 of Zif268, TG-ZFD-006 "VSTR" demonstrates recognition specificity for the 3-bp target sequence GCT. Its binding site preference is GCT > GCG > GAG as determined by *in vivo* screening results and EMSA. In EMSA, the TG-ZFD-006 "VSTR" fusion to fingers 1 and 2 of Zif268 and the GST purification handles has an apparent K_{d} of 0.53 nM for the GCT containing site, 0.76 for the GCG containing site, and 1.4 nM for the GAG containing site.

TG-ZFD-006 "VSTR" can be used as a module to construct a chimeric DNA binding protein comprising multiple zinc finger domains, e.g., for the purpose of recognizing a DNA site containing the sequence GCT or GCG.

### Example 17: TG-ZFD-007 "CSNR2"

TG-ZFD-007 "CSNR2" was identified by *in vivo* screening from human genomic sequence. Its amino acid sequence is: YQCNICGKCFSCNSNLHRHQRTH (SEQ ID NO:35). It is encoded by the human nucleic acid sequence:

As a polypeptide fusion to fingers 1 and 2 of Zif268, TG-ZFD-007 "CSNR2" demonstrates recognition specificity for 3-bp target sequences GAA, GAC, and GAG. Its binding site preference is GAA > GAC > GAG as determined by *in vivo* screening results.

TG-ZFD-007 "CSNR2" can be used as a module to construct a chimeric DNA binding protein comprising multiple zinc finger domains, e.g., for the purpose of recognizing a DNA site containing the sequence GAA, GAC, or GAG.

### Example 18: TG-ZFD-008 "QSHR1"

TG-ZFD-008 "QSHR1" was identified by *in vivo* screening from human genomic sequence. Its amino acid sequence is: YACHLCGKAFTQSSHLRRHEKTH (SEQ ID NO:37). It is encoded by the human nucleic acid sequence:

As a polypeptide fusion to fingers 1 and 2 of Zif268, TG-ZFD-008 "QSHR1" demonstrates recognition specificity for 3-bp target sequences GGA, GAA, and AGA. Its binding site preference is GGA > GAA > AGA as determined by *in vivo* screening results.

TG-ZFD-008 "QSHR1" can be used as a module to construct a chimeric DNA binding protein comprising multiple zinc finger domains, e.g., for the purpose of recognizing a DNA site containing the sequence GGA, GAA, or AGA.

### Example 19: TGZFD-009 "QSHR2"

TG-ZFD-009 "QSHR2" was identified by *in vivo* screening from human genomic sequence. Its amino acid sequence is: YKCGQCGKFYSQVSHLTRHQKIH (SEQ ID NO:39). It is encoded by the human nucleic acid sequence:

As a polypeptide fusion to fingers 1 and 2 of Zif268, TG-ZFD-009 "QSHR2" demonstrates recognition specificity for the 3-bp target sequence GGA.

TG-ZFD-009 "QSHR2" can be used as a module to construct a chimeric DNA binding protein comprising multiple zinc finger domains, e.g., for the purpose of recognizing a DNA site containing the sequence GGA.

### Example 20: TG-ZFD-010 "QSHR3"

TG-ZFD-010 "QSHR3" was identified *by in vivo* screening from human genomic sequence. Its amino acid sequence is: YACHLCGKAFTQCSHLRRHEKTH (SEQ ID NO:41). It is encoded by the human nucleic acid sequence:

As a polypeptide fusion to fingers 1 and 2 of Zif268, TG-ZFD-010 "QSHR3" demonstrates recognition specificity for 3-bp target sequences GGA and GAA. Its binding site preference is GGA > GAA as determined by *in vivo* screening results.

TG-ZFD-010 "QSHR3" can be used as a module to construct a chimeric DNA binding protein comprising multiple zinc finger domains, e.g., for the purpose of recognizing a DNA site containing the sequence GGA or GAA.

### Example 21: TG-ZFD-011 "QSHR4"

TG-ZFD-011 "QSHR4" was identified *by in vivo* screening from human genomic sequence. Its amino acid sequence is: YACHLCAKAFIQCSHLRRHEKTH (SEQ ID NO:43). It is encoded by the human nucleic acid sequence:

As a polypeptide fusion to fingers 1 and 2 of Zif268, TG-ZFD-011 "QSHR4" demonstrates recognition specificity for 3-bp target sequences GGA and GAA. Its binding site preference is GGA > GAA as determined by *in vivo* screening results.

TG-ZFD-011 "QSHR4" can be used as a module to construct a chimeric DNA binding protein comprising multiple zinc finger domains, e.g., for the purpose of recognizing a DNA site containing the sequence GGA or GAA.

### Example 22: TG-ZFD-012 "RSHR5"

TG-ZFD-012 "QSHR5" was identified *by in vivo* screening from human genomic sequence. Its amino acid sequence is: YVCRECGRGFRQHSHLVRHKRTH (SEQ ID NO:45). It is encoded by the human nucleic acid sequence:

As a polypeptide fusion to fingers 1 and 2 of Zif268, TG-ZFD-012 "QSHR5" demonstrates recognition specificity for 3-bp target sequences GGA, AGA, GAA, and CGA. Its binding site preference is GGA > AGA > GAA > CGA as determined by *in vivo* screening results.

TG-ZFD-012 "QSHR5" can be used as a module to construct a chimeric DNA binding protein comprising multiple zinc finger domains, e.g., for the purpose of recognizing a DNA site containing the sequence GGA, AGA, GAA, or CGA.

### Example 23: TG-ZFD-013 "QSNR1"

TG-ZFD-013 "QSNR1" was identified by *in vivo* screening from human genomic sequence. Its amino acid sequence is: FECKDCGKAFIQKSNLIRHQRTH (SEQ ID NO:47). It is encoded by the human nucleic acid sequence:

As a polypeptide fusion to fingers 1 and 2 of Zif268, TG-ZFD-013 "QSNR1" demonstrates recognition specificity for the 3-bp target sequence GAA.

TG-ZFD-013 "QSNR1" can be used as a module to construct a chimeric DNA binding protein comprising multiple zinc finger domains, e.g., for the purpose of recognizing a DNA site containing the sequence GAA.

### Example 24: TG-ZFD-014 "RSNR2"

TG-ZFD-014 "QSNR2" was identified by *in vivo* screening from human genomic sequence. Its amino acid sequence is: YVCRECRRGFSQKSNLIRHQRTH (SEQ ID NO:49). It is encoded by the human nucleic acid sequence:

As a polypeptide fusion to fingers 1 and 2 of Zif268, TG-ZFD-014 "QSNR2" demonstrates recognition specificity for the 3-bp target sequence GAA.

TG-ZFD-014 "QSNR2" can be used as a module to construct a chimeric DNA binding protein comprising multiple zinc finger domains, e.g., for the purpose of recognizing a DNA site containing the sequence GAA.

### Example 25: TG-ZFD-015 "QSNV1"

TG-ZFD-015 "QSNV1" was identified by *in vivo* screening from human genomic sequence. Its amino acid sequence is: YECNTCRKTFSQKSNLIVHQRTH (SEQ ID NO:51). It is encoded by the human nucleic acid sequence:

As a polypeptide fusion to fingers 1 and 2 of Zif268, TG-ZFD-015 "QSNV1" demonstrates recognition specificity for 3-bp target sequences AAA and CAA. Its binding site preference is AAA > CAA as determined by *in vivo* screening results.

TG-ZFD-015 "QSNV1" can be used as a module to construct a chimeric DNA binding protein comprising multiple zinc finger domains, e.g., for the purpose of recognizing a DNA site containing the sequence AAA or CAA.

### Example 26: TGZFD-016 "QSNV2"

TG-ZFD-016 "QSNV2" was identified by *in vivo* screening from human genomic sequence. Its amino acid sequence is: YVCSKCGKAFTQSSNLTVHQKIH (SEQ ID NO:53). It is encoded by the human nucleic acid sequence:

As a polypeptide fusion to fingers 1 and 2 of Zif268, TG-ZFD-016 "QSNV2" demonstrates recognition specificity for 3-bp target sequences AAA and CAA. Its binding site preference is AAA > CAA as determined by *in vivo* screening results.

TG-ZFD-016 "QSNV2" can be used as a module to construct a chimeric DNA binding protein comprising multiple zinc finger domains, e.g., for the purpose of recognizing a DNA site containing the sequence AAA or CAA.

### Example 27: TG-ZFD-017 "QSNV3"

TG-ZFD-017 "QSNV3" was identified by *in vivo* screening from human genomic sequence. Its amino acid sequence is: YKCDECGKNFTQSSNLIVHKRIH (SEQ ID NO:55). It is encoded by the human nucleic acid sequence:

As a polypeptide fusion to fingers 1 and 2 of Zif268, TG-ZFD-017 "QSNV3" demonstrates recognition specificity for a 3-bp target sequence AAA.

TG-ZFD-017 "QSNV3" can be used as a module to construct a chimeric DNA binding protein comprising multiple zinc finger domains, e.g., for the purpose of recognizing a DNA site containing the sequence AAA.

### Example 28: TGZFD-018 "QSNV4"

TG-ZFD-018 "QSNV4" was identified by *in vivo* screening from human genomic sequence. Its amino acid sequence is: YECDVCGKTFTQKSNLGVHQRTH (SEQ ID NO:57). It is encoded by the human nucleic acid sequence:

As a polypeptide fusion to fingers 1 and 2 of Zif268, TG-ZFD-018 "QSNV4" demonstrates recognition specificity for the 3-bp target sequence AAA.

TG-ZFD-018 "QSNV4" can be used as a module to construct a chimeric DNA binding protein comprising multiple zinc finger domains, e.g., for the purpose of recognizing a DNA site containing the sequence AAA.

### Example 29: TG-ZFD-019 "QSSR1"

TG-ZFD-019 "QSSR1" was identified by *in vivo* screening from human genomic sequence. Its amino acid sequence is: YKCPDCGKSFSQSSSLIRHQRTH (SEQ ID NO:59). It is encoded by the human nucleic acid sequence:

As a polypeptide fusion to fingers 1 and 2 of Zif268, TG-ZFD-019 "QSSR1" demonstrates recognition specificity for 3-bp target sequences GTA and GCA. Its binding site preference is GTA > GCA as determined by *in vivo* screening results.

TG-ZFD-019 "QSSR1" can be used as a module to construct a chimeric DNA binding protein comprising multiple zinc finger domains, e.g., for the purpose of recognizing a DNA site containing the sequence GTA or GCA.

### Example 30: TGZFD-020 "QSSR2"

TG-ZFD-020 "QSSR2" was identified by *in vivo* screening from human genomic sequence. Its amino acid sequence is: YECQDCGRAFNQNSSLGRHKRTH (SEQ ID NO:61). It is encoded by the human nucleic acid sequence:

As a polypeptide fusion to fingers 1 and 2 of Zif268, TG-ZFD-020 "QSSR2" demonstrates recognition specificity for the 3-bp target sequence GTA.

TG-ZFD-020 "QSSR2" can be used as a module to construct a chimeric DNA binding protein comprising multiple zinc finger domains, e.g., for the purpose of recognizing a DNA site containing the sequence GTA.

### Example 31: TGZFD-021 "QSTR"

TG-ZFD-021 "QSTR" was identified by *in vivo* screening from human genomic sequence. Its amino acid sequence is: YKCEECGKAFNQSSTLTRHKIVH (SEQ ID NO:63). It is encoded by the human nucleic acid sequence:

As a polypeptide fusion to fingers 1 and 2 of Zif268, TG-ZFD-021 "QSTR" demonstrates recognition specificity for 3-bp target sequences GTA and GCA. Its binding site preference is GTA > GCA as determined by *in vivo* screening results.

TG-ZFD-021 "QSTR" can be used as a module to construct a chimeric DNA binding protein comprising multiple zinc finger domains, e.g., for the purpose of recognizing a DNA site containing the sequence GTA or GCA.

### Example 32: TG-ZFD-022 "RSHR"

TG-ZFD-022 "RSHR" was identified by *in vivo* screening from human genomic sequence. Its amino acid sequence is: YKCMECGKAFNRRSHLTRHQRIH (SEQ ID NO:65). It is encoded by the human nucleic acid sequence:

As a polypeptide fusion to fingers 1 and 2 of Zif268, TG-ZFD-022 "RSHR" demonstrates recognition specificity for the 3-bp target sequence GGG.

TG-ZFD-022 "RSHR" can be used as a module to construct a chimeric DNA binding protein comprising multiple zinc finger domains, e.g., for the purpose of recognizing a DNA site containing the sequence GGG.

### Example 33: TG-ZFD-023 "VSSR"

TG-ZFD-023 "VSSR" was identified by *in vivo* screening from human genomic sequence. Its amino acid sequence is: YTCKQCGKAFSVSSSLRRHETTH (SEQ ID NO:67). It is encoded by the human nucleic acid sequence:

As a polypeptide fusion to fingers 1 and 2 of Zif268, TG-ZFD-023 "VSSR" demonstrates recognition specificity for 3-bp target sequences GTT, GTG, and GTA. Its binding site preference is GTT > GTG > GTA as determined by *in vivo* screening results.

TG-ZFD-023 "VSSR" can be used as a module to construct a chimeric DNA binding protein comprising multiple zinc finger domains, e.g., for the purpose of recognizing a DNA site containing the sequence GTT, GTG, or GTA.

### Example 34: TG-ZFD-024 "QAHR"

TG-ZFD-024 "QAHR" was identified by *in vivo* screening from human genomic sequence. Its amino acid sequence is: YKCKECGQAFRQRAHLIRHHKLH (SEQ ID NO:103). It is encoded by the human nucleic acid sequence:

As a polypeptide fusion to fingers 1 and 2 of Zif268, TG-ZFD-024 "QAHR" demonstrates recognition specificity for the 3-bp target sequence GGA as determined by *in vivo* screening results.

TG-ZFD-024 "QAHR" can be used as a module to construct a chimeric DNA binding protein comprising multiple zinc finger domains, e.g., for the purpose of recognizing a DNA site containing the sequence GGA

### Example 35: TGZFD-025 "QFNR"

TG-ZFD-025 "QFNR" was identified by *in vivo* screening from human genomic sequence. Its amino acid sequence is: YKCHQCGKAFIQSFNLRRHERTH (SEQ ID NO:105). It is encoded by the human nucleic acid sequence:

As a polypeptide fusion to fingers 1 and 2 of Zif268, TG-ZFD-025 "QFNR" demonstrates recognition specificity for the 3-bp target sequence GAG as determined by *in vivo* screening results.

TG-ZFD-025 "QFNR" can be used as a module to construct a chimeric DNA binding protein comprising multiple zinc finger domains, e.g., for the purpose of recognizing a DNA site containing the sequence GAG.

### Example 36: TGZFD-026 "QGNR"

TG-ZFD-026 "QGNR" was identified by *in vivo* screening from human genomic sequence. Its amino acid sequence is: FQCNQCGASFTQKGNLLRHIKLH (SEQ ID NO:107). It is encoded by the human nucleic acid sequence:

As a polypeptide fusion to fingers 1 and 2 of Zif268, TG-ZFD-026 "QGNR" demonstrates recognition specificity for the 3-bp target sequence GAA as determined by *in vivo* screening results.

TG-ZFD-026 "QGNR" can be used as a module to construct a chimeric DNA binding protein comprising multiple zinc finger domains, e.g., for the purpose of recognizing a DNA site containing the sequence GAA.

### Example 37: TG-ZFD-028 "QSHT"

TG-ZFD-028 "QSHT" was identified by *in vivo* screening from human genomic sequence. Its amino acid sequence is: YKCEECGKAFRQSSHLTTHKIIH (SEQ ID NO:111). It is encoded by the human nucleic acid sequence:

As a polypeptide fusion to fingers 1 and 2 of Zif268, TG-ZFD-028 "QSHT" demonstrates recognition specificity for the 3-bp target sequence AGA, CGA, TGA, and GGA. Its binding site preference is (AGA and CGA) > TGA > GGA as determined by *in vivo* screening results.

TG-ZFD-028 "QSHT" can be used as a module to construct a chimeric DNA binding protein comprising multiple zinc finger domains, e.g., for the purpose of recognizing a DNA site containing the sequence AGA, CGA, TGA, and GGA.

### Example 38: TG-ZFD-029 "QSHV"

TG-ZFD-029 "QSHV" was identified by *in vivo* screening from human genomic sequence. Its amino acid sequence is: YECDHCGKSFSQSSHLNVHKRTH (SEQ ID NO:113). It is encoded by the human nucleic acid sequence:

As a polypeptide fusion to fingers 1 and 2 of Zif268, TG-ZFD-029 "QSHV" demonstrates recognition specificity for the 3-bp target sequence CGA, AGA, and TGA. Its binding site preference is CGA > AGA > TGA as determined by *in vivo* screening results.

TG-ZFD-029 "QSHV" can be used as a module to construct a chimeric DNA binding protein comprising multiple zinc finger domains, e.g., for the purpose of recognizing a DNA site containing the sequence CGA, AGA, and TGA.

### Example 39: TGZFD-030 "QSNI"

TG-ZFD-030 "QSNI" was identified by *in vivo* screening from human genomic sequence. Its amino acid sequence is: YMCSECGRGFSQKSNLIIHQRTH (SEQ ID NO:115). It is encoded by the human nucleic acid sequence:

As a polypeptide fusion to fingers 1 and 2 of Zif268, TG-ZFD-030 "QSNI" demonstrates recognition specificity for the 3-bp target sequence AAA and CAA as determined by *in vivo* screening results.

TG-ZFD-030 "QSNI" can be used as a module to construct a chimeric DNA binding protein comprising multiple zinc finger domains, e.g., for the purpose of recognizing a DNA site containing the sequence AAA or CAA.

### Example 40: TG-ZFD-031 "QSNR3"

TG-ZFD-031 "QSNR3" was identified by *in vivo* screening from human genomic sequence. Its amino acid sequence is: YECEKCGKAFNQSSNLTRHKKSH (SEQ ID NO:117). It is encoded by the human nucleic acid sequence:

As a polypeptide fusion to fingers 1 and 2 of Zif268, TG-ZFD-031 "QSNR3" demonstrates recognition specificity for the 3-bp target sequence GAA as determined by *in vivo* screening results.

TG-ZFD-031 "QSNR3" can be used as a module to construct a chimeric DNA binding protein comprising multiple zinc finger domains, e.g., for the purpose of recognizing a DNA site containing the sequence GAA.

### Example 41: TGZFD-032 "QSSR3"

TG-ZFD-032 "QSSR3" was identified by in vivo screening from human genomic sequence. Its amino acid sequence is:YECNECGKFFSQSSSLIRHRRSH (SEQ ID NO:119). It is encoded by the human nucleic acid sequence:

As a polypeptide fusion to fingers 1 and 2 of Zif268, TG-ZFD-032 "QSSR3" demonstrates recognition specificity for the 3-bp target sequence GTA and GCA. Its binding site preference is GTA > GCA as determined by in vivo screening results.

TG-ZFD-032 "QSSR3" can be used as a module to construct a chimeric DNA binding protein comprising multiple zinc finger domains, e.g., for the purpose of recognizing a DNA site containing the sequence GTA or GCA.

### Example 42: TGZFD-033 "QTHQ"

TG-ZFD-033 "QTHQ" was identified by *in vivo* screening from human genomic sequence. Its amino acid sequence is: YECHDCGKSFRQSTHLTQHRRIH (SEQ ID NO:121). It is encoded by the human nucleic acid sequence:

As a polypeptide fusion to fingers 1 and 2 of Zif268, TG-ZFD-033 "QTHQ" demonstrates recognition specificity for the 3-bp target sequence AGA, TGA, and CGA. Its binding site preference is AGA > (TGA and CGA) as determined by *in vivo* screening results.

TG-ZFD-033 "QTHQ" can be used as a module to construct a chimeric DNA binding protein comprising multiple zinc finger domains, e.g., for the purpose of recognizing a DNA site containing the sequence AGA, TGA, and CGA.

### Example 43: TG-ZFD-034 "QTHR1"

TG-ZFD-034 "QTHR1" was identified by *in vivo* screening from human genomic sequence. Its amino acid sequence is: YECHDCGKSFRQSTHLTRHRRIH (SEQ ID NO:123). It is encoded by the human nucleic acid sequence:

As a polypeptide fusion to fingers 1 and 2 of Zif268, TG-ZFD-034 "QTHR1" demonstrates recognition specificity for the 3-bp target sequence GGA, GAA, and AGA. Its binding site preference is GGA > (GAA and AGA) as determined by *in vivo* screening results.

TG-ZFD-034 "QTHR1" can be used as a module to construct a chimeric DNA binding protein comprising multiple zinc finger domains, e.g., for the purpose of recognizing a DNA site containing the sequence GGA, GAA, and AGA.

### Example 44: TG-ZFD-035 "QTHR2"

TG-ZFD-035 "QTHR2" was identified by *in vivo* screening from human genomic sequence. Its amino acid sequence is: HKCLECGKCFSQNTHLTRHQRT (SEQ ID NO:125). It is encoded by the human nucleic acid sequence:

As a polypeptide fusion to fingers 1 and 2 of Zif268, TG-ZFD-035 "QTHR2" demonstrates recognition specificity for the 3-bp target sequence GGA as determined by *in vivo* screening results.

TG-ZFD-035 "QTHR2" can be used as a module to construct a chimeric DNA binding protein comprising multiple zinc finger domains, e.g., for the purpose of recognizing a DNA site containing the sequence GGA.

### Example 45: TG-ZFD-036 "RDER2"

TG-ZFD-036 "RDER2" was identified *by in vivo* screening from human genomic sequence. Its amino acid sequence is: YHCDWDGCGWKFARSDELTRHYRKH (SEQ ID NO:127). It is encoded by the human nucleic acid sequence:

As a polypeptide fusion to fingers 1 and 2 of Zif268, TG-ZFD-036 "RDER2" demonstrates recognition specificity for the 3-bp target sequence GCG and GTG. Its binding site preference is GCG > GTG as determined by *in vivo* screening results.

TG-ZFD-036 "RDER2" can be used as a module to construct a chimeric DNA binding protein comprising multiple zinc finger domains, e.g., for the purpose of recognizing a DNA site containing the sequence GCG and GTG.

### Example 46: TG-ZFD-037 "RDER3"

TG-ZFD-037 "RDER3" was identified by *in vivo* screening from human genomic sequence. Its amino acid sequence is: YRCSWEGCEWRFARSDELTRHFRKH (SEQ ID NO:129). It is encoded by the human nucleic acid sequence:

As a polypeptide fusion to fingers 1 and 2 of Zif268, TG-ZFD-037 "RDER3" demonstrates recognition specificity for the 3-bp target sequence GCG and GTG as determined by *in vivo* screening results.

TG-ZFD-037 "RDER3" can be used as a module to construct a chimeric DNA binding protein comprising multiple zinc finger domains, e.g., for the purpose of recognizing a DNA site containing the sequence GCG and GTG.

### Example 47: TG-ZFD-038 "RDER4"

TG-ZFD-038 "RDER4" was identified *by in vivo* screening from human genomic sequence. Its amino acid sequence is: FSCSWKGCERRFARSDELSRHRRTH (SEQ ID NO:131). It is encoded by the human nucleic acid sequence:

As a polypeptide fusion to fingers 1 and 2 of Zif268, TG-ZFD-038 "RDER4" demonstrates recognition specificity for the 3-bp target sequence GCG and GTG as determined by *in vivo* screening results.

TG-ZFD-038 "RDER4" can be used as a module to construct a chimeric DNA binding protein comprising multiple zinc finger domains, e.g., for the purpose of recognizing a DNA site containing the sequence GCG and GTG.

### Example 48: TG-ZFD-039 "RDER5"

TG-ZFD-039 "RDER5" was identified by *in vivo* screening from human genomic sequence. Its amino acid sequence is: FACSWQDCNKKFARSDELARHYRTH (SEQ ID NO:133). It is encoded by the human nucleic acid sequence:

As a polypeptide fusion to fingers 1 and 2 of Zif268, TG-ZFD-039 "RDER5" demonstrates recognition specificity for the 3-bp target sequence GCG as determined *by in vivo* screening results.

TG-ZFD-039 "RDER5" can be used as a module to construct a chimeric DNA binding protein comprising multiple zinc finger domains, e.g., for the purpose of recognizing a DNA site containing the sequence GCG.

### Example 49: TG-ZFD-040 "RDER6"

TG-ZFD-040 "RDER6" was identified *by in vivo* screening from human genomic sequence. Its amino acid sequence is: YHCNWDGCGWKFARSDELTRHYRKH (SEQ ID NO:135). It is encoded by the human nucleic acid sequence:

As a polypeptide fusion to fingers 1 and 2 of Zif268, TG-ZFD-040 "RDER6" demonstrates recognition specificity for the 3-bp target sequence GCG and GTG. Its binding site preference is GCG > GTG as determined by *in vivo* screening results.

TG-ZFD-040 "RDER6" can be used as a module to construct a chimeric DNA binding protein comprising multiple zinc finger domains, e.g., for the purpose of recognizing a DNA site containing the sequence GCG and GTG.

### Example 50: TG-ZFD-041 "RDHR1"

TG-ZFD-041 "RDHR1" was identified by *in vivo* screening from human genomic sequence. Its amino acid sequence is: FLCQYCAQRFGRKDHLTRHMKKSH (SEQ ID NO:137). It is encoded by the human nucleic acid sequence:

As a polypeptide fusion to fingers 1 and 2 of Zif268, TG-ZFD-041 "RDHR1" demonstrates recognition specificity for the 3-bp target sequence GAG and GGG as determined by *in vivo* screening results.

TG-ZFD-041 "RDHR1" can be used as a module to construct a chimeric DNA binding protein comprising multiple zinc finger domains, e.g., for the purpose of recognizing a DNA site containing the sequence GAG and GGG.

### Example 51: TG-ZFD-043 "RDHT"

TG-ZFD-043 "RDHT" was identified by *in vivo* screening from human genomic sequence. Its amino acid sequence is: FQCKTCQRKFSRSDHLKTHTRTH (SEQ ID NO:141). It is encoded by the human nucleic acid sequence:

As a polypeptide fusion to fingers 1 and 2 of Zif268, TG-ZFD-043 "RDHT" demonstrates recognition specificity for the 3-bp target sequence TGG, AGG, CGG, and GGG as determined by *in vivo* screening results.

TG-ZFD-043 "RDHT" can be used as a module to construct a chimeric DNA binding protein comprising multiple zinc finger domains, e.g., for the purpose of recognizing a DNA site containing the sequence TGG, AGG, CGG, and GGG.

### Example 52: TG-ZFD-044 "RDKI"

TG-ZFD-044 "RDKI" was identified by *in vivo* screening from human gnomic sequence. Its amino acid sequence is: FACEVCGVRPTRNDKLKIHMRKH (SEQ ID NO:143). It is encoded by the human nucleic acid sequence:

As a polypeptide fusion to fingers 1 and 2 of Zif268, TG-ZFD-044 "RDKI" demonstrates recognition specificity for the 3-bp target sequence GGG as determined *by in vivo* screening results.

TG-ZFD-044 "RDKI" can be used as a module to construct a chimeric DNA binding protein comprising multiple zinc finger domains, e.g., for the purpose of recognizing a DNA site containing the sequence GGG.

### Example 53: TG-ZFD-045 "RDKR"

TG-ZFD-045 "RDKR" was identified by *in vivo* screening from human genomic sequence. Its amino acid sequence is: YVCDVEGCTWKFARSDKLNRHKKRH (SEQ ID NO:145). It is encoded by the human nucleic acid sequence:

As a polypeptide fusion to fingers 1 and 2 of Zif268, TG-ZFD-045 "RDKR" demonstrates recognition specificity for the 3-bp target sequence GGG and AGG. Its binding site preference is GGG > AGG as determined by *in vivo* screening results.

TG-ZFD-045 "RDKR" can be used as a module to construct a chimeric DNA binding protein comprising multiple zinc finger domains, e.g., for the purpose of recognizing a DNA site containing the sequence GGG and AGG.

### Example 54: TG-ZFD-046 "RSNR"

TG-ZFD-046 "RSNR" was identified by *in vivo* screening from human genomic sequence. Its amino acid sequence is: YICRKCGRGFSRKSNLIRHQRTH (SEQ ID NO:147). It is encoded by the human nucleic acid sequence:

As a polypeptide fusion to fingers 1 and 2 of Zif268, TG-ZFD-046 "RSNR" demonstrates recognition specificity for the 3-bp target sequence GAG and GTG. Its binding site preference is GAG > GTG as determined by *in vivo* screening results.

TG-ZFD-046 "RSNR" can be used as a module to construct a chimeric DNA binding protein comprising multiple zinc finger domains, e.g., for the purpose of recognizing a DNA site containing the sequence GAG and GTG.

### Example 55: TG-ZFD-047 "RTNR"

TG-ZFD-047 "RTNR" was identified by *in vivo* screening from human genomic sequence. Its amino acid sequence is: YLCSECDKCFSRSTNLIRHRRTH (SEQ ID NO:149). It is encoded by the human nucleic acid sequence:

As a polypeptide fusion to fingers 1 and 2 of Zif268, TG-ZFD-047 "RTNR" demonstrates recognition specificity for the 3-bp target sequence GAG as determined by *in vivo* screening results.

TG-ZFD-047 "RTNR" can be used as a module to construct a chimeric DNA binding protein comprising multiple zinc finger domains, e.g., for the purpose of recognizing a DNA site containing the sequence GAG.

### Example 56: TG-ZFD-048 "HSSR"

TG-ZFD-048 "HSSR" was identified *by in vivo* screening from human genomic sequence. Its amino acid sequence is: FKCPVCGKAFRHSSSLVRHQRTH (SEQ ID NO:173). It is encoded by the human nucleic acid sequence:

As a polypeptide fusion to fingers 1 and 2 of Zif268, TG-ZFD-048 "HSSR" demonstrates recognition specificity for the 3-bp target sequence GTT, as determined by *in vivo* screening results.

TG-ZFD-048 "HSSR" can be used as a module to construct a chimeric DNA binding protein comprising multiple zinc finger domains, e.g., for the purpose of recognizing a DNA containing the sequence GTT.

### Comparative Example 57: TG-ZFD-049 "ISNR"

TG-ZFD-049 "ISNR" was identified by in vivo screening from human genomic sequence. Its amino acid sequence is: YRCKYCDRSFSISSNLQRHVRNIH (SEQ ID NO:175). It is encoded by the human nucleic acid sequence:

As a polypeptide fusion to fingers 1 and 2 of Zif268, TG-ZFD-049 "ISNR" demonstrates recognition specificity for the 3-bp target sequences GAA, GAT, and GAC, as determined by *in vivo* screening results. Its binding site preference is GAA>GAT>GAC, as determined by *in vivo* screening results.

TG-ZFD-049 "ISNR" can be used as a module to construct a chimeric DNA binding protein comprising multiple zinc finger domains, e.g., for the purpose of recognizing a DNA containing the sequence GAA, GAT or GAC.

### Comparative Example 58: TG-ZFD-050 "KSNR"

TG-ZFD-050 "KSNR" was identified by *in vivo* screening from human genomic sequence. Its amino acid sequence is: YGCHLCGKAFSKSSNLRRHEMIH (SEQ ID NO:177). It is encoded by the human nucleic acid sequence:

As a polypeptide fusion to fingers 1 and 2 of Zif268, TG-ZFD-050 "KSNR" demonstrates recognition specificity for the 3-bp target sequence GAG, as determined by *in vivo* screening results.

TG-ZFD-050 "KSNR" can be used as a module to construct a chimeric DNA binding protein comprising multiple zinc finger domain, e.g., for the purpose of recognizing a DNA containing the sequence GAG.

### Example 59: TG-ZFD-051 "QSNK"

TG-ZFD-051 "QSNK" was identified by *in vivo* screening from human genomic sequence. Its amino acid sequence is: YKCEECGKAFTQSSNLTKHKKIH (SEQ ID NO:179). It is encoded by the human nucleic acid sequence:

As a polypeptide fusion to fingers 1 and 2 of Zif268, TG-ZFD-051 "QSNK" demonstrates recognition specificity for the 3-bp target sequences AAA, GAA, and TAA, as determined by *in vivo* screening results. Its binding site preference is GAA>TAA>AAA, as determined by *in vivo* screening results.

TG-ZFD-051 "QSNK" can be used as a module to construct a chimeric DNA binding protein comprising multiple zinc finger domains, e.g., for the purpose of recognizing a DNA containing the sequence GAA, TAA or AAA.

### Comparative Example 60: TGZFD-052 "QSNT"

TG-ZFD-052 "QSNT" was identified by in vivo screening from human genomic sequence. Its amino acid sequence is: YECVQCGKGFTQSSNLITHQRVH (SEQ ID NO:181). It is encoded by the human nucleic acid sequence:

As a polypeptide fusion to fingers 1 and 2 of Zif268, TG-ZFD-052 "QSNT" demonstrates recognition specificity for the 3-bp target.sequence AAA, as determined by *in vivo* screening results. Its binding site preference is AAA, as determined *by in vivo* screening results.

TG-ZFD-052 "QSNT" can be used as a module to construct a chimeric DNA binding protein comprising multiple zinc finger domain, e.g., for the purpose of recognizing a DNA containing the sequence AAA.

### Example 61: TG-ZFD-053 "VSNV"

TG-ZFD-053 "VSNV" was identified by in vivo screening from human genomic sequence. Its amino acid sequence is: YECDHCGKAFSVSSNLNVHRRIH (SEQ ID NO:183). It is encoded by the human nucleic acid sequence:

As a polypeptide fusion to finger 1 and 2 of Zif268, TG-ZFD-053 "VSNV" demonstrates recognition specificity for the 3-bp target sequences AAT, CAT, and TAT, as determined *by in vivo* screening results. Its binding site preference is AAT>CAT>TAT, as determined by *in vivo* screening results.

TG-ZFD-053 "VSNV" can be used as a module to construct a chimeric DNA binding protein comprising multiple zinc finger domains, e.g., for the purpose of recognizing a DNA containing the sequence AAT, CAT or TAT.

### Comparative Example 62: TG-ZFD-054 "DSCR"

TG-ZFD-054 "DSCR" was identified by in vivo screening from human genomic sequence. Its amino acid sequence is: YTCSDCGKAFRDKSCLNRHRRTH (SEQ ID NO:185). It is encoded by the human nucleic acid sequence:

As a polypeptide fusion to finger 1 and 2 of Zif268, TG-ZFD-054 "DSCR" demonstrates recognition specificity for the 3-bp target sequence GCC as determined by in vivo screening results.

TG-ZFD-054 "DSCR" can be used as a module to construct a chimeric DNA binding protein comprising multiple zinc finger domain, e.g., for the purpose of recognizing a DNA containing the sequence GCC.

### Comparative Example 63: TG-ZFD-055 "ISNV"

TG-ZFD-055 "ISNV" was identified by in vivo screening from human genomic sequence. Its amino acid sequence is: YECDHCGKAFSIGSNLNVHRRIH (SEQ ID NO:187). It is encoded by the human nucleic acid sequence:

As a polypeptide fusion to finger 1 and 2 of Zif268, TG-ZFD-055 "ISNV" demonstrates recognition specificity for the 3-bp target sequence AAT as determined by in vivo screening results.

TG-ZFD-055 "ISNV" can be used as a module to construct a chimeric DNA binding protein comprising multiple zinc finger domain, e.g., for the purpose of recognizing a DNA containing the sequence AAT.

### Example 64: TG-ZFD-056 "WSNR"

TG-ZFD-056 "WSNR" was identified by in vivo screening from human genomic sequence. Its amino acid sequence is: YRCEECGKAFRWPSNLTRHKRIH (SEQ ID NO:189). It is encoded by the human nucleic acid sequence:

As a polypeptide fusion to finger 1 and 2 of Zif268, TG-ZFD-056 "WSNR" demonstrates recognition specificity for the 3-bp target sequence GGT, and GGA as determined by in vivo screening results. Its binding site preference is GGT>GGA as determined by in vivo screening results.

TG-ZFD-056 "WSNR" can be used as a module to construct a chimeric DNA binding protein comprising multiple zinc finger domain, e.g., for the purpose of recognizing a DNA containing the sequence GGT or GGA.

### Example 65: TG-ZFD-057 "DSAR"

TG-ZFD-057 "DSAR" was identified by in vivo screening from human genomic sequence. Its amino acid sequence is: FMCTWSYCGKRFTDRSALARHKRTH (SEQ ID NO:191). It is encoded by the human nucleic acid sequence:

As a polypeptide fusion to finger 1 and 2 of Zif268, TG-ZFD-057 "DSAR" demonstrates recognition specificity for the 3-bp target sequence GTC as determined by in vivo screening results.

TG-ZFD-057 "DSAR" can be used as a module to construct a chimeric DNA binding protein comprising multiple zinc finger domain, e.g., for the purpose of recognizing a DNA containing the sequence GTC.

The afore-mentioned zinc finger domains and their specificities are summarized in the following Tables.

**Table 3**

| Binding site Name of ZFD (SEQ ID NO:) | |
|---|---|
| AAA | QSNI(115), QSNV1(51), QSNV2(53), QSNV3(55), QSNV4(57) |
| ACA | QSTV(31) |
| AGA | QSHR1(37), QSHR5(45), QSHT(111), QSHV(113), QTHQ(121), QTHR1(123) |
| AGG | RDHT(141), RDKR(145) |
| CAA | QSNI(51), QSNV1(51), QSNV2(53) |
| CGA | QTHQ(121), QSHR5(45), QSHT(111), QSHV(113) |
| CGG | RDHT(141) |
| GAA | CSNR1(23), CSNR2(35), QGNR(107), QSHR1(37), QSHR3(41), QSHR4(43), QSHR5(45), QSNR1(47), QSNR2(49), QSNR3(117), QTHR1(123) |
| GAC | CSNR1(23), CSNR2(35), HSNK(25), SSNR(27) |
| GAG | CSNR1(23), CSNR2(35), RDER1(29), RDHR1(137), RSNR(147), RTNR(149), SSNR(27), QFNR(105), |
| GCA | QSSR1(59), QSSR3(119), QSTR(63) |
| GCG | RDER1(29), RDER2(127), REDR3(129), RDER4(131), RDER5(133), RDER6(135), VSTR(33) |
| GCT | VSTR(33) |
| GGA | QAHR(103), QSHR1(37), QSHR2(39), QSHR3(41), QSHR4(43), QSHR5(45), QSHT(111), QTHR1(123), QTHR2(125) |
| GGG | RDKI(143), RDHR1(137), RDHT(141), RDKR(143), RSHR(65), |
| GTA | QSSR1(59), QSSR2(61), QSSR3(119), QSTR(63), VSSR(67) |
| GTG | RSNR(147), RDER1(29), RDER2(127), RDER3(129), RDER4(131), RDER6(135), VSSR(67) |
| GTT | VSSR(67) |
| TGA | QSHT(111), QSHV(113), QTHQ(121) |
| TGG | RDHT(141) |

**Table 4A**

| Binding site Name of ZFD (SEQ ID NO:) | |
|---|---|
| AAA | QSNK(179), QSNT(181) |
| AAT | VSNV(183) |
| CAT | VSNV(183) |
| GAA | ISNR(175), QSNK(179) |
| GAC | ISNR(175) |
| GAG | KSNR(177) |
| GAT | ISNR(175) |
| GTT | HSSR(173) |
| TAA | QSNK(179) |
| TAT | VSNV(183) |

**Table 4B**

| Binding site Name of ZFD (SEQ ID NO:) | |
|---|---|
| AAT | ISNV (187) |
| GCC | DSCR (185) |
| GGT | WSNR (189) |
| GGA | WSNR (189) |
| GTC | DSAR (191) |

**Table 5**

| Name of ZFD | Binding site | Polypeptide SEQ ID NO: | Nucleic Acid** SEQ ID NO: |
|---|---|---|---|
| CSNR1 | GAA>GAC>GAG | 23 | 22 |
| HSNK | GAC | 25 | 24 |
| SSNR | GAG>GAC | 27 | 26 |
| RDER1 | GCG>GTG, GAG | 29 | 28 |
| QSTV | ACA | 31 | 30 |
| VSTR | GCT>GCG | 33 | 32 |
| CSNR2 | GAA>GAC>GAG | 35 | 34 |
| QSHR1 | GGA>GAA>AGA | 37 | 36 |
| QSHR2 | GGA | 39 | 38 |
| QSHR3 | GGA>GAA | 41 | 40 |
| QSHR4 | GGA>GAA | 43 | 42 |
| QSHR5 | | 45 | 44 |
| QSNR1 | GAA | 47 | 46 |
| QSNR2 | GAA | 49 | 48 |
| QSNV1 | AAA>CAA | 51 | 50 |
| QSNV2 | AAA>CAA | 53 | 52 |
| QSNV3 | AAA | 55 | 54 |
| QSNV4 | AAA | 57 | 56 |
| QSSR1 | GTA>GCA | 59 | 58 |
| QSSR2 | GTA | 61 | 60 |
| QSTR | GTA>GCA | 63 | 62 |
| RSHR | GGG | 65 | 64 |
| VSSR | GTT>GTG>GTA | 67 | 66 |
| QAHR | GGA | 103 | 102 |
| QFNR | GAG | 105 | 104 |
| QGNR | GAA | 107 | 106 |
| QSHT | AGA,CGA>TGA>GGA | 111 | 110 |
| QSHV | CGA>AGA>TGA | 113 | 112 |
| QSNI | AAA,CAA | 115 | 114 |
| QSNR3 | GAA | 117 | 116 |
| QSSR3 | GTA>GCA | 119 | 118 |
| QTHQ | AGA>CGA, TGA | 121 | 120 |
| QTHR1 | GGA>GAA, AGA | 123 | 122 |
| QTHR2 | GGA | 125 | 124 |
| RDER2 | GCG>GTG | 127 | 126 |
| RDER3 | GCG>GTG | 129 | 128 |
| RDER4 | GCG>GTG | 131 | 130 |
| RDER5 | GCG | 133 | 132 |
| RDER6 | GCG>GTG | 135 | 134 |
| RDHR1 | GAG, GGG | 137 | 136 |
| RDHT | TGG, AGG, CGG, GGG | 141 | 140 |
| RDKI | GGG | 143 | 142 |
| RDKR* | GGG>AGG | 145 | 144 |
| RSNR | GAG>GTG | 147 | 146 |
| RTNR | GAG | 149 | 148 |

| | | | |
|---|---|---|---|
| **The indicated nucleic acid SEQ ID refers to a nucleic acid that encodes the zinc finger domain. *Domains followed by the "*" symbol are zinc domains obtained by mutagenesis. The domains not so indicated are human zinc tinger domains according to the present invention. | | | |

**Table 6**

| Name of ZFD | Binding site | Polypeptide SEQ ID NO: | Nucleic Acrid** SEQ ID NO: |
|---|---|---|---|
| HSSR | GTT | 173 | 174 |
| ISNR | GAA>GAT>GAC | 175* | 176* |
| KSNR | GAG | 177* | 178* |
| HSNK | GAA>TAA>AAA | 179 | 180 |
| QSNT | AAA | 181* | 182* |
| VSNV | AAT>CAT>TAT | 183 | 184 |

| | | | |
|---|---|---|---|
| ** The indicated nucleic acid SEQ ID refers to a nucleic acid that encodes the zinc finger domain. *The indicated SEQ ID NO refers to a comparative example. | | | |

**Table 7**

| Name of ZFD | Binding site | Polypeptide SEQ ID NO: | Nucleic Acid** SEQ ID NO: |
|---|---|---|---|
| DSCR | GCC | 185* | 186* |
| ISNV | AAT | 187* | 188* |
| WSNR | GGT > GGA | 189 | 190 |
| DSAR | GTC | 191 | 192 |

| | | | |
|---|---|---|---|
| **The indicated nucleic acid SEQ ID refers to a nucleic acid that encodes the zinc finger domain. * The indicated SEQ ID NO refers to a comparative example. | | | |

### Example 66: Construction of Individual Three-Finger Proteins

The vector P3 was used to express chimeric zinc finger proteins in mammalian cells. P3 was constructed by modification of the pcDNA3 vector (Invitrogen, San Diego CA). A synthetic oligonucleotide duplex having compatible overhangs was ligated into the pcDNA3 vector digested with HindIII and XhoI. The duplex contains nucleic acid that encodes the hemagglutinin (HA) tag and a nuclear localization signal. The duplex also includes BamHI, EcoRI and NotI and BgIII restriction site sites and a stop codon (FIG. 11A). Further, the XmaI site in SV40 origin of the resulting vector was destroyed by digestion with XmaI, filling in the overhanging ends of the digested restriction site, and religation of the ends.

To construct a zinc finger protein that includes three particular zinc finger domains, a nucleic acid encoding the first zinc finger was ligated into the P3 vector. Nucleic acids encoding the second and third zinc finger domains were ligated together using Dynabeads® and MPC-S (Dynal) as follows. Nucleic acids encoding the second and third zinc finger domains were synthesized using forward primers that contain an XmaI site and reverse primers that contain AgeI and NotI sites. The forward primer of the second zinc finger domain was biotinylated. The nucleic acid encoding the second zinc finger domain was digested with AgeI and ligated to XmaI digested nucleic acid encoding the third zinc finger domain. After ligation for one hour at room temperature, the ligation sample was bound to Dynabeads® M-280 streptavidin (Dynal) for 15 minutes at room temperature. The beads were washed three times with TE buffer (10 mM Tris HCl 0.1 mM EDTA, pH 8.0). The attached ligation sample was digested with XmaI and NotI for three hours at 37°C. Nucleic acid released by the XmaI and NotI digestion was purified using the PCR purification kit (Qiagen) and ligated to the P3 vector that included the nucleic acid encoding the first zinc finger. Products of this final ligation were transformed in *E. coli.* Clones containing the correct size insert in the P3 vector were identified. The nucleic acid encoding the resultant three-finger ZFP was confirmed by DNA sequencing.

### Example 67: Construction of a Library of Three-Finger Proteins

FIG. 11B depicts one method of constructing a diverse three finger library. First, nucleic acid encoding each zinc finger domain was cloned into the P3 vector to form "single finger" vectors. Equal amounts of each "single finger" vector were combined to form a pool. The pool was separately digested with two sets of enzymes; AgeI and XhoI, and XmaI and XhoI. After phosphatase treatment for 30 min, digested vector nucleic acid from the AgeI and XhoI digested pool were ligated to the nucleic acid segments released from the vector by the XmaI and XhoI digestion. These segments each encode a single zinc finger domain. The ligation of the digested vector nucleic acids to the nucleic acid segments forms vectors encoding two zinc finger domains. After transformation into *E. coli,* the ligation products yielded approximately 1.4 x 10⁴ transformants, thereby forming a two-finger library. The size of the insert region of the two-finger library was verified by colony PCR analysis of 40 colonies. The correct size insert was present in 95 % of the library.

Subsequently, the 2-finger library was digested with *Age*I and *Xho*I. The digested vector which retains nucleic acid sequences encoding two zinc finger domains was ligated to the pool of one-finger fragment prepared above by digestion with *Xma*I and *Xho*I. The products of this ligation were transformed into *E. coli* to yield about 2.4 x 10⁵ independent transformants. Verification of the inset region indicated that library members were predominantly correctly constructed, i.e., they each encoded three zinc finger domains.

### Example 68: In vivo Assays for Three-Finger Proteins

Kim and Pabo ((1997) J Biol Chem 272:29795-29800) demonstrated that the Zif268 protein efficiently repressed VP16-activated transcription of a target gene when the Zif268 protein was bound near the transcription start site of a target gene. It was hypothesized that such a bound zinc finger protein would inhibit the binding of the basal transcription machinery such as the RNA polymerase II complex to the promoter or the binding of TFIID to the transcription start site or TATA box.

A similar *in vivo* repression assay was used to determine if the new three-finger proteins were functional *in vivo.* The assay utilized a luciferase reporter construct in which a target site is located at a position comparable to the position of the Zif268 site in the construct of Kim and Pabo, *supra.*

The luciferase reporter plasmids were constructed from pΔS-modi, a modified version of pGL3-TATA/Inr (Kim and Pabo, *supra*). These reporters utilize firefly luciferase as the reporter protein. The SacI site in upstream of the TATA box was deleted from pΔS-modi. A new SacI site was inserted following the transcription initiation site. An oligomer containing a specific 9 base pairs of binding site of each ZFP replaced 14 base pairs located after 12 bases downstream of the transcription initiation site in pΔS-modi by digestion of SacI and HindIII. The resulting reporter plasmid was named as p1G-ZFP ID (The ZFP ID is determined according to the binding site of the specific reporter). The sequences of pΔS-modi and p1G are set forth in Fig. 12.

The *in vivo* activity assay for a particular three-finger protein was carried out as follows. HEK 293 cells were transfected with four plasmids: 14 ng of a plasmid expressing the particular three-finger protein; 14 ng of the reporter plasmid described above; 70 ng of a plasmid that expresses GAL4-VP16; and 1.4 ng of a plasmid that expresses Renillar luciferase. The GAL4-VP16 activates transcription of the minimal synthetic promoter in the reporter so that the repression effected by a particular three-finger protein could be clearly detected and compared to other three-finger proteins. The plasmid expressing Renillar luciferase provides a control of transfection efficiency.

Lipofectamine (Gibco-BRL) was used for the transfection procedures. Cells were transfected at 30-50% confluency in wells of a 96 well plate. The cells were incubated for two days prior to harvesting for the luciferase assay. Then luciferase activities were measured using the Dual-Luciferase™ Reporter Assay System (Promega). The observed firefly luciferase activity was normalized using the observed level of Renilla luciferase. The extent of repression or "fold-repression" was calculated by dividing a value for normalized reporter expression in the absence of a zinc finger protein by a value for normalized reporter expression in the presence of the zinc finger protein.

Zinc finger proteins were classified as satisfying a high stringency cut-off value if they repressed transcription at least 2-fold in the transfection assay or as satisfying a low stringency cut-off value if they repressed between 1.5 and 2-fold in the transfection assay.

### Example 69: Binding Assay Result of ZFPs with Their Specific Reporter

Gel shift assays were used to correlate activity observed in the *in vivo* assays to binding affinity. A good correlation was observed between the dissociation constants measured by gel shift assays and the level of transcriptional repression in the transfection assays described above. Table 8 relates the binding affinity of Zif268 for a variety of DNA sites and the corresponding *in vivo* repression data using the transfection assay above.

In general, zinc finger proteins exhibiting more than 2-fold repression (that is, 50% repression) in the transfection assays showed a dissociation constant of less than 1 nM as determined by gel shift assays.

**Table 8. In vivo and in vitro DNA-binding activities of Zif268.**

| Target sequence | Dissociation Constant (nM) for Zif268 binding | Fold repression in human cells expressing Zif268 |
|---|---|---|
| GCT TGG GCG | 2.1 ± 0.3 | 1.5 ± 0.1 |
| GCG TGG GCG | 0.024 ± 0.004 | 28 ± 1 |
| GAG TGG GCG | 0.17 ± 0.04 | 5.9 ± 0.1 |
| GAG CGG GCG | 2.3 ± 0.9 | 1.6 ± 0.0 |
| GAC TGG GCG | 4.9 ± 0.6 | 1.2 ± 0.1 |
| ACA TGG GCG | 1.3 ± 0.3 | 1.3 ± 0.1 |

### Example 70: Characterization of Three-Finger Proteins

Two types of "three-finger" chimeric zinc finger proteins were constructed. One type includes chimeric proteins that are composed exclusively of human zinc finger domain that are identical to naturally-occurring zinc finger domains. The other type includes chimeric proteins that include zinc finger domains that are not identical to a naturally-occurring zinc finger domain. The latter zinc finger domains were identified by *in vitro* mutagenesis of a naturally-occurring zinc finger domain followed by phage display selection. Such domains have avoided the scrutiny of natural evolution.

The component zinc finger domains are listed in Table 9. A total of 36 zinc finger domains, 18 human zinc finger domains and 18 mutated zinc finger domains, were used to assemble a set of test three-finger proteins. The mutated zinc finger domains have been reported in Choo and Klug. (1994) Proc. Natl. Acad. Sci. USA 91:11168-11172; Desjarlais and Berg (1994) Proc. Natl. Acad. Sci. USA. 91:11099-11103; Dreier et al. (2001) J Biol Chem. 276:29466-29478; Dreier et al. (2000) J Mol Biol. 303:489-502; Fairall et al. (1993) Nature 366:483-487; Greisman and Pabo. (1997) Science. 275:657-661; Kim and Pabo (1997) J. Biol. Chem. 272:29795-29800; and Segal et al. (1999) Proc. Natl. Acad. Sci. USA 96:2758-2763.

**Table 9. Some Exemplary Domains for Construction of Three-Fingers ZFP**

| Domain name | Source | SEQ ID NO or reference | target sites |
|---|---|---|---|
| CSNR1 | human | 23 | GAA, GAC, GAG |
| HSNK | human | 25 | GAC |
| ISNR | human | 175 | GAA, GAT, GAC |
| QSHR2 | human | 39 | GGA |
| QSHR3 | human | 41 | GGA, GAA |
| QSHT | human | 111 | NGA |
| QSNR1 | human | 47 | GAA |
| QSNR3 | human | 117 | GAA |
| QSNV2 | human | 53 | AAA, CAA |
| QSSR1 | human | 59 | GTA, GCA |
| QTHQ | human | 121 | AGA, CGA, TGA |
| RDHT | human | 141 | NGG |
| RDER1 | human | 29 | GCG, GTG |
| RSHR | human | 65 | GGG |
| RSNR | human | 147 | GAG, GTG |
| VSNV | human | 183 | AAT |
| VSSR | human | 67 | GTT, GTG, GTA |
| VSTR | human | 33 | GCT |
| DGAR | mutated/phage display | PNAS, Segal, D. J. et al. (1999) | GTC |
| DGHR | mutated/phage display | PNAS, Segal, D. J. et al. (1999) | GGC |
| DGNR | mutated/phage display | PNAS, Segal, D. J. et al. (1999) | GAC |
| DGNV | mutated/phage display | J.B.C., Dreier, B. et al. (2001) | AAC |
| DRDR | mutated/phage display | PNAS, Segal, D. J. et al. (1999) | GCC |
| GRER | mutated/phage display | J.B.C., Dreier, B. et al. (2001) | GCC |
| NDTR | mutated/phage display | PNAS, Choo & Klug, (1994) | GTT |
| QAHR | mutated/phage display | PNAS, Segal, D. J. et al. (1999) | GGA |
| QGDR | mutated/phage display | PNAS, Segal, D. J. et al. (1999) | GCA, GCC, GCT |
| QGTR | mutated/phage display | Science, Greisman, H. A. & Pabo, C. (1997) | ACA |
| QSDR | mutated/phage display | PNAS, Desjarlais, J. R. & Berg, J. M. (1994) | GCT |
| QSNR | mutated/phage display | PNAS, Segal, D. J. et al. (1999) | GAA |
| QSSR | mutated/phage display | PNAS, Segal, D. J. et al. (1999) | GTA |
| RDKR | mutated/phage display | J.B.C., Dreier, B. et al. (2001) | GGG |
| RDNR | mutated/phage display | PNAS, Segal, D. J. et al. (1999) | GAG |
| RDTN | mutated/phage display | J.B.C., Dreier, B. et al. (2001) | AAG |
| TDKR | mutated/phage display | PNAS, Segal, D. J. et al. (1999) | GGG, GGT |
| TGNR | mutated/phage display | PNAS, Segal, D. J. et al. (1999) | GAT, GAA |

| | | | |
|---|---|---|---|
| N in the target site can be any of A, C, G, and T. | | | |

Nucleic acids encoding the 36 domains were individually subcloned into P3 vector digested with *Eco*RI and *Not*I*,* and the resulting plasmids were used as starting material for the chimeric zinc finger protein construction.

Nucleic acids encoding chimeric 3-finger proteins were prepared by two different methods.

In the first method, nucleic acids encoding all the zinc finger domains were randomly mixed, as described in Example 68, and 3-finger constructs were randomly picked for further analysis. Each construct was sequenced to determine the component zinc finger domains in the polypeptide that it encodes. Subsequently, target DNA sequences were synthesized for each randomly assorted 3-finger protein. Target DNA sequences were based on the expected preferred target site. The targets were cloned into the luciferase reporter vector described above. This approach is referred to as "zinc finger protein-first" approach.

In the second method, nucleic acid encoding chimeric 3-finger proteins were assembled based on a given target DNA sequences, as described in Example 66. A computer algorithm was used to a match recognition sites of zinc finger domains and target DNA sequences. Promoter sequences of known genes were used as the input target DNA sequences. The promoter sequences were scanned to identify segments that are nine nucleotides in length and that are acceptable target sites for recognition by chimeric 3-finger proteins given the available collection of zinc finger domains. Once identified, a nucleic acid was constructed that encoded the chimeric 3-finger proteins. This approach is referred to as "target site-first" approach.

Zinc finger domains that include an aspartate residue at position 2 of the base contacting residues were analyzed with special consideration. Such zinc finger domains include RDER1, RDHT, RDNR, RDKR, RDTN, TDKR, and NDTR The X-ray co-crystal structure of Zif268 bound to DNA showed that an aspartate at position 2 can form a hydrogen bond with a base outside of the 3-basepair subsite recognized by zinc fingers. As a result, the RDER finger containing an aspartate residue at position 2 prefers the 4-basepair site: 5'-GCG, (G/T)-3'. The computer algorithm accounted for this additional specificity. Similarly, randomly-assembled 3-finger proteins that include a finger having aspartate at position 2 and that violate this rule for the 4-bp site were excluded in other analyses described herein.

A total of 153 three-finger chimeric proteins were constructed from both the "zinc finger protein-first" and the "target site-first" approaches. These proteins were tested using the transient cotransfection assay described in Example 68. The results are set forth in Table 10.

**Table 10. Zinc finger proteins and their DNA-binding activity**

| | Domain Name | | | Target composite sequence | Fold repression |
|---|---|---|---|---|---|
| | 1 | 2 | 3 | | |
| 1 | RSNR | RDHT | RSNR | 5'-GAG AGG GAG C-3' (SEQ ID NO:199) | 8.1 |
| 2 | CSNR1 | RSHR | RDHT | 5'-TGG GGG GAC A-3' (SEQ ID NO:200) | 4.5 |
| 3 | RDHT | QSNV2 | QSSR1 | 5'-GCA CAA TGG-3' | 4.0 |
| 4 | RSHR | RDER1 | RDER1 | (SEQ ID GCG GGG C-3' (SEQ ID NO:201) | 3.8 |
| 5 | QSHR3 | QAHR* | QSSR* | 5'-GTA GGA GGA T-3' (SEQ ID NO:202) | 3.7 |
| 6 | RSHR | RDER1 | RDHT | 5'-AGG GCG GGG C-3' (SEQ ID NO:203) | 3.6 |
| 7 | RDHT | QSNV2 | RSHR | 5'-GGG AAA CGG G-3' (SEQ ID NO:204) | 3.6 |
| 8 | RSNR | QSHR2 | QSSR1 | 5'-GTA GGA GAG T-3' (SEQ ID NO:205) | 3.3 |
| 9 | QSDR* | RDHT | QSHR3 | 5'-GGA AGG GCT T-3' (SEQ ID NO:206) | 3.3 |
| 10 | RDHT | RSHR | QSHR2 | 5- GGA GGG TGG -3' | 3.1 |
| 11 | QSHT | RSHR | RDHT | 5'-TGG GGG TGA-3' | 3.0 |
| 12 | QSSR1 | QSNV2 | RSNR | 5'-GAG CAA GTA G-3' (SEQ ID NO:207) | 2.9 |
| 13 | QSHR2 | RDER1 | RSNR | 5'-GAG GTG GGA G-3' (SEQ ID NO:208) | 2.8 |
| 14 | DGHR* | QSNR1 | RDHT | 5'-GTA GGC GCC-3' | 2.6 |
| 15 | VSNV | CSNR1 | RSNR | 5'-GAG GAC AAT G -3' (SEQ ID NO:209) | 2.5 |
| 16 | QSHR2 | RDER1 | RSHR | 5'-GGG GCG GGA T-3' (SEQ ID NO:210) | 2.3 |
| 17 | VSSR | QGDR* | RDHT | 5'-GGG GCA GTT-3' | 2.3 |
| 18 | RDER1 | QSSR1 | QSHT | 5'-CGA GCA GCG-3' | 2.2 |
| 19 | RDER1 | VSSR | QSHT | 5'-CGA GTT GCG-3' | 2.1 |
| 20 | QSNR3 | RSHR2 | RSNR | 5'-GAG GGA GAA G-3' (SEQ ID NO:211) | 2.1 |
| 21 | RDHT | QSHT | RSNR | 5'-GGA GAG AGA-3' | 2.1 |
| 22 | RSNR | VSSR | RSHR | 5'-GGG GTT GAG-3' | 2.1 |
| 23 | RDHT | RSNR | QSNR3 | 5'-GAA GAG AGG T-3' (SEQ ID NO:212) | 2.1 |
| 24 | CSNR1 | QSHT | RSNR | 5'-GAG TGA GAC C-3' (SEQ ID NO:213) | 2.1 |
| 25 | QSNR3 | RDER1 | RSNR | 5'-GAG GCG GAA A3' (SEQ ID NO:214) | 2.1 |
| 26 | QSNR3 | QSNV2 | RSHR | 5'-GGG AAA GAA C-3' (SEQ ID NO:215) | 2.0 |
| 27 | RSHR | RDER1 | RSHR | 5'-GGG GTG GGG-3' | 2.0 |
| 28 | VSSR | QSNR3 | RDER1 | 5'-GCG GAA GTT C-3' (SEQ ID NO:216) | 2.0 |
| 29 | QSNR3 | RDHT | RSNR | 5'-GAG TGG GAA A-3' (SEQ ID NO:217) | 2.0 |
| 30 | RSHR | RSHR | QSHR2 | 5-GGA GGG GGG C-3' (SEQ ID NO:218) | 2.0 |
| 31 | ISNR | RSNR | RDHT | 5'-TGG GAG GAT C-3' (SEQ ID NO:219) | 2.0 |
| 32 | QSNV2 | RSNR | RDHT | 5'-GGG GAG AAA-3' | 1.9 |
| 33 | QSNV2 | RSHR | RDER1 | 5'-GTG GGG AAA A-3' (SEQ ID NO:220) | 1.9 |
| 34 | VSSR | HSNK | RSNR | 5'-GAG GAC GTG -3' | 1.9 |
| 35 | VSSR | QSHT | RSHR | 5'-GGG TGA GTG-3' | 1.9 |
| 36 | RSNR | VSSR | RSNR | 5'-GAG GTT GAG G-3' (SEQ ID NO:221) | 1.8 |
| 37 | OAHR* | QSNR | QTHQ | 5'-AGA GAA GGA G-3' (SEQ ID NO:222) | 1.8 |
| 38 | RSNR | ISNR | QSHT | 5'-TGA GAT GAG C-3' (SEQ ID NO:223) | 1.8 |
| 39 | VSTR | RSNR | QSHR2 | 5'-GGA GAG GCT C-3' (SEQ ID NO:224) | 1.8 |
| 40 | ISNR | VSTR | RDHT | 5'-AGG GCT GAT T-3' (SEQ ID NO:225) | 1.8 |
| 41 | QSNR3 | RSNR | RSHR | 5'-GGG GAG GAA A-3' (SEQ ID NO:226) | 1.7 |
| 42 | RDHT | QSHR2 | QSHT | 5'-AGA GGA AGG T-3' (SEQ ID NO:227) | 1.7 |
| 43 | QSSR1 | QSNR3 | QSHR2 | 5'-GGA GAA GTA G-3' (SEQ ID NO:228) | 1.7 |
| 44 | RDHT | DGHR* | TDKR* | 5'-GGT GGC AGG T-3' (SEQ ID NO:229) | 1.7 |
| 45 | HSNK | QSNV2 | VSSR | 5'-GTT CAA GAC-3' | 1.7 |
| 46 | HSNK | QSHT | RSNR | 5'-GAG AGA GAC-3' | 1.7 |
| 47 | RSHR | QSHR2 | VSTR | 5'-GCT GGA GGG G-3' (SEQ ID NO:230) | 1.7 |
| 48 | RDHT | RSHR | RDER1 | 5'-GCG GGG AGG G-3' (SEQ ID NO:231) | 1.6 |
| 49 | RSHR | QSNV2 | QSHT | 5'-AGA AAA GGG-3' | 1.6 |
| 50 | RSHR | RDER1 | QSNV2 | 5'-AAA GTG GGG A-3' (SEQ ID NO:232) | 1.6 |
| 51 | VSNV | QSNV2 | QSHT | 5'-AGA AAA AAT A-3' (SEQ ID NO:233) | |
| 52 | QSHR2 | QSHT | RSNR | 5'-GAG TGA GGA-3' | 1.6 |
| 53 | QSHR2 | RDHT | CSNR1 | 5'-GAC AGG GGA G-3' (SEQ ID NO:234) | 1.6 |
| 54 | QSHT | VSSR | RSHR | 5'-TGA GTT GGG A-3' (SEQ ID NO:235) | 1.6 |
| 55 | QSNV2 | QSHR2 | QSNR3 | 5'-GAA GGA AAA T-3' (SEQ ID NO:236) | .61 |
| 56 | RSNR | VSTR | RSHR | 5'-GGG GGT GAG G-3' (SEQ ID NO:237) | 1.5 |
| 57 | QSHR2 | CSNR1 | QSHT | 5'-TGA GAC GGA G-3' (SEQ ID NO:238) | 1.5 |
| 58 | VSNV | QSHR2 | VSTR | 5'-GCT GGA AAT T-3' (SEQ ID NO:239) | 1.5 |
| 59 | DGAR* | DGNR* | RDKR* | 5'-GGG GAC GTC-3' | 1.5 |
| 60 | QSNR3 | QSSR1 | QSNV2 | 5'-CAA GTA GAA G-3' (SEQ ID NO:240) | 1.5 |
| 61 | QSNR3 | RDER1 | RSNR | 5'-GAG GCG GAA A-3' (SEQ ID NO:241) | 1.5 |
| 62 | RDER1 | QSSR1 | VSTR | 5'-GCT GCA GCG T-3' (SEQ ID NO:242) | 1.5 |
| 63 | QSHR3 | DGHR* | RDHT | 5'-GGG GGC GGA-3' | 1.5 |
| 64 | VSSR | RSHR | ISNR | 5'-GAT GGG GTT T-3' (SEQ ID NO:243) | 1.5 |
| 65 | RSNR | RDER1 | RSHR | 5'-GGG GCG GAG-3' | 1.5 |
| 66 | RSNR | RDER1 | QSNR3 | 5'-GAA GCG GAG G-3' (SEQ ID NO:244) | 1.4 |
| 67 | QSHR3 | QSNV2 | DGHR* | 5'-GGC AAA GGA-3' | 1.4 |
| 68 | QSNV2 | QSNR3 | QSSR1 | 5'-GTA GAA AAA-3' | 1.4 |
| 69 | QSNR3 | RDER1 | RDER1 | 5'-GTG GCG GAA G-3' (SEQ ID NO:245) | 1.4 |
| 70 | QSNV2 | QSDR* | QSSR1 | 5'-GTA GCT AAA-3' | 1.4 |
| 71 | QSNV2 | QSHR2 | QSNV2 | 5'-AAA GGA AAA G-3' (SEQ ID NO:246) | 1.4 |
| 72 | QSNV2 | VSSR | RDHT | 5'-CGG GTT AAA A-3' (SEQ ID NO:247) | 1.4 |
| 73 | QSHR3 | QTHQ | QSDR* | 5'-GCT AGA GGA-3' | 1.4 |
| 74 | RSHR | VSTR | QSSR1 | 5'-GTA GCT GGG A-3' (SEQ ID NO:248) | 1.4 |
| 75 | RDER1 | QSNV2 | QSHR2 | 5'-GGA CAA GCG G-3' (SEQ ID NO:249) | 1.3 |
| 76 | QSNV2 | QSHT | QSNV2 | 5'-AAA AGA AAA A-3' (SEQ ID NO:250) | 1.3 |
| 77 | QSHR2 | QSHR2 | QSSR1 | 5'-GTA GGA GGA T-3' (SEQ ID NO:202) | 1.3 |
| 78 | QSNR3 | QSSR1 | QSHT | 5'-AGA GTA GAA T-3' (SEQ ID NO:252) | 1.3 |
| 79 | QSNV2 | QSSR1 | QSNV2 | 5'-AAA GTA AAA A-3' (SEQ ID NO:253) | 1.3 |
| 80 | QSHR2 | RSNR | RDHT | 5'-AGG GAG GGA G-3' (SEQ ID NO:254) | 1.3 |
| 81 | RSNR | VSNV | QSNV2 | 5'-AAA AAT GAG C-3' (SEQ ID NO:255) | 1.3 |
| 82 | QSHT | QSNR3 | RDHT | 5'-CGG GAA AGA A-3' (SEQ ID NO:256) | 1.3 |
| 83 | QTHQ | QGDR* | QSSR* | 5'-GTA GCA AGA C-3' (SEQ ID NO:257) | 1.3 |
| 84 | QSNV2 | QSSR1 | VSNV | 5'-AAT GTA AAA A-3' (SEQ ID NO:258) | 1.3 |
| 85 | RDKR* | QAHR* | RDHT | 5'-CGG GGA GGG G-3' (SEQ ID NO:259) | 1.3 |
| 86 | QSNR* | QSNR* | QAHR* | 5'-TTC TTC TCC-3' | 1.3 |
| 87 | DGNR* | RSNR | QSSR1 | 5'-GTA GAG GAC-3' | 1.2 |
| 88 | CSNR1 | QSHT | QSNV2 | 5'-CAA AGA GAC T-3' (SEQ ID NO:260) | 1.2 |
| 89 | RDER1 | ISNR | QSNR3 | 5'-GAA GAT GCG T-3' (SEQ ID NO:261) | 1.2 |
| 90 | RDHT | QSSR1 | QSHT | 5'-CGA GCA TGG G-3' (SEQ ID NO:262) | 1.2 |
| 91 | RDHT | QGTR* | QGTR* | 5'-ACA ACA GGG G-3' (SEQ ID NO:263) | 1.20 |
| 92 | RSHR | RSHR | VSSR | 5'-GTT GGG GGG C-3' (SEQ ID NO:264) | 1.2 |
| 93 | RDER1 | RSNR | RDHT | 5'-AGG GAG GTG T-3' (SEQ ID NO:265) | 1.2 |
| 94 | RSHR | CSNR1 | QSHT | 5'-TGA GAC GGG G-3' (SEQ ID NO:266) | 1.2 |
| 95 | QSHR2 | VSSR | QSNR3 | 5'-GAA GTT GGA A-3' (SEQ ID NO:267) | 1.2 |
| 96 | QSNR3 | QSNV2 | QSHT | 5'-AGA AAA GAA A-3' (SEQ ID NO:268) | 1.2 |
| 97 | QSNV2 | QSHT | CSNR1 | 5'-GAC TGA CAA T-3' (SEQ ID NO:269) | 1.2 |
| 98 | TGNR* | RDNR* | QSDR* | 5'-GCT GAG GAT G-3' (SEQ ID NO:270) | 1.2 |
| 99 | QSNV2 | RSNR | RSHR | 5'-GGG GAG AAA T-3' (SEQ ID NO:271) | 1.2 |
| 100 | QSNR3 | QSHT | QSHT | 5'-TGA TGA GAA A-3' (SEQ ID NO:272) | 1.2 |
| 101 | HSNK | QSHR2 | QSSR1 | 5'-GCA GGA GAC T-3' (SEQ ID NO:273) | 1.2 |
| 102 | TGNR* | QAHR* | RDHT | 5'-TGG GGA GAT T-3' (SEQ ID NO:274) | 1.1 |
| 103 | RDHT | QSNR3 | RDER1 | 5'-GCG GAA TGG A-3' (SEQ ID NO:275) | 1.1 |
| 104 | QSHR3 | RDHT | DGAR* | 5'-GTC TGG GGA C-3' (SEQ ID NO:276) | 1.1 |
| 105 | RDER1 | RSHR | RSNR | 5'-GAG GGG GCG T-3' (SEQ ID NO:277) | 1.1 |
| 106 | VSTR | VSTR | QSSR1 | 5'-GAC GCT GCT T-3' (SEQ ID NO:278) | 1.1 |
| 107 | TGNR* | QAHR* | QAHR* | 5'-ATC TCC TCC-3' | 1.1 |
| 108 | DGHR* | QGOR* | ROKR* | 5'-GGG GCA GGC G-3' (SEQ ID NO:279) | 1.1 |
| 109 | RSHR | RSHR | TGNR* | 5'-GAT GGG GGG-3' | 1.1 |
| 110 | QSNV2 | QSNV2 | VSNV2 QSNV2 | 5'-AAA AAA AAA G-3' (SEQ ID NO:280) | 1.1 |
| 111 | RSNR | QSHT | QSHR2 | 5'-GGA AGA GAG G-3' (SEQ ID NO:281) | 1.1 |
| 112 | QSNV2 | RSHR | QSNV2 | 5'-CAA GGG AAA A-3' (SEQ ID NO:282) | 1.1 |
| 113 | QGDR* | TGNR* | TDKR* | 5'-GGT GAT GCA C-3 (SEQ ID NO:283) | 1.1 |
| 114 | RDER1 | DGAR* | RDTN* | 5'-AAG GTC GCG G-3' (SEQ ID NO:284) | 1.0 |
| 115 | QAHR* | QSDR* | RDKR* | 5'-GGG GCT GGA G-3' (SEQ ID NO:285) | 1.0 |
| 116 | VSSR | TDKR* | RDHT | 5'-GGG GGG GTT-3' | 1.0 |
| 117 | QSSR* | TDKR* | RDKR* | 5'-GGG GGT GTA C-3' (SEQ ID NO:286) | 1.0 |
| 118 | QSDR* | TGNR* | TDKR* | 5'-GGT GAT GCT C-3' (SEQ ID NO:287) | 1.0 |
| 119 | CSNR1 | QSHT | VSSR | 5'-GTT TGA GAC A-3' (SEQ ID NO:288) | 1.0 |
| 120 | RSNR | QSHR3 | DGHR* | 5'-GGC GGA GAG-3' | 1.0 |
| 121 | VSNV | QSNV2 | VSTR | 5'-GCT AAA AAT C-3' (SEQ ID NO:289) | 1.0 |
| 122 | QSDR* | QAHR* | QTHQ | 5'-AGA GGA GCT T-3' (SEQ ID NO:290) | 1.0 |
| 123 | RSHR | ISNR | QSHT | 5'-TGA GAT GGG G-3' (SEQ ID NO:291) | 1.0 |
| 124 | QSNR* | QAHR* | RDKR* | 5'-TTC TCC CCC-3' | 0.9 |
| 125 | DGHR* | RDHT | QSDR* | 5'-GCT TGG GGC T-3' (SEQ ID NO:292) | 0.9 |
| 126 | RDER1 | QGDR | VSSR | 5'-GTT GGG GCG G-3' (SEQ ID NO:293) | 0.9 |
| 127 | QSDR* | QSDR* | QAHR* | 5'-GGA GCT GCT T-3' (SEQ ID NO:294) | 0.9 |
| 128 | DGHR* | RSNR | DGNV* | 5'-AAC GAG GGC-3' | 0.9 |
| 129 | QSHR3 | QGDR* | TGNR* | 5'-GAT GCA GGA C-3' (SEQ ID NO:295) | 0.9 |
| 130 | QSNR1 | DRDR* | TGNR* | 5'-GAT GCC GAA-3' | 0.9 |
| 131 | DGAR* | RDHT | DGHR* | 5'-GGC CGG GTC G-3' (SEQ ID NO:296) | 0.9 |
| 132 | TDKR* | TDKR* | TGNR* | 5'-GAT GGT GGT T-3' (SEQ ID NO:297) | 0.9 |
| 133 | NDTR* | QSNR* | QAHR* | 5'-GGA GAA GTT-3' | 0.9 |
| 134 | TGNR* | QGTR* | QSDR* | 5'-GCT ACA GAT-3' | 0.8 |
| 135 | RDER1 | TDKR* | DRDR* | 5'-GCC GGG GCG G-3' (SEQ ID NO:298) | 0.8 |
| 136 | QSNR1 | VSSR | DGNV* | 5'-AAC GTT GAA-3' | 0.8 |
| 137 | DGAR* | QSSR* | GRER* | 5'-GCC GTA GTC-3' | 0.8 |
| 138 | CSNR1 | RSHR | VSTR | 5'-GCT GGG GAC T-3' (SEQ ID NO:299) | 0.8 |
| 139 | QSSR* | QSDR* | QSSR* | 5'-GTA GCT GTA A-3' (SEQ ID NO:300) | 0.8 |
| 140 | QSNV2 | RSNR | QSSR1 | 5'-GTA GAG AAA-3' | 0.8 |
| 141 | TDKR* | DGHR* | RDKR* | 5'-GGG GGC GGT T-3' (SEQ ID NO:301) | 0.8 |
| 142 | TGNR* | QSDR* | TDKR* | 5'-GGT GCT GAT T-3' (SEQ ID NO:302) | 0.8 |
| 143 | DRDR* | DGHR* | QSSR1 | 5'-GTA GGC GCC-3' | 0.8 |
| 144 | QAHR* | QSSR* | QGDR* | 5'-GCA GTA GGA G-3' (SEQ ID NO:303) | 0.8 |
| 145 | QSHT | RSNR | QSDR* | 5'-GCT GAG AGA-3' | 0.8 |
| 146 | DGNV* | QGDR* | DGNV* | 5'-AAC GCA AAC-3' | 0.7 |
| 147 | QGDR* | TDKR* | DGAR* | 5'-GTC GGG GCA-3' | 0.7 |
| 148 | TDKR* | QSNR1 | DGNR* | 5'-GAC GAA GGG G-3' (SEQ ID NO:304) | 0.7 |
| 149 | QSNR* | QGDR* | NDTR* | 5'-GTT GCT GAA-3' | 0.7 |
| 150 | QSNR* | RDKR* | DGHR* | 5'-GGC GGG GAA-3' | 0.7 |
| 151 | DGNV* | QGDR* | DGNR* | 5'-GAC GCA AAC-3' | 0.7 |
| 152 | QSDR* | DGNR* | DGNR* | 5'-GAC GAC GCT T-3' (SEQ ID NO:305) | 0.7 |
| 153 | CSNR1 | TGNR* | DGNR* | 5'-GAC GAT GAA-3' | 0.6 |

| | | | | | |
|---|---|---|---|---|---|
| * Domains followed by the "*" symbol are zinc finger domains obtained by mutagenesis. The domains not so indicated are human zinc finger domains according to the present invention. The target composite sequences were designed by juxtaposing individual target sites of each domain. | | | | | |

The distribution of results relative to the high and low stringency criteria are tabulated in Tables 11 and 12. As shown in Table 11, 31 of 153 chimeric zinc finger proteins demonstrated greater than 2-fold repression, the high stringency criterion (RF 2; RF = fold repression). Table 12 demonstrates that, of the proteins constructed entirely from naturally-occurring human zinc finger domains, 28.1% (27 of 96) exceeded the high stringency criterion and 59.4% exceeded the low stringency criterion (RF 1.5). Of the proteins constructed from two naturally-occurring zinc finger domains and one mutated domain, 33.3% exceeded the high stringency criterion, and only 20% exceeded the low stringency criterion.

In contrast, of the 17 proteins constructed from one human domain and two mutated domains, only one protein (5.9%) exceeded the high stringency criterion. and only two proteins (11.8%) exceeded the low stringency criterion. Strikingly, no zinc finger proteins composed exclusively of mutated domains satisfied the high stringency criterion in the repression assay. Only one such protein (4%) satisfied the low stringency criterion. These results indicate that naturally-occurring human zinc finger domains are in general much better building blocks for the construction of new DNA-binding proteins than mutated domains.

**Table 11.**

| No of Tested ZFPs (A) | No. of Active ZFPs (B) | | B/A (%) | |
|---|---|---|---|---|
| | RF> 1.5 | RF> 2.0 | RF> 1.5 | RF>2.0 |
| 153 | 65 | 31 | 42.5 | 20.3 |

**Table 12.**

| Compositions of | | No. of | No. of | Active | B/A(%) | |
|---|---|---|---|---|---|---|
| ZFPs | | Tested | ZFPs (B) | | | |
| Human D | Mutated D | ZFPs (A) | RF>1.5 | RF>2.0 | RF> 1.5 | RF>2.0 |
| 3 | 0 | 96 | 57 | 27 | 59.4 | 28.1 |
| 2 | 1 | 15 | 5 | 3 | 33.3 | 20 |
| 1 | 2 | 17 | 2 | 1 | 11.8 | 5.9 |
| 0 | 3 | 25 | 1 | 0 | 4.0 | 0 |

## Claims

1. A library comprising a plurality of polypeptides, each polypeptide comprising a first and a second zinc finger domain, wherein
(1) the first and second zinc finger domains of each polypeptide are each identical to a zinc finger domain from a naturally occurring protein, and either
(i) do not occur in the same naturally occurring protein, or
(ii) occur in the same naturally occurring protein but are positioned in said polypeptide in a configuration that differs from their relative positions in said naturally-occurring protein,
(2) the first zinc finger domain differs between the polypeptides of the plurality, and
(3) the second zinc finger domain differs between the polypeptides of the plurality.

2. The library of claim 1, wherein each polypeptide of the plurality binds to a target DNA site with a dissociation constant (K_{d}) of less than 5 nM.

3. The library of claim 1, wherein the first zinc finger domain of at least one polypeptide of the plurality is selected from the zinc finger domains listed in any of Tables 5, 6, and 7.

4. The library of claim 3, wherein the first zinc finger domain of each polypeptide of the plurality is selected from the zinc finger domains listed in any of Tables 5, 6, and 7.

5. The library of claim 3, wherein the first and second zinc finger domains of at least one polypeptide of the plurality are selected from the zinc finger domains listed in any of Tables 5, 6, and 7.

6. The library of claim 1, wherein the naturally occurring protein is a eukaryotic protein.

7. The library of claim 6, wherein the naturally occurring protein is a mammalian protein.

8. The library of claim 7, wherein the naturally occurring protein is a human protein.

9. The library of claim 1, wherein each polypeptide of the plurality is immobilized on a solid support.

10. The library of claim 1, wherein each polypeptide of the plurality is displayed on the surface of a virus or viral particle.

11. The library of claim 1, wherein each polypeptide of the plurality further comprises a third zinc finger domain.

12. The library of claim 11, wherein the third zinc finger domain is a domain of a naturally occurring protein.

13. The library of claim 11, wherein the third zinc finger domain is not a domain of a naturally occurring protein.

14. The library of claim 13, wherein the third zinc finger domain differs from a domain of a naturally occurring protein by insertion, deletion, or substitution of no more than six amino acids.

15. The library of claim 1, wherein each polypeptide of the plurality further comprises a transcriptional regulatory domain.

16. The library of claim 1, wherein the plurality comprises at least 100 different polypeptides.

17. A library comprising a plurality of polynucleotides, each polynucleotide encoding a different polypeptide of the library of any one of claims 1 to 8 or 11 to 16.

18. The library of claim 17, wherein each polynucleotide is a segment of a plasmid or phagemid.

19. The library of claim 17, wherein each polynucleotide resides within a cell.

20. The library of claim 19, wherein the cell is a eukaryotic cell.

21. The library of claim 20, wherein the cell is a yeast cell.

22. The library of claim 19, wherein the cell contains a heterologous reporter construct comprising a reporter gene operably linked to a promoter.

23. The library of claim 17, wherein each polynucleotide is packaged into a virus or a viral particle.

24. A polypeptide comprising a first and a second zinc finger domain, wherein the first and second zinc finger domains are from different naturally occurring polypeptides, and wherein each zinc finger domain has a sequence selected from Tables 5, 6, and 7.

25. A polypeptide of claim 24 that further comprises a third zinc finger domain, wherein the set of three zinc finger domains is listed in a row of Table 10.

26. A nucleic acid sequence comprising a polynucleotide that encodes the polypeptide of claim 24.

27. A method of producing the library of claim 17, which comprises the steps of:
(a) providing a set of nucleic acids, each nucleic acid of the set comprising a sequence encoding a zinc finger domain from a naturally occurring protein; and
(b) joining each nucleic acid of the set to one or more other nucleic acids of the set to form a plurality of chimeric nucleic acids, wherein the chimeric nucleic acids comprise either
i) at least two sequences that encode zinc finger domains from different naturally occurring proteins, or
ii) at least two sequences that encode zinc finger domains from the same naturally occurring protein but which zinc finger domains are positioned in said polypeptide in a configuration that differs from their relative positions in said naturally-occurring protein.

28. The method of claim 27, wherein step (a) comprises amplifying a collection of polynucleotides encoding zinc finger domains from genomic DNA, a messenger RNA (mRNA) mixture, or a complementary DNA (cDNA) mixture using an oligonucleotide primer that anneals to sequences that encode a conserved domain boundary.

29. The method of claim 27, wherein step (a) comprises:
(i) selecting a plurality of zinc finger domains, each domain having specificity for a sequence within a target site of interest; and
(ii) providing a plurality of polynucleotides, each polynucleotide encoding at least one of the selected zinc finger domains, thereby providing the set of polynucleotides,

30. The method of claim 29, wherein the plurality of zinc finger domains are selected by querying a database that includes information relating zinc finger domains to their respective binding sites.

31. A method of generating an artificial zinc finger polypeptide that specifically binds to a target DNA site, the method comprising:
providing the polypeptide library of claim 1;
contacting the target DNA site with the polypeptides of the library; and,
identifying one or more polypeptides that specifically bind to the target DNA site.

32. The method of claim 31, wherein the polypeptides of the library are immobilized on a solid support.

33. The method of claim 31, wherein each polypeptide of the library is displayed on the surface of a virus or viral particle.

34. A method of identifying a nucleic acid encoding a zinc finger polypeptide that specifically recognizes a target DNA site, the method comprising:
providing the library of polynucleotides of claim 17;
providing cells containing a reporter gene operably linked to a target DNA site;
expressing the plurality of polynucleotides in the cells;
identifying a cell having altered expression of the reporter gene relative to the level of expression in the absence of a polypeptide that recognizes the target DNA site level; and
identifying a polynucleotide expressed in the cell, the polynucleotide being a polynucleotide of the plurality, thereby identifying a polynucleotide encoding a polypeptide that specifically recognizes the target DNA site.

35. The method of claim 34, further comprising the step of modifying the amino acid sequence of the identified zinc finger polypeptide without altering the binding specificity of the zinc finger polypeptide for the target DNA site.

36. The method of claim 34, wherein the target site comprises at least six predetermined nucleotides.

37. The method of claim 34, wherein the cells are yeast cells.

38. The method of claim 34, further comprising the step of introducing the polynucleotides into each of the cells.

39. The method of claim 34, further comprising the step of fusing the cells containing the reporter gene to a cell that includes the polynucleotides of the library.

## Patentansprüche

1. Bibliothek, umfassend eine Vielzahl von Polypeptiden, wobei jedes Polypeptid eine erste und eine zweite Zinkfingerdomäne umfasst, wobei
(1) jeweils die erste und die zweite Zinkfingerdomäne jedes Polypeptids mit einer Zinkfingerdomäne von einem natürlich vorkommenden Protein identisch sind und entweder
(i) nicht in dem selben natürlich vorkommenden Protein vorkommen, oder
(ii) in dem selben natürlich vorkommenden Protein vorkommen, aber in dem Polypeptid in einer Konfiguration angeordnet sind, die sich von ihren relativen Positionen in dem natürlich vorkommenden Protein unterscheidet,
(2) die erste Zinkfingerdomäne innerhalb der Vielzahl von Polypeptiden unterschiedlich ist, und
(3) die zweite Zinkfingerdomäne innerhalb der Vielzahl von Polypeptiden unterschiedlich ist.

2. Bibliothek nach Anspruch 1, wobei jedes Polypeptid aus der Vielzahl von Polypeptiden an eine Ziel-DNA-Stelle mit einer Dissoziationskonstante (K_{d}) von weniger als 5nM bindet.

3. Bibliothek nach Anspruch 1, wobei die erste Zinkfingerdomäne wenigstens eines Polypeptids der Vielzahl von Polypeptiden ausgewählt ist aus den Zinkfinderdomänen, die in einer der Tafeln 5, 6 und 7 aufgelistet sind.

4. Bibliothek nach Anspruch 3, wobei die erste Zinkfingerdomäne eines jeden Polypeptids der Vielzahl von Polypeptiden ausgewählt ist aus den Zinkfingerdomänen, die in einer der Tabellen 5, 6 und 7 aufgelistet sind.

5. Bibliothek nach Anspruch 3, wobei die erste und die zweite Zinkfingerdomäne wenigstens eines Polypeptids der Vielzahl von Polypeptiden ausgewählt sind aus den Zinkfingerdomänen, die in einer der Tabellen 5, 6 und 7 aufgelistet sind.

6. Bibliothek nach Anspruch 1, wobei das natürlich vorkommende Protein ein eukaryotisches Protein ist.

7. Bibliothek nach Anspruch 6, wobei das natürlich vorkommende Protein ein Säugetierprotein ist.

8. Bibliothek nach Anspruch 7, wobei das natürlich vorkommende Protein ein humanes Protein ist.

9. Bibliothek nach Anspruch 1, wobei jedes Polypeptid der Vielzahl von Polypeptiden auf einem festen Träger immobilisiert ist.

10. Bibliothek nach Anspruch 1, wobei jedes Polypeptid der Vielzahl von Polypeptiden auf der Oberfläche eines Virus oder viralen Partikels dargestellt ist.

11. Bibliothek nach Anspruch 1, wobei jedes Polypeptid der Vielzahl von Polypeptiden weiterhin eine dritte Zinkfingerdomäne umfasst.

12. Bibliothek nach Anspruch 11, wobei die dritte Zinkfingerdomäne eine Domäne eines natürlich vorkommenden Proteins ist.

13. Bibliothek nach Anspruch 11, wobei die dritte Zinkfingerdomäne keine Domäne eines natürlich vorkommenden Proteins ist.

14. Bibliothek nach Anspruch 13, wobei sich die dritte Zinkfingerdomäne von einer Domäne eines natürlich vorkommenden Proteins durch Insertion, Deletion oder Substitution von nicht mehr als sechs Aminosäuren unterscheidet.

15. Bibliothek nach Anspruch 1, wobei jedes Polypeptid der Vielzahl von Polypeptiden weiterhin eine transkriptionelle regulatorische Domäne umfasst.

16. Bibliothek nach Anspruch 1, wobei die Vielzahl von Polypeptiden wenigstens 100 verschiedene Polypeptide umfasst.

17. Bibliothek, umfassend eine Vielzahl von Polynukleotiden, wobei jedes Polynukleotid für ein unterschiedliches Polypeptid der Bibliothek nach einem der Ansprüche 1 bis 8 oder 11-16 kodiert.

18. Bibliothek nach Anspruch 17, wobei jedes Polynukleotid ein Segment eines Plasmids oder Phagemids ist.

19. Bibliothek nach Anspruch 17, wobei jedes Polynukleotid innerhalb einer Zelle liegt.

20. Bibliothek nach Anspruch 19, wobei die Zelle einer eukaryotische Zelle ist.

21. Bibliothek nach Anspruch 20, wobei die Zelle eine Hefezelle ist.

22. Bibliothek nach Anspruch 19, wobei die Zelle ein heterologes Reporterkonstrukt enthält, umfassend ein Reportergen, das mit einem Promoter operabel verknüpft ist.

23. Bibliothek nach Anspruch 17, wobei jedes Polynukleotid in ein Virus oder ein virales Partikel verpackt ist.

24. Polypeptid, umfassend eine erste und eine zweite Zinkfingerdomäne, wobei die erste und zweite Zinkfingerdomäne aus verschiedenen natürlich vorkommenden Polypeptiden stammen, und wobei jede Zinkfingerdomäne eine Sequenz hat, die ausgewählt ist aus den Tabellen 5, 6 und 7.

25. Polypeptid nach Anspruch 24, das weiterhin eine dritte Zinkfingerdomäne umfasst, wobei der Satz von drei Zinkfingerdomänen in einer Reihe der Tabelle 10 aufgelistet ist.

26. Nukleinsäuresequenz, umfassend ein Polynukleotid, das für das Polypeptid von Anspruch 24 kodiert.

27. Verfahren zum Herstellen der Bibliothek nach Anspruch 17, welches die Schritte umfasst:
(a) Bereitstellen eines Satzes Nukleinsäuren, wobei jede Nukleinsäure des Satzes eine Sequenz umfasst, die für eine Zinkfingerdomäne eines natürlich vorkommenden Proteins kodiert; und
(b) Verbinden jeder Nukleinsäure des Satzes mit einer oder mehreren anderen Nukleinsäuren des Satzes, um eine Vielzahl an chimären Nukleinsäuren zu bilden, wobei die chimären Nukleinsäuren entweder
(i) wenigstens zwei Sequenzen, die für Zinkfingerdomänen aus verschiedenen natürlich vorkommenden Proteinen kodieren, oder
(ii) wenigstens zwei Sequenzen umfassen, die für Zinkfingerdomänen aus dem selben natürlich vorkommenden Protein kodieren, wobei aber die Zinkfingerdomänen in dem Polypeptid in einer Konfiguration angeordnet sind, die sich von ihren relativen Positionen in dem natürlich vorkommenden Protein unterscheidet.

28. Verfahren nach Anspruch 27, wobei Schritt a) das Amplifizieren einer Sammlung von Polynukleotiden, die für Zinkfingerdomänen kodieren, aus genomischer DNA, einer Messenger-RNA (mRNA)-Mischung oder einer komplementären DNA (cDNA)-Mischung unter Verwendung eines Oligonukleotidprimers umfasst, der sich an Sequenzen anlagert, die für eine konservierte Domänengrenze kodieren.

29. Verfahren nach Anspruch 27, wobei Schritt a) umfasst:
(i) Auswählen einer Vielzahl von Zinkfingerdomänen, wobei jede Domäne eine Spezifität für eine Sequenz innerhalb einer Zielstelle von Interesse aufweist; und
(ii) Bereitstellen einer Vielzahl von Polynukleotiden, wobei jedes Polynukleotid für wenigstens eine der ausgewählten Zinkfingerdomänen kodiert, wodurch der Satz Polynukleotide bereitgestellt wird.

30. Verfahren nach Anspruch 29, wobei die Vielzahl von Zinkfingerdomänen ausgewählt werden, indem eine Datenbank durchsucht wird, die Informationen enthält, welche Zinkfingerdomänen mit ihren jeweiligen Bindungsstellen in Beziehung setzt.

31. Verfahren zum Erzeugen eines künstlichen Zinkfingerpolypeptids, das spezifisch an eine Ziel-DNA-Stelle bindet, wobei das Verfahren umfasst:
Bereitstellen der Polypeptidbibliothek nach Anspruch 1,
In-Kontakt-Bringen der Ziel-DNA-Stelle mit den Polypeptiden der Bibliothek; und
Identifizieren von einem oder mehreren Polypeptiden, die spezifisch an die Ziel-DNA-Stelle binden.

32. Verfahren nach Anspruch 31, wobei die Polypeptide der Bibliothek auf einem festen Träger immobilisiert sind.

33. Verfahren nach Anspruch 31, wobei jedes Polypeptid der Bibliothek auf der Oberfläche eines Virus oder viralen Partikels dargestellt wird.

34. Verfahren zum Identifizieren einer Nukleinsäure, die für ein Zinkfingerpolypeptid kodiert, das spezifisch eine Ziel-DNA-Stelle erkennt, wobei das Verfahren umfasst:
Bereitstellen der Bibliothek von Polynukleotiden nach Anspruch 17;
Bereitstellen von Zellen, enthaltend ein Reportergen, das operabel mit einer Ziel-DNA-Stelle verknüpft ist;
Exprimieren der Vielzahl von Polynukleotiden in den Zellen;
Identifizieren einer Zelle mit veränderter Expression des Reportergens relativ zu dem Expressionsniveau in der Abwesenheit eines Polypeptids, das die Ziel-DNA-Stelle erkennt; und
Identifizieren eines Polynukleotids, das in der Zelle exprimiert wird, wobei das Polynukleotid ein Polynukleotid aus der Vielzahl von Polynukleotiden ist, wodurch ein Polynukleotid identifiziert wird, das für ein Polypeptid kodiert, das spezifisch die Ziel-DNA-Stelle erkennt.

35. Verfahren nach Anspruch 34, weiterhin umfassend den Schritt der Modifikation der Aminosäuresequenz des identifizierten Zinkfingerpolypeptids, ohne die Bindungsspezifität des Zinkfingerpolypeptids für die Ziel-DNA-Stelle zu verändern.

36. Verfahren nach Anspruch 34, wobei die Ziel-Stelle wenigstens sechs vorbestimmte Nukleotide umfasst.

37. Verfahren nach Anspruch 34, wobei die Zellen Hefezellen sind.

38. Verfahren nach Anspruch 34, weiterhin umfassend den Schritt des Einbauens der Polynukleotide in jede der Zellen.

39. Verfahren nach Anspruch 34, weiterhin umfassend den Schritt der Fusionierung der Zellen, enthaltend das Reportergen, mit einer Zelle, die die Polynukleotide der Bibliothek enthält.

## Revendications

1. Banque comprenant une pluralité de polypeptides, chaque polypeptide comprenant un premier et un deuxième domaines en doigt de zinc, où
(1) le premier et le deuxième domaines en doigt de zinc de chaque polypeptide sont chacun identiques à un domaine en doigt de zinc provenant d'une protéine existant à l'état naturel, et
(i) soit ils n'apparaissent pas dans la même protéine existant à l'état naturel,
(ii) soit ils apparaissent dans la même protéine existant à l'état naturel mais sont positionnés dans ledit polypeptide dans une configuration qui diffère de leurs positions relatives dans ladite protéine existant à l'état naturel,
(2) le premier domaine en doigt de zinc diffère entre les polypeptides de la pluralité, et
(3) le deuxième domaine en doigt de zinc diffère entre les polypeptides de la pluralité.

2. Banque selon la revendication 1, dans laquelle chaque polypeptide de la pluralité se lie à un site cible sur l'ADN avec une constante de dissociation (K_{d}) inférieure à 5 nM.

3. Banque selon la revendication 1, dans laquelle le premier domaine en doigt de zinc d'au moins un polypeptide de la pluralité est choisi parmi les domaines en doigt de zinc énumérés dans l'un quelconque des tableaux 5, 6 et 7.

4. Banque selon la revendication 3, dans laquelle le premier domaine en doigt de zinc de chaque polypeptide de la pluralité est choisi parmi les domaines en doigt de zinc énumérés dans l'un quelconque des tableaux 5, 6 et 7.

5. Banque selon la revendication 3, dans laquelle les premier et deuxième domaines en doigt de zinc d'au moins un polypeptide de la pluralité sont choisis parmi les domaines en doigt de zinc énumérés dans l'un quelconque des tableaux 5, 6 et 7.

6. Banque selon la revendication 1, où la protéine existant à l'état naturel est une protéine eucaryote.

7. Banque selon la revendication 6, où la protéine existant à l'état naturel est une protéine de mammifère.

8. Banque selon la revendication 7, où la protéine existant à l'état naturel est une protéine humaine.

9. Banque selon la revendication 1, dans laquelle chaque polypeptide de la pluralité est immobilisé sur un support solide.

10. Banque selon la revendication 1, dans laquelle chaque polypeptide de la pluralité est présenté à la surface d'un virus ou d'une particule virale.

11. Banque selon la revendication 1, dans laquelle chaque polypeptide de la pluralité comprend en outre un troisième domaine en doigt de zinc.

12. Banque selon la revendication 11, dans laquelle le troisième domaine en doigt de zinc est un domaine d'une protéine existant à l'état naturel.

13. Banque selon la revendication 11, dans laquelle le troisième domaine en doigt de zinc n'est pas un domaine d'une protéine existant à l'état naturel.

14. Banque selon la revendication 13, dans laquelle le troisième domaine en doigt de zinc diffère d'un domaine d'une protéine existant à l'état naturel par insertion, délétion ou substitution de pas plus de six acides aminés.

15. Banque selon la revendication 1, dans laquelle chaque polypeptide de la pluralité comprend en outre un domaine de régulation de la transcription.

16. Banque selon la revendication 1, dans laquelle la pluralité comprend au moins 100 polypeptides différents.

17. Banque comprenant une pluralité de polynucléotides, chaque polynucléotide codant pour un polypeptide différent de la banque selon l'une quelconque des revendications 1 à 8 ou 11 à 16.

18. Banque selon la revendication 17, dans laquelle chaque polynucléotide est un segment d'un plasmide ou d'un phagemide.

19. Banque selon la revendication 17, dans laquelle chaque polynucléotide réside au sein d'une cellule.

20. Banque selon la revendication 19, où la cellule est une cellule eucaryote.

21. Banque selon la revendication 20, où la cellule est une cellule de levure.

22. Banque selon la revendication 19, où la cellule contient un rapporteur hétérologue comprenant un gène rapporteur lié de manière fonctionnelle à un promoteur.

23. Banque selon la revendication 17, dans laquelle chaque polynucléotide est encapsidé dans un virus ou une particule virale.

24. Polypeptide comprenant un premier et un deuxième domaines en doigt de zinc, où le premier et le deuxième domaines en doigt de zinc proviennent de polypeptides différents existant à l'état naturel, et où chaque domaine en doigt de zinc a une séquence choisie parmi les tableaux 5, 6 et 7.

25. Polypeptide selon la revendication 24, comprenant en outre un troisième domaine en doigt de zinc, et dans lequel l'ensemble des trois domaines en doigt de zinc est énuméré sur une ligne du tableau 10.

26. Séquence d'acide nucléique comprenant un polynucléotide qui code pour le peptide selon la revendication 24.

27. Procédé de production de la banque selon la revendication 17, qui comprend les étapes consistant à :
(a) fournir un ensemble d'acides nucléiques, dans lequel chaque acide nucléique comprend une séquence codant pour un domaine en doigt de zinc provenant d'une protéine existant à l'état naturel ; et
(b) lier chaque acide nucléique de l'ensemble à un ou plusieurs autres acides nucléiques de l'ensemble pour former une pluralité d'acides nucléiques chimériques, dans laquelle les acides nucléiques chimériques comprennent soit
i) au moins deux séquences qui codent pour des domaines en doigt de zinc provenant de protéines existant à l'état naturel différentes, soit
ii) au moins deux séquences qui codent pour des domaines en doigt de zinc provenant de la même protéine existant à l'état naturel, lesdits domaines en doigt de zinc étant positionnés dans ledit polypeptide dans une configuration qui diffère de leurs positions relatives dans ladite protéine existant à l'état naturel.

28. Procédé selon la revendication 27, dans lequel l'étape (a) comprend l'amplification d'une collection de polynucléotides codant pour des domaines en doigt de zinc provenant d'ADN génomique, d'un mélange d'ARN messagers (ARNm) ou d'un mélange d'ADN complémentaires (ADNc) en utilisant une amorce oligonucléotidique qui s'hybride à des séquences codant pour un domaine frontière conservé.

29. Procédé selon la revendication 27, dans lequel l'étape (a) comprend :
(i) la sélection d'une pluralité de domaines en doigt de zinc, chaque domaine étant spécifique d'une séquence au sein d'un site cible d'intérêt ; et
(ii) la fourniture d'une pluralité de polynucléotides, chaque polynucléotide codant pour au moins l'un des domaines en doigt de zinc sélectionnés, fournissant de cette manière l'ensemble des polynucléotides.

30. Procédé selon la revendication 29, dans lequel la pluralité de domaines en doigt de zinc sont choisis en cherchant dans une base de données comprenant des informations mettant en relation les domaines en doigt de zinc avec leurs sites de liaison respectifs.

31. Procédé de génération d'un polypeptide en doigt de zinc artificiel se liant spécifiquement à un site d'ADN cible, le procédé comprenant :
la fourniture de la banque de polypeptides selon la revendication 1 ;
la mise en contact du site d'ADN cible avec les polypeptides de la banque ; et
l'identification d'un ou plusieurs polypeptides qui se lient spécifiquement au site d'ADN cible.

32. Procédé selon la revendication 31, dans lequel les polypeptides de la banque sont immobilisés sur un support solide.

33. Procédé selon la revendication 31, dans lequel chaque polypeptide de la banque est présenté à la surface d'un virus ou d'une particule virale.

34. Procédé d'identification d'un acide nucléique codant pour un polypeptide en doigt de zinc reconnaissant spécifiquement un site d'ADN cible, le procédé comprenant :
la fourniture de la banque de polynucléotides selon la revendication 17 ;
la fourniture de cellules contenant un gène rapporteur lié de manière fonctionnelle à un site d'ADN cible ;
l'expression de la pluralité de polynucléotides dans les cellules ;
l'identification d'une cellule présentant une expression modifiée du gène rapporteur par rapport au taux d'expression en l'absence du polypeptide reconnaissant le site d'ADN cible ; et
l'identification d'un polynucléotide exprimé dans la cellule, le polynucléotide étant un polynucléotide de la pluralité, permettant ainsi d'identifier un polynucléotide codant pour un polypeptide reconnaissant spécifiquement le site d'ADN cible.

35. Procédé selon la revendication 34, comprenant en outre l'étape consistant à modifier la séquence d'acides aminés du polypeptide en doigt de zinc identifié, sans modifier la spécificité de liaison du polypeptide en doigt de zinc au site d'ADN cible.

36. Procédé selon la revendication 34, dans lequel le site ciblé comprend au moins six nucléotides prédéterminés.

37. Procédé selon la revendication 34, dans lequel les cellules sont des cellules de levures.

38. Procédé selon la revendication 34, comprenant en outre l'étape consistant à introduire les polynucléotides dans chacune des cellules.

39. Procédé selon la revendication 34, comprenant en outre l'étape consistant à fusionner les cellules contenant le gène rapporteur à une cellule qui comprend les polynucléotides de la banque.


### SEQUENCE LISTING

<110> ToolGen, Inc.
<120> ZINC FINGER DOMAIN LIBRARIES
<130> A20952/TGI
<150> US60/374,355
   <151> 2002-04-22
<150> US60/313,402
   <151> 2001-08-17
<160> 305
<170> FastSEQ for Windows Version 4.0
<210> 1
   <211> 10
   <212> DNA
   <213> HIV-1
<400> 1
   gacatcgagc
   10
<210> 2
   <211> 10
   <212> DNA
   <213> HIV-1
<400> 2
   gcagctgctt
   10
<210> 3
   <211> 10
   <212> DNA
   <213> HIV-1
<400> 3
   gctggggact
<210> 4
   <211> 10
   <212> DNA
   <213> Homo sapiens
<400> 4
   agggtggagt
   10
<210> 5
   <211> 10
   <212> DNA
   <213> Homo sapiens
<400> 5
   gctgagacat
   10
<210> 6
   <211> 47
   <212> DNA
   <213> Artificial Sequence
<220>
   <223> optimal binding site
<400> 6
   ccggcgtgggcggctgcgtgggcgtgcgtgggcggactgc gtgggcg 47
<210> 7
   <211> 47
   <212> DNA
   <213> Artificial Sequence
<220>
   <223> optimal binding site
<400> 7
   tcgacgcccacgcagtccgcccacgcacgcccacgcagccgcccacg 47
<210> 8
   <211> 49
   <212> DNA
   <213> HIV-1
<400> 8
   ccggcgagcgggcggtcgagcgggcgtgagcgggcggatcgagcgggcg 49
<210> 9
   <211> 49
   <212> DNA
   <213> HV-1
<400> 9
   tcgacgcccgctcgatccgcccgctcacgcccgctcgaccgcccgctcg 49
<210> 10
   <211> 50
   <212> DNA
   <213> HIV-1
<400> 10
   ccggctgcttgggcggctgcttgggcgtgcttgggcgggctgcttgggcg 50
<210> 11
   <211> 50
   <212> DNA
   <213> HIV-1
<400> 11
   tcgacgcccaagcagcccgcccaagcacgcccaagcagccgcccaagcag 50
<210> 12
   <211> 47
   <212> DNA
   <213> HIV-1
<400> 12
   ccggactgggcgggggactgggcgtgactgggcggagggactgggcg 47
<210> 13
   <211> 47
   <212> DNA
   <213> HIV-1
<400> 13
   tcgacgcccagtccctccgcccagtcacgcccagtcccccgcccagt 47
<210> 14
   <211> 47
   <212> DNA
   <213> Homo sapiens
<400> 14
   ccggagtgggcggtggagtgggcgtgagtgggcggatggagtgggcg 47
<210> 15
   <211> 47
   <212> DNA
   <213> Homo sapiens
<400> 15
   tcgacgcccactccatccgcccactcacgcccactccaccgcccact 47
<210> 16
   <211> 48
   <212> DNA
   <213> Homo sapiens
<400> 16
   ccggacatgggcggagacatgggcgtacatgggcggaagacatgggcg 48
<210> 17
   <211> 48
   <212> DNA
   <213> Homo sapiens
<400> 17
   tcgacgcccatgtcttccgcccatgtacgcccatgtctccgcccatgt 48
<210> 18
   <211> 120
   <212> DNA
   <213> Artificial Sequence
<220>
   <223> plasmid sequence
<221> CDS
   <222> (1)...(81)
<400> 18
   aaa gag ggt ggg tcg acc ttc cgg act ggc cag gaa cgc cca gat ccg 48
   Lys Glu Gly Gly Ser Thr Phe Arg Thr Gly Gln Glu Arg Pro Asp Pro
   1 5 10 15
   cgg gaa ttc aga tct act agt gcg gcc gct aag taagtaagac gtcgagctcg 101
   Arg Glu Phe Arg Ser Thr Ser Ala Ala Ala Lys
   20 25
   ccatcgcggtggaagcttt 120
<210> 19
   <211> 27
   <212> PRT
   <213> Artificial Sequence
<220>
   <223> plasmid sequence
<400> 19
   Lys Glu Gly Gly Ser Thr Phe Arg Thr Gly Gln Glu Arg Pro Asp Pro
   1 5 10 15
   Arg Glu Phe Arg Ser Thr Ser Ala Ala Ala Lys
   20 25
<210> 20
   <211> 303
   <212> DNA
   <213> Artificial Sequence
<220>
   <223> plasmid sequence
<221> CDS
   <222> (25)...(291)
<400> 20
   gggtcgacct tccggactgg ccag gaa cgc cca tat gct tgc cct gtc gag 51
   Glu Arg Pro Tyr Ala Cys Pro Val Glu
   1 5
   tcc tgc gat cgc cgc ttt tct cgc tcg gat gag ctt acc cgc cat atc 99
   Ser Cys Asp Arg Arg Phe Ser Arg Ser Asp Glu Leu Thr Arg His Ile
   10 15 20 25
   cgc atc cac act ggc cag aag ccc ttc cag tgt cga atc tgc atg cgt 147
   Arg Ile His Thr Gly Gln Lys Pro Phe Gln Cys Arg Ile Cys Met Arg
   30 35 40
   aac ttc agt cgt agt gac cac ctt acc acc cac atc cgg acc cac acc 195
   Asn Phe Ser Arg Ser Asp His Leu Thr Thr His Ile Arg Thr His Thr
   45 50 55
   ggc gag aag cct ttt gcc tgt gac att tgt ggg agg aag ttt gcc agg 243
   Gly Glu Lys Pro Phe Ala Cys Asp Ile Cys Gly Arg Lys Phe Ala Arg
   60 65 70
   agt gat gaa cgc aag agg cat acc aaa atc cat tta aga cag aag gat 291
   Ser Asp Glu Arg Lys Arg His Thr Lys Ile His Leu Arg Gln Lys Asp
   75 80 85
   ccgcgggaat cc 303
<210> 21
   <211> 89
   <212> PRT
   <213> Artificial Sequence
<220>
   <223> plasmid sequence
<400> 21
   Glu Arg Pro Tyr Ala Cys Pro Val Glu Ser Cys Asp Arg Arg Phe Ser
   1 5 10 15
   Arg Ser Asp Glu Leu Thr Arg His Ile Arg Ile His Thr Gly Gln Lys
   20 25 30
   Pro Phe Gln Cys Arg Ile Cys Met Arg Asn Phe Ser Arg Ser Asp His
   35 40 45
   Leu Thr Thr His Ile Arg Thr His Thr Gly Glu Lys Pro Phe Ala Cys
   50 55 60
   Asp Ile Cys Gly Arg Lys Phe Ala Arg Ser Asp Glu Arg Lys Arg His
   65 70 75 80
   Thr Lys Ile His Leu Arg Gln Lys Asp
   85
<210> 22
   <211> 69
   <212> DNA
   <213> Homo sapiens
<400> 22
   tataaatgta agcaatgtgg gaaagctttt ggatgtccct caaaccttcg aaggcatgga 60
   aggactcac 69
<210> 23
<211> 23
   <212> PRT
   <213> Homo sapiens
<400> 23
   Tyr Lys Cys Lys Gln Cys Gly Lys Ala Phe Gly Cys Pro Ser Asn Leu
   1 5 10 15
   Arg Arg His Gly Arg Thr His
   20
<210> 24
   <211> 69
   <212> DNA
   <213> Homo sapiens
<400> 24
   tataagtgta aggagtgtgg gaaagccttc aaccacagct ccaacttcaa taaacaccac 60
   agaatccac 69
<210> 25
   <211> 23
   <212> PRT
   <213> Homo sapiens
<400> 25
   Tyr Lys Cys Lys Glu Cys Gly Lys Ala Phe Asn His Ser Ser Asn Phe
   1 5 10 15
   Asn Lys His His Arg Ile His
   20
<210> 26
   <211> 69
   <212> DNA
   <213> Homo sapiens
<400> 26
   tatgaatgta aggaatgtgg gaaagccttt agtagtggtt caaacttcac tcgacatcag 60
   agaattcac 69
<210> 27
   <211> 23
   <212> PRT
   <213> Homo sapiens
<400> 27
   Tyr Glu Cys Lys Glu Cys Gly Lys Ala Phe Ser Ser Gly Ser Asn Phe
   1 5 10 15
   Thr Arg His Gln Arg Ile His
   20
<210> 28
   <211> 75
   <212> DNA
   <213> Homo sapiens
<400> 28
   tatgtatgcg atgtagaggg atgtacgtgg aaatttgccc gctcagatga gctcaacaga 60
   cacaagaaaaggcac 75
<210> 29
   <211> 25
<212> PRT
   <213> Homo sapiens
<400> 29
   Tyr Val Cys Asp Val Glu Gly Cys Thr Trp Lys Phe Ala Arg Ser Asp
   1 5 10 15
   Glu Leu Asn Arg His Lys Lys Arg His
   20 25
<210> 30
   <211> 69
   <212> DNA
   <213> Homo sapiens
<400> 30
   tatgagtgta atgaatgcgg gaaagctttt gcccaaaatt caactctcag agtacaccag 60
   agaattcac 69
<210> 31
   <211> 23
   <212> PRT
   <213> Homo sapiens
<400> 31
   Tyr Glu Cys Asn Glu Cys Gly Lys Ala Phe Ala Gln Asn Ser Thr Leu
   1 5 10 15
   Arg Val His Gln Arg Ile His
   20
<210> 32
   <211> 69
   <212> DNA
   <213> Homo sapiens
<400> 32
   tatgagtgta attactgtgg aaaaaccttt agtgtgagct caacccttat tagacatcag 60
   agaatccac 69
<210> 33
   <211> 23
   <212> PRT
   <213> Homo sapiens
<400> 33
   Tyr Glu Cys Asn Tyr Cys Gly Lys Thr Phe Ser Val Ser Ser Thr Leu
   1 5 10 15
   Ile Arg His Gln Arg Ile His
   20
<210> 34
   <211> 69
   <212> DNA
   <213> Homo sapiens
<220>
   <221> CDS
   <222> (1)...(69)
<400> 34
   tat cag tgc aac att tgc gga aaa tgt ttc tcc tgc aac tcc aac ctc 48
   Tyr Gln Cys Asn Ile Cys Gly Lys Cys Phe Ser Cys Asn Ser Asn Leu
   1 5 10 15
   cac agg cac cag aga acg cac 69
   His Arg His Gln Arg Thr His
   20
<210> 35
   <211> 23
   <212> PRT
   <213> Homo sapiens
<400> 35
   Tyr Gln Cys Asn Ile Cys Gly Lys Cys Phe Ser Cys Asn Ser Asn Leu
   1 5 10 15
   His Arg His Gln Arg Thr His
   20
<210> 36
   <211> 69
   <212> DNA
   <213> Homo sapiens
<220>
   <221> CDS
   <222> (1)...(69)
<400> 36
   tat gca tgt cat cta tgt gga aaa gcc ttc act cag agt tct cac ctt 48
   Tyr Ala Cys His Leu Cys Gly Lys Ala Phe Thr Gln Ser Ser His Leu
   1 5 10 15
   aga aga cat gag aaa act cac 69
   Arg Arg His Glu Lys Thr His
   20
<210> 37
   <211> 23
   <212> PRT
   <213> Homo sapiens
<400> 37 <210> 38
   <211> 69
   <212> DNA
   <213> Homo sapiens
<220>
   <221> CDS
   <222> (1)...(69)
<400> 38
<210> 39
   <211> 23
   <212> PRT
   <213> Homo sapiens
<400> 39
   Tyr Lys Cys Gly Gln Cys Gly Lys Phe Tyr Ser Gln Val Ser His Leu
   1 5 10 15
   Thr Arg His Gln Lys Ile His
   20
<210> 40
   <211> 69
   <212> DNA
   <213> Homo sapiens
<220>
   <221> CDS
   <222> (1)...(69)
<400> 40
   tat gca tgt cat cta tgt gga aaa gcc ttc act cag tgt tct cac ctt 48
   Tyr Ala Cys His Leu Cys Gly Lys Ala Phe Thr Gln Cys Ser His Leu
   1 5 10 15
   aga aga cat gag aaa act cac 69
   Arg Arg His Glu Lys Thr His
   20
<210> 41
   <211> 23
   <212> PRT
   <213> Homo sapiens
<400> 41
   Tyr Ala Cys His Leu Cys Gly Lys Ala Phe Thr Gln Cys Ser His Leu
   1 5 10 15
   Arg Arg His Glu Lys Thr His
   20
<210> 42
   <211> 69
   <212> DNA
   <213> Homo sapiens
<220>
   <221> CDS
   <222> (1)...(69)
<400> 42
   tat gca tgt cat cta tgt gca aaa gcc ttc att cag tgt tct cac ctt 48
   Tyr Ala Cys His Leu Cys Ala Lys Ala Phe Ile Gln Cys Ser His Leu
   1 5 10 15
   aga aga cat gag aaa act cac 69
   Arg Arg His Glu Lys Thr His
   20
<210> 43
   <211> 23
   <212> PRT
   <213> Homo sapiens
<400> 43
   Tyr Ala Cys His Leu Cys Ala Lys Ala Phe Ile Gln Cys Ser His Leu
   1 5 10 15
   Arg Arg His Glu Lys Thr His
   20
<210> 44
   <211> 69
   <212> DNA
   <213> Homo sapiens
<220>
   <221> CDS
   <222> (1)...(69)
<400> 44
   tat gtt tgc agg gaa tgt ggg cgt ggc ttt cgc cag cat tca cac ctg 48
   Tyr Val Cys Arg Glu Cys Gly Arg Gly Phe Arg Gln His Ser His Leu
   1 5 10 15
   gtc aga cac aag agg aca cat 69
   Val Arg His Lys Arg Thr His
   20
<210> 45
   <211> 23
   <212> PRT
   <213> Homo sapiens
<400> 45
   Tyr Val Cys Arg Glu Cys Gly Arg Gly Phe Arg Gln His Ser His Leu
   1 5 10 15
   Val Arg His Lys Arg Thr His
   20
<210> 46
   <211> 69
   <212> DNA
   <213> Homo sapiens
<220>
   <221> CDS
   <222> (1)...(69)
<400> 46
   ttt gag tgt aaa gat tgc ggg aaa gct ttc att cag aag tca aac ctc 48
   Phe Glu Cys Lys Asp Cys Gly Lys Ala Phe Ile Gln Lys Ser Asn Leu
   1 5 10 15
   atc aga cac cag aga act cac 69
   Ile Arg His Gln Arg Thr His
   20
<210> 47
   <211> 23
   <212> PRT
   <213> Homo sapiens
<400> 47
   Phe Glu Cys Lys Asp Cys Gly Lys Ala Phe Ile Gln Lys Ser Asn Leu
   1 5 10 15
   Ile Arg His Gln Arg Thr His
   20
<210> 48
   <211> 69
   <212> DNA
   <213> Homo sapiens
<220>
   <221> CDS
   <222> (1)...(69)
<400> 48
   tat gtc tgc agg gag tgt agg cga ggt ttt agc cag aag tca aat ctc 48
   Tyr Val Cys Arg Glu Cys Arg Arg Gly Phe Ser Gln Lys Ser Asn Leu
   1 5 10 15
   atc aga cac cag agg acg cac 69
   Ile Arg His Gln Arg Thr His
   20
<210> 49
   <211> 23
   <212> PRT
   <213> Homo sapiens
<400> 49
   Tyr Val Cys Arg Glu Cys Arg Arg Gly Phe Ser Gln Lys Ser Asn Leu
   1 5 10 15
   Ile Arg His Gln Arg Thr His
   20
<210> 50
   <211> 69
   <212> DNA
   <213> Homo sapiens
<220>
   <221> CDS
   <222> (1)...(69)
<400> 50
   tat gaa tgt aac aca tgc agg aaa acc ttc tct caa aag tca aat ctc 48
   Tyr Glu Cys Asn Thr Cys Arg Lys Thr Phe Ser Gln Lys Ser Asn Leu
   1 5 10 15
   att gta cat cag aga aca cac 69
   Ile Val His Gln Arg Thr His
   20
<210> 51
   <211> 23
   <212> PRT
   <213> Homo sapiens
<400> 51
   Tyr Glu Cys Asn Thr Cys Arg Lys Thr Phe Ser Gln Lys Ser Asn Leu
   1 5 10 15
   Ile Val His Gln Arg Thr His
   20
<210> 52
   <211> 69
   <212> DNA
   <213> Homo sapiens
<220>
   <221> CDS
   <222> (1)...(69)
<400> 52
   tat gtt tgc tca aaa tgt ggg aaa gcc ttc act cag agt tca aat ctg 48
   Tyr Val Cys Ser Lys Cys Gly Lys Ala Phe Thr Gln Ser Ser Asn Leu
   1 5 10 15
   act gta cat caa aaa atc cac 69
   Thr Val His Gln Lys Ile His
   20
<210> 53
   <211> 23
   <212> PRT
   <213> Homo sapiens
<400> 53
   Tyr Val Cys Ser Lys Cys Gly Lys Ala Phe Thr Gln Ser Ser Asn Leu
   1 5 10 15
   Thr Val His Gln Lys Ile His
   20
<210> 54
   <211> 69
   <212> DNA
   <213> Homo sapiens
<220>
   <221> CDS
   <222> (1)...(69)
<400> 54
   tac aaa tgt gac gaa tgt gga aaa aac ttt acc cag tcc tcc aac ctt 48
   Tyr Lys Cys Asp Glu Cys Gly Lys Asn Phe Thr Gln Ser Ser Asn Leu
   1 5 10 15
   att gta cat aag aga att cat 69
   Ile Val His Lys Arg Ile His
   20
<210> 55
   <211> 23
   <212> PRT
   <213> Homo sapiens
<400> 55
   Tyr Lys Cys Asp Glu Cys Gly Lys Asn Phe Thr Gln Ser Ser Asn Leu
   1 5 10 15
   Ile Val His Lys Arg Ile His
   20
<210> 56
   <211> 69
   <212> DNA
   <213> Homo sapiens
<220>
   <221> CDS
   <222> (1)...(69)
<400> 56
   tat gaa tgt gat gtg tgt gga aaa acc ttc acg caa aag tca aac ctt 48
   Tyr Glu Cys Asp Val Cys Gly Lys Thr Phe Thr Gln Lys Ser Asn Leu
   1 5 10 15
   ggt gta cat cag aga act cat 69
   Gly Val His Gln Arg Thr His
   20
<210> 57
   <211> 23
   <212> PRT
   <213> Homo sapiens
<400> 57
   Tyr Glu Cys Asp Val Cys Gly Lys Thr Phe Thr Gln Lys Ser Asn Leu
   1 5 10 15
   Gly Val His Gln Arg Thr His
   20
<210> 58
   <211> 69
   <212> DNA
   <213> Homo sapiens
<220>
   <221> CDS
   <222> (1)...(69)
<400> 58
   tat aag tgc cct gat tgt ggg aag agt ttt agt cag agt tcc agc ctc 48
   Tyr Lys Cys Pro Asp Cys Gly Lys Ser Phe Ser Gln Ser Ser Ser Leu
   1 5 10 15
   att cgc cac cag cgg aca cac 69
   Ile Arg His Gln Arg Thr His
   20
<210> 59
   <211> 23
   <212> PRT
   <213> Homo sapiens
<400> 59
   Tyr Lys Cys Pro Asp Cys Gly Lys Ser Phe Ser Gln Ser Ser Ser Leu
   1 5 10 15
   Ile Arg His Gln Arg Thr His
   20
<210> 60
   <211> 69
   <212> DNA
   <213> Homo sapiens
<220>
   <221> CDS
   <222> (1)...(69)
<400> 60
   tat gag tgt cag gac tgt ggg agg gcc ttc aac cag aac tcc tcc ctg 48
   Tyr Glu Cys Gln Asp Cys Gly Arg Ala Phe Asn Gln Asn Ser Ser Leu
   1 5 10 15
   ggg cgg cac aag agg aca cac 69
   Gly Arg His Lys Arg Thr His
   20
<210> 61
   <211> 23
   <212> PRT
   <213> Homo sapiens
<400> 61
   Tyr Glu Cys Gln Asp Cys Gly Arg Ala Phe Asn Gln Asn Ser Ser Leu
   1 5 10 15
   Gly Arg His Lys Arg Thr His
   20
<210> 62
   <211> 69
   <212> DNA
   <213> Homo sapiens
<220>
   <221> CDS
   <222> (1)...(69)
<400> 62
   tac aaa tgt gaa gaa tgt ggc aaa gct ttt aac cag tcc tca acc ctt 48
   Tyr Lys Cys Glu Glu Cys Gly Lys Ala Phe Asn Gln Ser Ser Thr Leu
   1 5 10 15
   act aga cat aag ata gtt cat 69
   Thr Arg His Lys Ile Val His
   20
<210> 63
   <211> 23
   <212> PRT
   <213> Homo sapiens
<400> 63
   Tyr Lys Cys Glu Glu Cys Gly Lys Ala Phe Asn Gln Ser Ser Thr Leu
   1 5 10 15
   Thr Arg His Lys Ile Val His
   20
<210> 64
   <211> 69
   <212> DNA
   <213> Homo sapiens
<220>
   <221> CDS
   <222> (1)...(69)
<400> 64
   tat aag tgc atg gag tgt ggg aag gct ttt aac cgc agg tca cac ctc 48
   Tyr Lys Cys Met Glu Cys Gly Lys Ala Phe Asn Arg Arg Ser His Leu
   1 5 10 15
   aca cgg cac cag cgg att cac 69
   Thr Arg His Gln Arg Ile His
   20
<210> 65
   <211> 23
   <212> PRT
   <213> Homo sapiens
<400> 65
   Tyr Lys Cys Met Glu Cys Gly Lys Ala Phe Asn Arg Arg Ser His Leu
   1 5 10 15
   Thr Arg His Gln Arg Ile His
   20
<210> 66
   <211> 69
   <212> DNA
   <213> Homo sapiens
<220>
   <221> CDS
   <222> (1)...(69)
<400> 66
   tat aca tgt aaa cag tgt ggg aaa gcc ttc agt gtt tcc agt tcc ctt 48
   Tyr Thr Cys Lys Gln Cys Gly Lys Ala Phe Ser Val Ser Ser Ser Leu
   1 5 10 15
   cga aga cat gaa acc act cac 69
   Arg Arg His Glu Thr Thr His
   20
<210> 67
   <211> 23
   <212> PRT
   <213> Homo sapiens
<400> 67
   Tyr Thr Cys Lys Gln Cys Gly Lys Ala Phe Ser Val Ser Ser Ser Leu
   1 5 10 15
   Arg Arg His Glu Thr Thr His
   20
<210> 68
   <211> 28
   <212> PRT
   <213> Artificial Sequence
<220>
   <223> purified polypeptide
<221> VARIANT
   <222> 2, 4-8 ,10-12, 14, 16, 20, 23-27
   <223> Xaa = any amino acid
<221> VARIANT
   <222> 1, 13
   <223> Xaa = Phe or Tyr
<221> VARIANT
   <222> 19
   <223> Xaa = hydrophobic residue
<400> 68
   Xaa Xaa Cys Xaa Xaa Xaa Xaa Xaa Cys Xaa Xaa Xaa Xaa Xaa Cys Xaa
   1 5 10 15
   Ser Asn Xaa Xaa Arg His Xaa Xaa Xaa Xaa Xaa His
   20 25
<210> 69
   <211> 28
   <212> PRT
   <213> Artificial Sequence
<220>
   <223> purified polypeptide
<221> VARIANT
   <222> 2, 4-8 ,10-12, 14, 16, 20, 23-27
   <223> Xaa = any amino acid
<221> VARIANT
   <222> 1, 13
   <223> Xaa = Phe or Tyr
<221> VARIANT
   <222> 19
   <223> Xaa = hydrophobic residue
<400> 69
   Xaa Xaa Cys Xaa Xaa Xaa Xaa Xaa Cys Xaa Xaa Xaa Xaa Xaa His Xaa
   1               5                   10                  15
   Ser Asn Xaa Xaa Lys His Xaa Xaa Xaa Xaa Xaa His
               20                  25
<210> 70
   <211>28
   <212> PRT
   <213> Artificial Sequence
<220>
   <223> purified polypeptide
<221> VARIANT
   <222> 2, 4-8 ,10-12, 14, 16, 20, 23-27
   <223> Xaa = any amino acid
<221> VARIANT
   <222> 1, 13
   <223> Xaa = Phe or Tyr
<221> VARIANT
   <222> 19
   <223> Xaa = hydrophobic residue
<400> 70
   Xaa Xaa Cys Xaa Xaa Xaa Xaa Xaa Cys Xaa Xaa Xaa Xaa Xaa Ser Xaa
   1               5                   10                  15
   Ser Asn Xaa Xaa Arg His Xaa Xaa Xaa Xaa Xaa His
               20                  25
<210> 71
   <211> 28
   <212> PRT
   <213> Artificial Sequence
<220>
   <223> purified polypeptide
<221> VARIANT
   <222> 2, 4-8 ,10-12, 14, 16, 20, 23-27
   <223> Xaa = any amino acid
<221> VARIANT
   <222> 1, 13
   <223> Xaa = Phe or Tyr
<221> VARIANT
   <222> 19
   <223> Xaa = hydrophobic residue
<400> 71
   Xaa Xaa Cys Xaa Xaa Xaa Xaa Xaa Cys Xaa Xaa Xaa Xaa Xaa Gln Xaa
   1 5 10 15
   Ser Thr Xaa Xaa Val His Xaa Xaa Xaa Xaa Xaa His
   20 25
<210> 72
   <211> 28
   <212> PRT
   <213> Artificial Sequence
<220>
   <223> purified polypeptide
<221> VARIANT
   <222> 2, 4-8, 10-12, 14, 16, 20, 23-27
   <223> Xaa = any amino acid
<221> VARIANT
   <222> 1, 13
   <223> Xaa = Phe or Tyr
<221> VARIANT
   <222> 18
   <223> Xaa = Ser or Thr
<221> VARIANT
   <222> 19
   <223> Xaa = hydrophobic residue
<400> 72
   Xaa Xaa Cys Xaa Xaa Xaa Xaa Xaa Cys Xaa Xaa Xaa Xaa Xaa Val Xaa
   1 5 10 15
   Ser Xaa Xaa Xaa Arg His Xaa Xaa Xaa Xaa Xaa His
   20 25
<210> 73
   <211> 28
   <212> PRT
   <213> Artificial Sequence
<220>
   <223> purified polypeptide
<221> VARIANT
   <222> 2, 4-8 ,10-12, 14, 16, 20, 23-27
   <223> Xaa = any amino acid
<221> VARIANT
   <222> 1, 13
   <223> Xaa = Phe or Tyr
<221> VARIANT
   <222> 19
   <223> Xaa = hydrophobic residue
<400> 73
   Xaa Xaa Cys Xaa Xaa Xaa Xaa Xaa Cys Xaa Xaa Xaa Xaa Xaa Gln Xaa
   1 5 10 15
   Ser His Xaa Xaa Arg His Xaa Xaa Xaa Xaa Xaa His
   20 25
<210> 74
   <211> 28
   <212> PRT
   <213> Artificial Sequence
<220>
   <223> purified polypeptide
<221> VARIANT
   <222> 2, 4-8 ,10-12, 14, 16, 20, 23-27
   <223> Xaa = any amino acid
<221> VARIANT
   <222> 1, 13
   <223> Xaa = Phe or Tyr
<221> VARIANT
   <222> 19
   <223> Xaa = hydrophobic residue
<400> 74
   Xaa Xaa Cys Xaa Xaa Xaa Xaa Xaa Cys Xaa Xaa Xaa Xaa Xaa Gln Xaa
   1 5 10 15
   Ser Asn Xaa Xaa Val His Xaa Xaa Xaa Xaa Xaa His
   20 25
<210> 75
   <211> 28
   <212> PRT
   <213> Artificial Sequence
<220>
   <223> purified polypeptide
<221> VARIANT
   <222> 2, 4-8 ,10-12, 14, 16, 20, 23-27
   <223> Xaa = any amino acid
<221> VARIANT
   <222> 1, 13
   <223> Xaa = Phe or Tyr
<221> VARIANT
   <222> 18
   <223> Xaa = Ser or Thr
<221> VARIANT
   <222> 19
   <223> Xaa = hydrophobic residue
<400> 75
   Xaa Xaa Cys Xaa Xaa Xaa Xaa Xaa Cys Xaa Xaa Xaa Xaa Xaa Gln Xaa
   1 5 10 15
   Ser Xaa Xaa Xaa Arg His Xaa Xaa Xaa Xaa Xaa His
   20 25
<210> 76
   <211> 28
   <212> PRT
   <213> Artificial Sequence
<220>
   <223> coordinating residue
<221> VARIANT
   <222> 1, 13
   <223> Xaa = Phe or Tyr
<221> VARIANT
   <222> 2, 4-8 ,10-12, 14, 16, 20, 23-27
   <223> Xaa = any amino acid
<221> VARIANT
   <222> 19
   <223> Xaa = hydrophobic residue
<400> 76
   Xaa Xaa Cys Xaa Xaa Xaa Xaa Xaa Cys Xaa Xaa Xaa Xaa Xaa Xaa Xaa
   1 5 10 15
   Xaa Xaa Xaa Xaa Xaa His Xaa Xaa Xaa Xaa Xaa His
   20 25
<210> 77
   <211> 24
   <212> PRT
   <213> Artificial Sequence
<220>
   <223> polypeptide motif
<221 > VARIANT
   <222> 1
   <223> Xaa = Leu, Ile, Val, Met, Phe, Tyr, or Gly
<221> VARIANT
   <222> 2
   <223> Xaa = Ala, Ser, Leu, Val, or Arg
<221> VARIANT
   <222> 3-4, 6, 8-11, 17, 19-23
   <223> Xaa = any amino acid
<221> VARIANT
   <222> 5
   <223> Xaa = Leu, Ile, Val, Met, Ser, Thr, Ala, Cys, or Asn
<221> VARIANT
   <222> 7
   <223> Xaa = Leu, Ile, Val, or Met
<221> VARIANT
   <222> 12
   <223> Xaa = Leu, Ile, or Val
<221> VARIANT
   <222> 13
   <223> Xaa = Arg, Lys, Asn, Gln, Glu, Ser, Thr, Ala, Ile, or Tyr
<221> VARIANT
   <222> 14
   <223> Xaa = Leu, Ile, Val, Phe, Ser, Thr, Asn, Lys, or His
<221> VARIANT
   <222> 16
   <223> Xaa = Phe, Tyr, Val, or Cys
<221> VARIANT
   <222> 18
   <223> Xaa = Asn, Asp, Gln, Thr, Ala, or His
<221> VARIANT
   <222> 24
   <223> Xaa = Arg, Lys, Asn, Ala, Ile, Met, or Trp
<400> 77
   Xaa Xaa Xaa Xaa Xaa Xaa Xaa Xaa Xaa Xaa Xaa Xaa Xaa Xaa Trp Xaa
   1 5 10 15
   Xaa Xaa Xaa Xaa Xaa Xaa Xaa Xaa
   20
<210> 78
   <211> 6
   <212> PRT
   <213> Eukaryote
<220>
   <221> VARIANT
   <222> 3
   <223> Xaa = Glu or Gln
<221> VARIANT
   <222> 4
   <223> Xaa = Lys or Arg
<221> VARIANT
   <222> 6
   <223> Xaa = Tyr or Phe
<400> 78
   Thr Gly Xaa Xaa Pro Xaa
   1 5
<210> 79
   <211> 29
   <212> DNA
   <213> Artificial Sequence
<220>
   <223> synthetic oligonucleotide
<400> 79
   tgcctgcagc atttgtggga ggaagtttg 29
<210> 80
   <211> 30
   <212> DNA
   <213> Artificial Sequence
<220>
   <223> synthetic oligonucleotide
<400> 80
   atgctgcagg cttaaggctt ctcgccggtg 30
<210> 81
   <211> 24
   <212> DNA
   <213> Artificial Sequence
<220>
   <223> primer for PCR
<221> misc_feature
   <222> 11, 17, 20
   <223> n = A, T, G, or C;
<400> 81
   gcgtccggac ncayacnggn sara 24
<210> 82
   <211> 24
   <212> DNA
   <213> Artificial Sequence
<220>
   <223> primer for PCR
<221> misc_feature
   <222> 10-11, 16,
   <223> n = A, T, G, or C;
<400> 82
   cggaattcan nbrwanggyy tytc 24
<210> 83
   <211> 7
   <212> PRT
   <213> Artificial Sequence
<220>
   <223> amino acid motif
<221> VARIANT
   <222> 4
   <223> Xaa = Glu or Gln
<221> VARIANT
   <222> 5
   <223> Xaa = Lys or Arg

<221> VARIANT
   <222> 3
   <223> Xaa = Tyr or Phe
<400> 83
   His Thr Gly Xaa Xaa Pro Xaa
   1 5
<210> 84
   <211> 54
   <212> DNA
   <213> Artificial Sequence
<220>
   <223> synthetic oligonucleotide
<400> 84
   gggcccgggg agaagcctta cgcatgtcca gtcgaatctt gtgatagaag attc 54
<210> 85
   <211> 75
   <212> DNA
   <213> Artificial Sequence
<220>
   <223> synthetic oligonucleotide
<221> misc_feature
   <222> 36, 39, 45, 51, 54,
   <223> n = A, T, G, or C;
<400> 85
   ctccccgcgg ttcgccggtg tggattctga tatgsnbsnb aagsnbsnbs nbsnbtgaga 60
   atcttctatc acaag 75
<210> 86
   <211> 23
   <212> DNA
   <213> Artificial Sequence
<220>
   <223> synthetic oligonucleotide
<400> 86
   ctagacccgg gaattcgtcg acg 23
<210> 87
   <211> 23
   <212> DNA
   <213> Artificial Sequence
<220>
   <223> synthetic oligonucleotide
<400> 87
   gatccgtcga cgaattcccg ggt 23
<210> 88
   <211> 38
   <212> DNA
   <213> Artificial Sequence
<220>
   <223> synthetic oligonucleotide
<221> misc_feature
   <222> 6-8, 18-20, 30-32
   <223> n = A, T, G, or C
<400> 88
   ccggtnnntg ggcgtacnnn tgggcgtcan nntgggcg 38
<210> 89
   <211> 38
   <212> DNA
   <213> Artificial Sequence
<220>
   <223> synthetic oligonucleotide
<221> misc_feature
   <222> 11-13, 23-25, 35-37
   <223> n = A, T, G, or C
<400> 89
   tcgacgccca nnntgacgcc cannngtacg cccannna 38
<210> 90
   <211> 24
   <212> DNA
   <213> Artificial Sequence
<220>
   <223> synthetic probe for gel shift assay
<400> 90
   ccgggtcgcg cgtgggcggt accg 24
<210> 91
   <211> 24
   <212> DNA
   <213> Artificial Sequence
<220>
   <223> synthetic probe for gel shift assay
<400> 91
   tcgacggtac cgcccacgcg cgac 24
<210> 92
   <211> 24
   <212> DNA
   <213> Artificial Sequence
<220>
   <223> synthetic probe for gel shift assay
<400> 92
   ccgggtcgcg agcgggcggt accg 24
<210> 93
   <211> 24
   <212> DNA
   <213> Artificial Sequence
<220>
   <223> synthetic probe for gel shift assay
<400> 93
   tcgacggtac cgcccgctcg cgac 24
<210> 94
   <211> 24
   <212> DNA
   <213> Artificial Sequence
<220>
   <223> synthetic probe for gel shift assay
<400> 94
   ccgggtcgtg cttgggcggt accg 24
<210> 95
   <211> 24
   <212> DNA
   <213> Artificial Sequence
<220>
   <223> synthetic probe for gel shift assay
<400> 95
   tcgacggtac cgcccaagca cgac 24
<210> 96
   <211> 24
   <212> DNA
   <213> Artificial Sequence
<220>
   <223> synthetic probe for gel shift assay
<400> 96
   ccgggtcggg actgggcggt accg 24
<210> 97
   <211> 24
   <212> DNA
   <213> Artificial Sequence
<220>
   <223> synthetic probe for gel shift assay
<400> 97
   tcgacggtac cgcccagtcc cgac 24
<210> 98
   <211> 24
   <212> DNA
   <213> Artificial Sequence
<220>
   <223> synthetic probe for gel shift assay
<400> 98
   ccgggtcggg agtgggcggt accg 24
<210> 99
   <211> 24
   <212> DNA
   <213> Artificial Sequence
<220>
   <223> synthetic probe for gel shift assay
<400> 99
   tcgacggtac cgcccactcc cgac 24
<210> 100
   <211> 24
   <212> DNA
   <213> Artificial Sequence
<220>
   <223> synthetic probe for gel shift assay
<400> 100
   ccgggtcgga catgggcggt accg 24
<210> 101
   <211> 24
   <212> DNA
   <213> Artificial Sequence
<220>
   <223> synthetic probe for gel shift assay
<400> 101
   tcgacggtac cgcccatgtc cgac 24
<210> 102
   <211> 69
   <212> DNA
   <213> Homo sapiens
<220>
   <221> CDS
   <222> (1)...(69)
<400> 102
   tat aag tgt aag gaa tgt ggg cag gcc ttt aga cag cgt gca cat ctt 48
   Tyr Lys Cys Lys Glu Cys Gly Gln Ala Phe Arg Gln Arg Ala His Leu
   1 5 10 15
   att cga cat cac aaa ctt cac 69
   Ile Arg His His Lys Leu His
   20
<210> 103
   <211> 23
   <212> PRT
   <213> Homo sapiens
<400> 103
   Tyr Lys Cys Lys Glu Cys Gly Gln Ala Phe Arg Gln Arg Ala His Leu
   1 5 10 15
   Ile Arg His His Lys Leu His
   20
<210> 104
   <211> 69
   <212> DNA
   <213> Homo sapiens
<220>
   <221> CDS
   <222> (1)...(69)
<400> 104
   tat aag tgt cat caa tgt ggg aaa gcc ttt att caa tcc ttt aac ctt 48
   Tyr Lys Cys His Gln Cys Gly Lys Ala Phe Ile Gln Ser Phe Asn Leu
   1 5 10 15
   cga aga cat gag aga act cac 69
   Arg Arg His Glu Arg Thr His
   20
<210> 105
   <211> 23
   <212> PRT
   <213> Homo sapiens
<400> 105
   Tyr Lys Cys His Gln Cys Gly Lys Ala Phe Ile Gln Ser Phe Asn Leu
   1 5 10 15
   Arg Arg His Glu Arg Thr His
   20
<210> 106
   <211> 69
   <212> DNA
   <213> Homo sapiens
<220>
   <221> CDS
   <222> (1)...(69)
<400> 106
   ttc cag tgt aat cag tgt ggg gca tct ttt act cag aaa ggt aac ctc 48
   Phe Gln Cys Asn Gln Cys Gly Ala Ser Phe Thr Gln Lys Gly Asn Leu
   1 5 10 15
   ctc cgc cac att aaa ctg cac 69
   Leu Arg His Ile Lys Leu His
   20
<210> 107
   <211> 23
   <212> PRT
   <213> Homo sapiens
<400> 107
   Phe Gln Cys Asn Gln Cys Gly Ala Ser Phe Thr Gln Lys Gly Asn Leu
   1 5 10 15
   Leu Arg His Ile Lys Leu His
   20
<210> 108
   <211> 72
   <212> DNA
   <213> Artificial Sequence
<220>
   <223> primer for PCR
<221> misc_feature
   <222> 22-72
   <223> n =A, T, G, or C
<400> 108
   acccacactg gccagaaacc cnnnnnnnnn nnnnnnnnnn nnnnnnnnnn nnnnnnnnnn 60
   nnnnnnnnnn nn 72
<210> 109
   <211> 66
   <212> DNA
   <213> Artificial Sequence
<220>
   <223> primer for PCR
<221> misc_feature
   <222> 22-66
   <223> n = A, T, G, or C
<400> 109
   gatctgaatt cattcaccgg tnnnnnnnnn nnnnnnnnnn nnnnnnnnnn nnnnnnnnnn 60
   nnnnnn 66
<210> 110
   <211> 69
   <212> DNA
   <213> Homo sapiens
<220>
   <221> CDS
   <222> (1)...(69)
<400> 110
   tac aaa tgt gaa gaa tgt ggc aaa gcc ttt agg cag tcc tca cac ctt 48
   Tyr Lys Cys Glu Glu Cys Gly Lys Ala Phe Arg Gln Ser Ser His Leu
   1 5 10 15
   act aca cat aag ata att cat 69
   Thr Thr His Lys Ile Ile His
   20
<210> 111
   <211> 23
   <212> PRT
   <213> Homo sapiens
<400> 111
   Tyr Lys Cys Glu Glu Cys Gly Lys Ala Phe Arg Gln Ser Ser His Leu
   1 5 10 15
   Thr Thr His Lys Ile Ile His
   20
<210> 112
   <211> 69
   <212> DNA
   <213> Homo sapiens
<220>
   <221> CDS
   <222> (1)...(69)
<400> 112
   tat gag tgt gat cac tgt gga aaa tcc ttt agc cag agc tct cat ctg 48
   Tyr Glu Cys Asp His Cys Gly Lys Ser Phe Ser Gln Ser Ser His Leu
   1 5 10 15
   aat gtg cac aaa aga act cac 69
   Asn Val His Lys Arg Thr His
   20
<210> 113
   <211> 23
   <212> PRT
   <213> Homo sapiens
<400> 113
   Tyr Glu Cys Asp His Cys Gly Lys Ser Phe Ser Gln Ser Ser His Leu
   1 5 10 15
   Asn Val His Lys Arg Thr His
   20
<210> 114
   <211> 69
   <212> DNA
   <213> Homo sapiens
<220>
   <221> CDS
   <222> (1)...(69)
<400> 114
   tac atg tgc agt gag tgt ggg cga ggc ttc agc cag aag tca aac ctc 48
   Tyr Met Cys Ser Glu Cys Gly Arg Gly Phe Ser Gln Lys Ser Asn Leu
   1 5 10 15
   atc ata cac cag agg aca cac 69
   Ile Ile His Gln Arg Thr His
   20
<210> 115
   <211> 23
   <212> PRT
   <213> Homo sapiens
<400> 115
   Tyr Met Cys Ser Glu Cys Gly Arg Gly Phe Ser Gln Lys Ser Asn Leu
   1 5 10 15
   Ile Ile His Gln Arg Thr His
   20
<210> 116
   <211> 69
   <212> DNA
   <213> Homo sapiens
<220>
   <221> CDS
   <222> (1)...(69)
<400> 116
   tat gaa tgt gaa aaa tgt ggc aaa gct ttt aac cag tcc tca aat ctt 48
   Tyr Glu Cys Glu Lys Cys Gly Lys Ala Phe Asn Gln Ser Ser Asn Leu
   1 5 10 15
   act aga cat aag aaa agt cat 69
   Thr Arg His Lys Lys Ser His
   20
<210> 117
   <211> 23
   <212> PRT
   <213> Homo sapiens
<400> 117
   Tyr Glu Cys Glu Lys Cys Gly Lys Ala Phe Asn Gln Ser Ser Asn Leu
   1 5 10 15
   Thr Arg His Lys Lys Ser His
   20
<210> 118
   <211> 69
   <212> DNA
   <213> Homo sapiens
<220>
   <221> CDS
   <222> (1)...(69)
<400> 118
   tat gag tgc aat gaa tgt ggg aag ttt ttt agc cag agc tcc agc ctc 48
   Tyr Glu Cys Asn Glu Cys Gly Lys Phe Phe Ser Gln Ser Ser Ser Leu
   1 5 10 15
   att aga cat agg aga agt cac 69
   Ile Arg His Arg Arg Ser His
   20
<210> 119
   <211> 23
   <212> PRT
   <213> Homo sapiens
<400> 119
   Tyr Glu Cys Asn Glu Cys Gly Lys Phe Phe Ser Gln Ser Ser Ser Leu
   1 5 10 15
   Ile Arg His Arg Arg Ser His
   20
<210> 120
   <211> 69
   <212> DNA
   <213> Homo sapiens
<220>
   <221> CDS
   <222> (1)...(69)
<400> 120
   tat gag tgt cac gat tgc gga aag tcc ttt agg cag agc acc cac ctc 48
   Tyr Glu Cys His Asp Cys Gly Lys Ser Phe Arg Gln Ser Thr His Leu
   1 5 10 15
   act cag cac cgg agg atc cac 69
   Thr Gln His Arg Arg Ile His
   20
<210> 121
   <211> 23
   <212> PRT
   <213> Homo sapiens
<400> 121
   Tyr Glu Cys His Asp Cys Gly Lys Ser Phe Arg Gln Ser Thr His Leu
   1 5 10 15
   Thr Gln His Arg Arg Ile His
   20
<210> 122
   <211> 69
   <212> DNA
   <213> Homo sapiens
<220>
   <221> CDS
   <222> (1)...(69)
<400> 122
   tat gag tgt cac gat tgc gga aag tcc ttt agg cag agc acc cac ctc 48
   Tyr Glu Cys His Asp Cys Gly Lys Ser Phe Arg Gln Ser Thr His Leu
   1 5 10 15
   act cgg cac cgg agg atc cac 69
   Thr Arg His Arg Arg Ile His
   20
<210> 123
   <211> 23
   <212> PRT
   <213> Homo sapiens
<400> 123
   Tyr Glu Cys His Asp Cys Gly Lys Ser Phe Arg Gln Ser Thr His Leu
   1 5 10 15
   Thr Arg His Arg Arg Ile His
   20
<210> 124
   <211> 69
   <212> DNA
   <213> Homo sapiens
<220>
   <221> CDS
   <222> (1)...(69)
<400> 124
   cac aag tgc ctt gaa tgt ggg aaa tgc ttc agt cag aac acc cat ctg 48
   His Lys Cys Leu Glu Cys Gly Lys Cys Phe Ser Gln Asn Thr His Leu
   1 5 10 15
   act cgc cac caa cgc acc cac 69
   Thr Arg His Gln Arg Thr His
   20
<210> 125
   <211> 23
   <212> PRT
   <213> Homo sapiens
<400> 125
   His Lys Cys Leu Glu Cys Gly Lys Cys Phe Ser Gln Asn Thr His Leu
   1 5 10 15
   Thr Arg His Gln Arg Thr His
   20
<210> 126
   <211> 75
   <212> DNA
   <213> Homo sapiens
<220>
   <221> CDS
   <222> (1)...(75)
<400> 126
   tac cac tgt gac tgg gac ggc tgt gga tgg aaa ttc gcc cgc tca gat 48
   Tyr His Cys Asp Trp Asp Gly Cys Gly Trp Lys Phe Ala Arg Ser Asp
   1 5 10 15
   gaa ctg acc agg cac tac cgt aaa cac 75
   Glu Leu Thr Arg His Tyr Arg Lys His
   20 25
<210> 127
   <211> 25
   <212> PRT
   <213> Homo sapiens
<400> 127
   Tyr His Cys Asp Trp Asp Gly Cys Gly Trp Lys Phe Ala Arg Ser Asp
   1 5 10 15
   Glu Leu Thr Arg His Tyr Arg Lys His
   20 25
<210> 128
   <211> 75
   <212> DNA
   <213> Homo sapiens
<220>
   <221> CDS
   <222> (1)...(75)
<400> 128
   tac aga tgc tca tgg gaa ggg tgt gag tgg cgt ttt gca aga agt gat 48
   Tyr Arg Cys Ser Trp Glu Gly Cys Glu Trp Arg Phe Ala Arg Ser Asp
   1 5 10 15
   gag tta acc agg cac ttc cga aag cac 75
   Glu Leu Thr Arg His Phe Arg Lys His
   20 25
<210> 129
   <211> 25
   <212> PRT
   <213> Homo sapiens
<400> 129
   Tyr Arg Cys Ser Trp Glu Gly Cys Glu Trp Arg Phe Ala Arg Ser Asp
   1 5 10 15
   Glu Leu Thr Arg His Phe Arg Lys His
   20 25
<210> 130
   <211> 75
   <212> DNA
   <213> Homo sapiens
<220>
   <221> CDS
   <222> (1)...(75)
<400> 130
   ttc agc tgt agc tgg aaa ggt tgt gaa agg agg ttt gcc cgt tct gat 48
   Phe Ser Cys Ser Trp Lys Gly Cys Glu Arg Arg Phe Ala Arg Ser Asp
   1 5 10 15
   gaa ctg tcc aga cac agg cga acc cac 75
   Glu Leu Ser Arg His Arg Arg Thr His
   20 25
<210> 131
   <211> 25
   <212> PRT
   <213> Homo sapiens
<400> 131
   Phe Ser Cys Ser Trp Lys Gly Cys Glu Arg Arg Phe Ala Arg Ser Asp
   1 5 10 15
   Glu Leu Ser Arg His Arg Arg Thr His
   20 25
<210> 132
   <211> 75
   <212> DNA
   <213> Homo sapiens
<220>
   <221> CDS
   <222> (1)...(75)
<400> 132
   ttc gcc tgc agc tgg cag gac tgc aac aag aag ttc gcg cgc tcc gac 48
   Phe Ala Cys Ser Trp Gln Asp Cys Asn Lys Lys Phe Ala Arg Ser Asp
   1 5 10 15
   gag ctg gcg cgg cac tac cgc aca cac 75
   Glu Leu Ala Arg His Tyr Arg Thr His
   20 25
<210> 133
   <211> 25
   <212> PRT
   <213> Homo sapiens
<400> 133 <210> 134
   <211> 75
   <212> DNA
   <213> Homo sapiens
<220>
   <221> CDS
   <222> (1)...(75)
<400> 134
<210> 135
   <211> 25
   <212> PRT
   <213> Homo sapiens
<400> 135
   Tyr His Cys Asn Trp Asp Gly Cys Gly Trp Lys Phe Ala Arg Ser Asp
   1 5 10 15
   Glu Leu Thr Arg His Tyr Arg Lys His
   20 25
<210> 136
   <211> 72
   <212> DNA
   <213> Homo sapiens
<220>
   <221> CDS
   <222> (1)...(72)
<400> 136
   ttc ctc tgt cag tat tgt gca cag aga ttt ggg cga aag gat cac ctg 48
   Phe Leu Cys Gln Tyr Cys Ala Gln Arg Phe Gly Arg Lys Asp His Leu
   1 5 10 15
   act cga cat atg aag aag agt cac 72
   Thr Arg His Met Lys Lys Ser His
   20
<210> 137
   <211> 24
   <212> PRT
   <213> Homo sapiens
<400> 137
   Phe Leu Cys Gln Tyr Cys Ala Gln Arg Phe Gly Arg Lys Asp His Leu
   1 5 10 15
   Thr Arg His Met Lys Lys Ser His
   20
<210> 138
   <211> 78
   <212> DNA
   <213> Artificial Sequence
<220>
   <223> primer for PCR
<400> 138
   tgtcgaatct gcatgcgtaa cttcagtcgt agtgaccacc ttaccaccca catccggacc 60
   cacactggcc agaaaccc 78
<210> 139
   <211> 81
   <212> DNA
   <213> Artificial Sequence
<220>
   <223> primer for PCR
<400> 139
   ggtggcggcc gttacttact tagagctcga cgtcttactt acttagcggc cgcactagta 60
   gatctgaatt cattcaccgg t 81
<210> 140
   <211> 69
   <212> DNA
   <213> Homo sapiens
<220>
   <221> CDS
   <222> (1)...(69)
<400> 140
   ttc cag tgt aaa act tgt cag cga aag ttc tcc cgg tcc gac cac ctg 48
   Phe Gln Cys Lys Thr Cys Gln Arg Lys Phe Ser Arg Ser Asp His Leu
   1 5 10 15
   aag acc cac acc agg act cat 69
   Lys Thr His Thr Arg Thr His
   20
<210> 141
   <211> 23
   <212> PRT
   <213> Homo sapiens
<400> 141
   Phe Gln Cys Lys Thr Cys Gln Arg Lys Phe Ser Arg Ser Asp His Leu
   1 5 10 15
   Lys Thr His Thr Arg Thr His
   20
<210> 142
   <211> 69
   <212> DNA
   <213> Homo sapiens
<220>
   <221> CDS
   <222> (1)...(69)
<400> 142
   ttt gcc tgc gag gtc tgc ggt gtt cga ttc acc agg aac gac aag ctg 48
   Phe Ala Cys Glu Val Cys Gly Val Arg Phe Thr Arg Asn Asp Lys Leu
   1 5 10 15
   aag atc cac atg cgg aag cac 69
   Lys Ile His Met Arg Lys His
   20
<210> 143
   <211> 23
   <212> PRT
   <213> Homo sapiens
<400> 143
   Phe Ala Cys Glu Val Cys Gly Val Arg Phe Thr Arg Asn Asp Lys Leu
   1 5 10 15
   Lys Ile His Met Arg Lys His
   20
<210> 144
   <211> 75
   <212> DNA
   <213> Homo sapiens
<220>
   <221> CDS
   <222> (1)...(75)
<400> 144
   tat gta tgc gat gta gag gga tgt acg tgg aaa ttt gcc cgc tca gat 48
   Tyr Val Cys Asp Val Glu Gly Cys Thr Trp Lys Phe Ala Arg Ser Asp
   1 5 10 15
   aag ctc aac aga cac aag aaa agg cac 75
   Lys Leu Asn Arg His Lys Lys Arg His
   20 25
<210> 145
   <211> 25
   <212> PRT
   <213> Homo sapiens
<400> 145
   Tyr Val Cys Asp Val Glu Gly Cys Thr Trp Lys Phe Ala Arg Ser Asp
   1 5 10 15
   Lys Leu Asn Arg His Lys Lys Arg His
   20 25
<210> 146
   <211> 69
   <212> DNA
   <213> Homo sapiens
<220>
   <221> CDS
   <222> (1)...(69)
<400> 146 <210> 147
   <211> 23
   <212> PRT
   <213> Homo sapiens
<400> 147
<210> 148
   <211> 69
   <212> DNA
   <213> Homo sapiens
<220>
   <221> CDS
   <222> (1)...(69)
<400> 148
   tat cta tgt agt gag tgt gac aaa tgc ttc agt aga agt aca aac ctc 48
   Tyr Leu Cys Ser Glu Cys Asp Lys Cys Phe Ser Arg Ser Thr Asn Leu
   1 5 10 15
   ata agg cat cga aga act cac 69
   Ile Arg His Arg Arg Thr His
   20
<210> 149
   <211> 23
   <212> PRT
   <213> Homo sapiens
<400> 149
   Tyr Leu Cys Ser Glu Cys Asp Lys Cys Phe Ser Arg Ser Thr Asn Leu
   1 5 10 15
   Ile Arg His Arg Arg Thr His
   20
<210> 150
   <211> 28
   <212> PRT
   <213> Artificial Sequence
<220>
   <223> purified polypeptide
<221 > VARIANT
   <222> 1, 13
   <223> Xaa = Phe or Tyr
<221 > VARIANT
   <222> 2, 4-8 ,10-12, 14, 16, 20, 23-27
   <223> Xaa = any amino acid
<221> VARIANT
   <222> 19
   <223> Xaa = hydrophobic residue
<400> 150
   Xaa Xaa Cys Xaa Xaa Xaa Xaa Xaa Cys Xaa Xaa Xaa Xaa Xaa Gln Xaa
   1 5 10 15
   Ala His Xaa Xaa Arg His Xaa Xaa Xaa Xaa Xaa His
   20 25
<210> 151
   <211> 28
   <212> PRT
   <213> Artificial Sequence
<220>
   <223> purified polypeptide
<221> VARIANT
   <222> 1, 13
   <223> Xaa = Phe or Tyr
<221> VARIANT
   <222> 2,4-8 ,10-12, 14, 16, 20, 23-27
   <223> Xaa = any amino acid
<221> VARIANT
   <222> 19
   <223> Xaa = hydrophobic residue
<400> 151
   Xaa Xaa Cys Xaa Xaa Xaa Xaa Xaa Cys Xaa Xaa Xaa Xaa Xaa Gln Xaa
   1 5 10 15
   Phe Asn Xaa Xaa Arg His Xaa Xaa Xaa Xaa Xaa His
   20 25
<210> 152
   <211> 28
   <212> PRT
   <213> Artificial Sequence
<220>
   <223> purified polypeptide
<221> VARIANT
   <222> 1, 13
   <223> Xaa = Phe or Tyr
<221> VARIANT
   <222> 2, 4-8 ,10-12, 14, 16, 20, 23-27
   <223> Xaa = any amino acid
<221 > VARIANT
   <222> 19
   <223> Xaa = hydrophobic residue
<400> 152
   Xaa Xaa Cys Xaa Xaa Xaa Xaa Xaa Cys Xaa Xaa Xaa Xaa Xaa Gln Xaa
   1 5 10 15
   Ser His Xaa Xaa Thr His Xaa Xaa Xaa Xaa Xaa His
   20 25
<210> 153
   <211> 28
   <212> PRT
   <213> Artificial Sequence
<220>
   <223> purified polypeptide
<221> VARIANT
   <222> 1, 13
   <223> Xaa = Phe or Tyr
<221> VARIANT
   <222> 2, 4-8 ,10-12, 14, 16, 20, 23-27
   <223> Xaa = any amino acid
<221> VARIANT
   <222> 19
   <223> Xaa = hydrophobic residue
<400> 153
   Xaa Xaa Cys Xaa Xaa Xaa Xaa Xaa Cys Xaa Xaa Xaa Xaa Xaa Gln Xaa
   1               5                   10                  15
   Ser His Xaa Xaa Val His Xaa Xaa Xaa Xaa Xaa His
               20                  25
<210> 154
   <211> 28
   <212> PRT
   <213> Artificial Sequence
<220>
   <223> purified polypeptide
<221 > VARIANT
   <222> 1, 13
   <223> Xaa = Phe or Tyr
<221> VARIANT
   <222> 2, 4-8 ,10-12, 14, 16, 20, 23-27
   <223> Xaa = any amino acid
<221> VARIANT
   <222> 19
   <223> Xaa = hydrophobic residue
<400> 154
   Xaa Xaa Cys Xaa Xaa Xaa Xaa Xaa Cys Xaa Xaa Xaa Xaa Xaa Gln Xaa
   1               5                   10                  15
   Ser Asn Xaa Xaa Ile His Xaa Xaa Xaa Xaa Xaa His
               20                  25
<210> 155
   <211> 28
   <212> PRT
   <213> Artificial Sequence
<220>
   <223> purified polypeptide
<221> VARIANT
   <222> 1, 13
   <223> Xaa = Phe or Tyr
<221> VARIANT
   <222> 2,4-8 ,10-12, 14, 16, 20, 23-27
   <223> Xaa = any amino acid
<221> VARIANT
   <222> 19
   <223> Xaa = hydrophobic residue
<400> 155
   Xaa Xaa Cys Xaa Xaa Xaa Xaa Xaa Cys Xaa Xaa Xaa Xaa Xaa Gln Xaa
    1               5                  10                  15
   Ser Asn Xaa Xaa Arg His Xaa Xaa Xaa Xaa Xaa His
               20                  25
<210> 156
   <211> 28
   <212> PRT
   <213> Artificial Sequence
<220>
   <223> purified polypeptide
<221> VARIANT
   <222> 1, 13
   <223> Xaa = Phe or Tyr
<221> VARIANT
   <222> 2, 4-8 ,10-12, 14, 16, 20, 23-27
   <223> Xaa = any amino acid
<221> VARIANT
   <222> 19
   <223> Xaa = hydrophobic residue
<400> 156
   Xaa Xaa Cys Xaa Xaa Xaa Xaa Xaa Cys Xaa Xaa Xaa Xaa Xaa Gln Xaa
    1               5                  10                  15
   Thr His Xaa Xaa Gln His Xaa Xaa Xaa Xaa Xaa His
               20                  25
<210> 157
   <211> 26
   <212> PRT
   <213> Artificial Sequence
<220>
   <223> purified polypeptide
<221> VARIANT
   <222> 2-6, 12, 14, 18, 21-26
   <223> Xaa = any amino acid
<221 > VARIANT
   <222> 11
   <223> Xaa = Phe or Tyr
<221> VARIANT
   <222> 17
   <223> Xaa = hydrophobic residue
<400> 157
   Cys Xaa Xaa Xaa Xaa Xaa Cys Xaa Xaa Xaa Xaa Xaa Gln Xaa Thr His
   1 5 10 15
   Xaa Xaa Arg His Xaa Xaa Xaa Xaa Xaa His
   20 25
<210> 158
   <211> 28
   <212> PRT
   <213> Artificial Sequence
<220>
   <223> purified polypeptide
<221> VARIANT
   <222> 1, 13
   <223> Xaa = Phe or Tyr
<221> VARIANT
   <222> 2, 4-8 ,10-12, 14, 16, 20, 23-27
   <223> Xaa = any amino acid
<221> VARIANT
   <222> 19
   <223> Xaa = hydrophobic residue
<400> 158
   Xaa Xaa Cys Xaa Xaa Xaa Xaa Xaa Cys Xaa Xaa Xaa Xaa Xaa Arg Xaa
   1 5 10 15
   Asp Lys Xaa Xaa Ile His Xaa Xaa Xaa Xaa Xaa His
   20 25
<210> 159
   <211> 28
   <212> PRT
   <213> Artificial Sequence
<220>
   <223> purified polypeptide
<221> VARIANT
   <222> 1, 13
   <223> Xaa = Phe or Tyr
<221> VARIANT
   <222> 2, 4-8 ,10-12, 14, 16, 20, 23-27
   <223> Xaa = any amino acid
<221> VARIANT
   <222> 19
   <223> Xaa = hydrophobic residue
<400> 159
   Xaa Xaa Cys Xaa Xaa Xaa Xaa Xaa Cys Xaa Xaa Xaa Xaa Xaa Arg Xaa
   1 5 10 15
   Ser Asn Xaa Xaa Arg His Xaa Xaa Xaa Xaa Xaa His
   20 25
<210> 160
   <211> 28
   <212> PRT
   <213> Artificial Sequence
<220>
   <223> purified polypeptide
<221> VARIANT
   <222> 1, 13
   <223> Xaa = Phe or Tyr
<221> VARIANT
   <222> 2,4-8 ,10-12, 14, 16, 20, 23-27
   <223> Xaa = any amino acid
<221> VARIANT
   <222> 19
   <223> Xaa = hydrophobic residue
<400> 160
   Xaa Xaa Cys Xaa Xaa Xaa Xaa Xaa Cys Xaa Xaa Xaa Xaa Xaa Arg Xaa
   1 5 10 15
   Thr Asn Xaa Xaa Arg His Xaa Xaa Xaa Xaa Xaa His
   20 25
<210> 161
   <211> 28
   <212> PRT
   <213> Artificial Sequence
<220>
   <223> purified polypeptide
<221 > VARIANT
   <222> 1, 13
   <223> Xaa = Phe or Tyr
<221> VARIANT
   <222> 2, 4-8 ,10-12, 14, 16, 20, 23-27
   <223> Xaa = any amino acid
<221> VARIANT
   <222> 19
   <223> Xaa = hydrophobic residue
<400> 161
   Xaa Xaa Cys Xaa Xaa Xaa Xaa Xaa Cys Xaa Xaa Xaa Xaa Xaa Gln Xaa
   1 5 10 15
   Gly Asn Xaa Xaa Arg His Xaa Xaa Xaa Xaa Xaa His
   20 25
<210> 162
   <211> 28
   <212> PRT
   <213> Artificial Sequence
<220>
   <223> purified polypeptide
<221> VARIANT
   <222> 1, 13
   <223> Xaa = Phe or Tyr
<221> VARIANT
   <222> 2, 4-8 ,10-12, 14, 16, 20, 23-27
   <223> Xaa = any amino acid
<221> VARIANT
   <222> 19
   <223> Xaa = hydrophobic residue

<400> 162
   Xaa Xaa Cys Xaa Xaa Xaa Xaa Xaa Cys Xaa Xaa Xaa Xaa Xaa Arg Xaa
   1 5 10 15
   Asp Glu Xaa Xaa Arg His Xaa Xaa Xaa Xaa Xaa His
   20 25
<210> 163
   <211> 28
   <212> PRT
   <213> Artificial Sequence
<220>
   <223> purified polypeptide
<221> VARIANT
   <222> 1, 13
   <223> Xaa = Phe or Tyr
<221> VARIANT
   <222> 2, 4-8 ,10-12, 14, 16,20, 23-27
   <223> Xaa = any amino acid
<221> VARIANT
   <222> 19
   <223> Xaa = hydrophobic residue
<400> 163
   Xaa Xaa Cys Xaa Xaa Xaa Xaa Xaa Cys Xaa Xaa Xaa Xaa Xaa Arg Xaa
   1 5 10   15
   Asp His Xaa Xaa Arg His Xaa Xaa Xaa Xaa Xaa His
   20 25
<210> 164
   <211> 28
   <212> PRT
   <213> Artificial Sequence
<220>
   <223> purified polypeptide
<221> VARIANT
   <222> 1, 13
   <223> Xaa = Phe or Tyr
<221> VARIANT
   <222> 2, 4-8 ,10-12, 14, 16, 20, 23-27
   <223> Xaa = any amino acid
<221> VARIANT
   <222> 19
   <223> Xaa = hydrophobic residue
<400> 164
   Xaa Xaa Cys Xaa Xaa Xaa Xaa Xaa Cys Xaa Xaa Xaa Xaa Xaa Arg Xaa
   1 5 10 15
   Asp His Xaa Xaa Thr His Xaa Xaa Xaa Xaa Xaa His
   20 25
<210> 165
   <211> 28
   <212> PRT
   <213> Artificial Sequence
<220>
   <223> purified polypeptide
<221> VARIANT
   <222> 1, 13
   <223> Xaa = Phe or Tyr
<221> VARIANT
   <222> 2, 4-8 ,10-12, 14, 16, 20, 23-27
   <223> Xaa = any amino acid
<221> VARIANT
   <222> 19
   <223> Xaa = hydrophobic residue
<400> 165
   Xaa Xaa Cys Xaa Xaa Xaa Xaa Xaa Cys Xaa Xaa Xaa Xaa Xaa Arg Xaa
   1 5 10 15
   Asp Lys Xaa Xaa Arg His Xaa Xaa Xaa Xaa Xaa His
   20 25
<210> 166
   <211> 28
   <212> PRT
   <213> Artificial Sequence
<220>
   <223> purified polypeptide
<221> VARIANT
   <222> 1, 13
   <223> Xaa = Phe or Tyr
<221> VARIANT
   <222> 2,4-8 ,10-12,14,16,20, 23-27)
   <223> Xaa = any amino acid
<221> VARIANT
   <222> 19
   <223> Xaa = hydrophobic residue
<400> 166
   Xaa Xaa Cys Xaa Xaa Xaa Xaa Xaa Cys Xaa Xaa Xaa Xaa Xaa Arg Xaa
   1 5 10 15
   Ser His Xaa Xaa Arg His Xaa Xaa Xaa Xaa Xaa His
   20 25
<210> 167
   <211> 28
   <212> PRT
   <213> Artificial Sequence
<220>
   <223> purified polypeptide
<221> VARIANT
   <222> 1, 13
   <223> Xaa = Phe or Tyr
<221> VARIANT
   <222> 19
   <223> Xaa = hydrophobic residue
<221> VARIANT
   <222> 2, 4-8, 10-12, 14, 16, 20, 23-27
   <223> Xaa = any amino acid
<400> 167
   Xaa Xaa Cys Xaa Xaa Xaa Xaa Xaa Cys Xaa Xaa Xaa Xaa Xaa His Xaa
   1 5 10 15
   Ser Ser Xaa Xaa Arg His Xaa Xaa Xaa Xaa Xaa His
   20 25
<210> 168
   <211> 28
   <212> PRT
   <213> Artificial Sequence
<220>
   <223> purified polypeptide
<221> VARIANT
   <222> 1, 13
   <223> Xaa = Phe or Tyr
<221> VARIANT
   <222> 19
   <223> Xaa = hydrophobic residue
<221> VARIANT
   <222> 2, 4-8 ,10-12, 14, 16, 20, 23-27
   <223> Xaa = any amino acid
<400> 168
   Xaa Xaa Cys Xaa Xaa Xaa Xaa Xaa Cys Xaa Xaa Xaa Xaa Xaa Ile Xaa
   1 5 10 15
   Ser Asn Xaa Xaa Arg His Xaa Xaa Xaa Xaa Xaa His
   20 25
<210> 169
   <211> 28
   <212> PRT
   <213> Artificial Sequence
<220>
   <223> purified polypeptide
<221> VARIANT
   <222> 1, 13
   <223> Xaa = Phe or Tyr
<221> VARIANT
   <222> 19
   <223> Xaa = hydrophobic residue
<221> VARIANT
   <222> 2,4 -8, 10-12, 14, 16, 20, 23-27
   <223> Xaa = any amino acid
<400> 169
   Xaa Xaa Cys Xaa Xaa Xaa Xaa Xaa Cys Xaa Xaa Xaa Xaa Xaa Lys Xaa
   1 5 10 15
   Ser Asn Xaa Xaa Arg His Xaa Xaa Xaa Xaa Xaa His
   20 25
<210> 170
   <211> 28
   <212> PRT
   <213> Artificial Sequence
<220>
   <223> purified polypeptide
<221 > VARIANT
   <222> 1, 13
   <223> Xaa = Phe or Tyr
<221> VARIANT
   <222> 19
   <223> Xaa = hydrophobic residue
<221> VARIANT
   <222> 2, 4-8, 10-12, 14, 16, 20, 23-27
   <223> Xaa = any amino acid
<400> 170
   Xaa Xaa Cys Xaa Xaa Xaa Xaa Xaa Cys Xaa Xaa Xaa Xaa Xaa Gln Xaa
   1 5 10 15
   Ser Asn Xaa Xaa Lys His Xaa Xaa Xaa Xaa Xaa His
   20 25
<210> 171
   <211> 28
   <212> PRT
   <213> Artificial Sequence
<220>
   <223> purified polypeptide
<221> VARIANT
   <222> 1, 13
   <223> Xaa = Phe or Tyr
<221> VARIANT
   <222> 19
   <223> Xaa = hydrophobic residue
<221> VARIANT
   <222> 2, 4-8, 10-12, 14, 16, 20, 23-27
   <223> Xaa = any amino acid
<400> 171
   Xaa Xaa Cys Xaa Xaa Xaa Xaa Xaa Cys Xaa Xaa Xaa Xaa Xaa Gln Xaa
   1 5 10 15
   Ser His Xaa Xaa Thr His Xaa Xaa Xaa Xaa Xaa His
   20 25
<210> 172
   <211> 28
   <212> PRT
   <213> Artificial Sequence
<220>
   <223> purified polypeptide
<221 > VARIANT
   <222> 1,13
   <223> Xaa = Phe or Tyr
<221> VARIANT
   <222> 19
   <223> Xaa = hydrophobic residue
<221> VARIANT
   <222> 2, 4-8, 10-12, 14, 16, 20, 23-27
   <223> Xaa = any amino acid
<400> 172
   Xaa Xaa Cys Xaa Xaa Xaa Xaa Xaa Cys Xaa Xaa Xaa Xaa Xaa Val Xaa
   1 5 10 15
   Ser Asn Xaa Xaa Val His Xaa Xaa Xaa Xaa Xaa His
   20 25
<210> 173
   <211> 23
   <212> PRT
   <213> Homo sapiens
<400> 173
   Phe Lys Cys Pro Val Cys Gly Lys Ala Phe Arg His Ser Ser Ser Leu
   1 5 10 15
   Val Arg His Gln Arg Thr His
   20
<210> 174
   <211> 69
   <212> DNA
   <213> Homo sapiens
<400> 174
   ttcaagtgcc cagtgtgcgg caaggccttc cggcatagct cctcgctggt gcggcaccag 60
   cgcacgcac 69
<210> 175
   <211> 24
   <212> PRT
   <213> Homo sapiens
<400> 175
   Tyr Arg Cys Lys Tyr Cys Asp Arg Ser Phe Ser Ile Ser Ser Asn Leu
   1 5 10 15
   Gln Arg His Val Arg Asn Ile His
   20
<210> 176
   <211> 72
   <212> DNA
   <213> Homo sapiens
<400> 176
   tacaggtgta agtactgcga ccgctccttc agcatctctt cgaacctcca gcggcacgtc 60
   cggaacatcc ac 72
<210> 177
   <211> 23
   <212> PRT
   <213> Homo sapiens
<400> 177
   Tyr Gly Cys His Leu Cys Gly Lys Ala Phe Ser Lys Ser Ser Asn Leu
   1 5 10 15
   Arg Arg His Glu Met Ile His
   20
<210> 178
   <211> 69
   <212> DNA
   <213> Homo sapiens
<400> 178
   tatggatgtc atctatgtgg gaaagccttc agtaaaagtt ctaaccttag acgacatgag 60
   atgattcac 69
<210> 179
   <211> 23
   <212> PRT
   <213> Homo sapiens
<400> 179
   Tyr Lys Cys Glu Glu Cys Gly Lys Ala Phe Thr Gln Ser Ser Asn Leu
   1 5 10 15
   Thr Lys His Lys Lys Ile His
   20
<210> 180
   <211> 69
   <212> DNA
   <213> Homo sapiens
<400> 180
   tacaagtgtg aagaatgtgg caaagctttt acccaatcct caaaccttac taaacataag 60
   aaaattcat 69
<210> 181
   <211> 23
   <212> PRT
   <213> Homo sapiens
<400> 181
   Tyr Glu Cys Val Gln Cys Gly Lys Gly Phe Thr Gln Ser Ser Asn Leu
   1 5 10 15
   Ile Thr His Gln Arg Val His
   20
<210> 182
   <211> 69
   <212> DNA
   <213> Homo sapiens
<400> 182
   tacgagtgtg tgcagtgtgg gaaaggtttc acccagagct ccaacctcat cacacatcaa 60
   agagttcac 69
<210> 183
   <211> 23
   <212> PRT
   <213> Homo sapiens
<400>183
   Tyr Glu Cys Asp His Cys Gly Lys Ala Phe Ser Val Ser Ser Asn Leu
   1 5 10 15
   Asn Val His Arg Arg Ile His
   20
<210> 184
   <211> 69
   <212> DNA
   <213> Homo sapiens
<400> 184
   tatgaatgcg atcactgtgg gaaagccttc agcgtcagct ccaacctgaa cgtgcacaga 60
   aggatccac 69
<210> 185
   <211> 23
   <212> PRT
   <213> Homo sapiens
<400> 185
   Tyr Thr Cys Ser Asp Cys Gly Lys Ala Phe Arg Asp Lys Ser Cys Leu
   1 5 10 15
   Asn Arg His Arg Arg Thr His
   20
<210> 186
   <211> 69
   <212> DNA
   <213> Homo sapiens
<400> 186
   tacacatgca gtgactgtgg gaaagctttc agagataaat catgtctcaa cagacatcgg 60
   agaactcat 69
<210> 187
   <211> 23
<212> PRT
   <213> Homo sapiens
<400> 187
   Tyr Glu Cys Asp His Cys Gly Lys Ala Phe Ser Ile Gly Ser Asn Leu
   1 5 10 15
   Asn Val His Arg Arg Ile His
   20
<210> 188
   <211> 69
   <212> DNA
   <213> Homo sapiens
<400> 188
   tacgaatgcg atcactgtgg gaaggccttc agcataggct ccaacctgaa tgtgcacagg 60
   cggatccat 69
<210> 189
   <211> 23
   <212> PRT
   <213> Homo sapiens
<400> 189
   Tyr Arg Cys Glu Glu Cys Gly Lys Ala Phe Arg Trp Pro Ser Asn Leu
   1 5 10 15
   Thr Arg His Lys Arg Ile His
   20
<210> 190
   <211> 69
   <212> DNA
   <213> Homo sapiens
<400> 190
   tacagatgtg aggaatgtgg caaagccttt aggtggccct caaaccttac tagacataag 60
   agaattcac 69
<210> 191
   <211> 25
   <212> PRT
   <213> Homo sapiens
<400> 191
   Phe Met Cys Thr Trp Ser Tyr Cys Gly Lys Arg Phe Thr Asp Arg Ser
   1 5 10 15
   Ala Leu Ala Arg His Lys Arg Thr His
   20 25
<210> 192
   <211> 69
   <212> DNA
   <213> Homo sapiens
<400> 192
   tactcctgtg gcatttgtgg caaatccttc tctgactcca gtgccaaaag gagacactgc 60
   attctacac 69
<210> 193
   <211> 28
   <212> PRT
   <213> Artificial Sequence
<220>
   <223> purified polypeptide
<221 > VARIANT
   <222> 1,13
   <223> Xaa = Phe or Tyr
<221> VARIANT
   <222> 19
   <223> Xaa = hydrophobic residue
<221> VARIANT
   <222> 2, 4-8, 10-12, 14, 16, 20, 23-27
   <223> Xaa = any amino acid
<400> 193
   Xaa Xaa Cys Xaa Xaa Xaa Xaa Xaa Cys Xaa Xaa Xaa Xaa Xaa Asp Xaa
   1 5 10 15
   Ser Cys Xaa Xaa Arg His Xaa Xaa Xaa Xaa Xaa His
   20 25
<210> 194
   <211> 28
   <212> PRT
   <213> Artificial Sequence
<220>
   <223> purified polypeptide
<221> VARIANT
   <222> 1, 13
   <223> Xaa = Phe or Tyr
<221> VARIANT
   <222> 19
   <223> Xaa = hydrophobic residue
<221> VARIANT
   <222> 2, 4-8, 10-12, 14, 16, 20, 23-27
   <223> Xaa = any amino acid
<400> 194
   Xaa Xaa Cys Xaa Xaa Xaa Xaa Xaa Cys Xaa Xaa Xaa Xaa Xaa Ile Xaa
   1 5 10 15
   Ser Asn Xaa Xaa Val His Xaa Xaa Xaa Xaa Xaa His
   20 25
<210> 195
   <211> 28
   <212> PRT
   <213> Artificial Sequence
<220>
   <223> purified polypeptide
<221> VARIANT
   <222> 1, 13
   <223> Xaa = Phe or Tyr
<221> VARIANT
   <222> 19
   <223> Xaa = hydrophobic residue
<221> VARIANT
   <222> 2, 4-8, 10-12, 14, 16, 20, 23-27
   <223> Xaa = any amino acid
<400> 195
   Xaa Xaa Cys Xaa Xaa Xaa Xaa Xaa Cys Xaa Xaa Xaa Xaa Xaa Trp Xaa
   1 5 10 15
   Ser Asn Xaa Xaa Arg His Xaa Xaa Xaa Xaa Xaa His
   20 25
<210> 196
   <211> 28
   <212> PRT
   <213> Artificial Sequence
<220>
   <223> purified polypeptide
<221> VARIANT
   <222> 1, 13
   <223> Xaa = Phe or Tyr
<221> VARIANT
   <222> 19
   <223> Xaa = hydrophobic residue
<221> VARIANT
   <222> 2, 4-8, 10-12, 14, 16, 20, 23-27
   <223> Xaa = any amino acid
<400> 196
   Xaa Xaa Cys Xaa Xaa Xaa Xaa Xaa Cys Xaa Xaa Xaa Xaa Xaa Asp Xaa
   1 5 10 15
   Ser Ala Xaa Xaa Arg His Xaa Xaa Xaa Xaa Xaa His
   20 25
<210> 197
   <211> 10
   <212> DNA
   <213> Artificial Sequence
<220>
   <223> synthetically generated oligonucleotide
<400> 197
   gcgtgggcgt 10
<210> 198
   <211> 56
   <212> PRT
   <213> Homo sapiens
<400> 198
   Glu Arg Pro Tyr Ala Cys Pro Val Glu Ser Cys Asp Arg Arg Phe Ser
   1 5 10 15
   Arg Ser Asp Glu Leu Thr Arg His Ile Arg Ile His Thr Gly Gln Lys
   20 25 30
   Pro Phe Gln Cys Arg Ile Cys Met Arg Asn Phe Ser Arg Ser Asp His
   35 40 45
   Leu Thr Thr His Ile Arg Thr His
   50 55
<210> 199
   <211> 10
   <212> DNA
   <213> Artificial Sequence
<220>
   <223> synthetically generated oligonucleotide
<400> 199
   gagagggagc 10
<210> 200
   <211> 10
   <212> DNA
   <213> Artificial Sequence
<220>
   <223> synthetically generated oligonucleotide
<400> 200
   tggggggaca 10
<210> 201
   <211> 10
   <212> DNA
   <213> Artificial Sequence
<220>
   <223> synthetically generated oligonucleotide
<400> 201
   gcggcggggc 10
<210> 202
   <211> 10
   <212> DNA
   <213> Artificial Sequence
<220>
   <223> synthetically generated oligonucleotide
<400> 202
   gtaggaggat 10
<210> 203
   <211> 10
   <212> DNA
   <213> Artificial Sequence
<220>
   <223> synthetically generated oligonucleotide
<400> 203
   agggcggggc 10
<210> 204
   <211> 10
   <212> DNA
   <213> Artificial Sequence
<220>
   <223> synthetically generated oligonucleotide
<400> 204
   gggaaacggg 10
<210> 205
   <211> 10
   <212> DNA
   <213> Artificial Sequence
<220>
   <223> synthetically generated oligonucleotide
<400> 205
   gtaggagagt 10
<210> 206
   <211> 10
   <212> DNA
   <213> Artificial Sequence
<220>
   <223> synthetically generated oligonucleotide
<400> 206
   ggaagggctt 10
<210> 207
   <211> 10
   <212> DNA
   <213> Artificial Sequence
<220>
   <223> synthetically generated oligonucleotide
<400> 207
   gagcaagtag 10
<210> 208
   <211> 10
   <212> DNA
   <213> Artificial Sequence
<220>
   <223> synthetically generated oligonucleotide
<400> 208
   gaggtgggag 10
<210> 209
   <211> 10
   <212> DNA
   <213> Artificial Sequence
<220>
   <223> synthetically generated oligonucleotide
<400> 209
   gaggacaatg 10
<210> 210
   <211> 10
   <212> DNA
   <213> Artificial Sequence
<220>
   <223> synthetically generated oligonucleotide
<400> 210
   ggggcgggat 10
<210> 211
   <211> 10
   <212> DNA
   <213> Artificial Sequence
<220>
   <223> synthetically generated oligonucleotide
<400> 211
   gagggagaag 10
<210> 212
   <211> 10
   <212> DNA
   <213> Artificial Sequence
<220>
   <223> synthetically generated oligonucleotide
<400> 212
   gaagagaggt 10
<210> 213
   <211> 10
   <212> DNA
   <213> Artificial Sequence
<220>
   <223> synthetically generated oligonucleotide
<400> 213
   gagtgagacc 10
<210> 214
   <211> 10
   <212> DNA
   <213> Artificial Sequence
<220>
   <223> synthetically generated oligonucleotide
<400> 214
   gaggcggaaa 10
<210> 215
   <211> 10
   <212> DNA
   <213> Artificial Sequence
<220>
   <223> synthetically generated oligonucleotide
<400> 215
   gggaaagaac 10
<210> 216
   <211> 10
   <212> DNA
   <213> Artificial Sequence
<220>
   <223> synthetically generated oligonucleotide
<400> 216
   gcggaagttc 10
<210> 217
   <211> 10
   <212> DNA
   <213> Artificial Sequence
<220>
   <223> synthetically generated oligonucleotide
<400> 217
   gagtgggaaa 10
<210> 218
   <211> 10
   <212> DNA
   <213> Artificial Sequence
<220>
   <223> synthetically generated oligonucleotide
<400> 218
   ggaggggggc 10
<210> 219
   <211> 10
   <212> DNA
   <213> Artificial Sequence
<220>
   <223> synthetically generated oligonucleotide
<400> 219
   tgggaggatc 10
<210> 220
   <211> 10
   <212> DNA
   <213> Artificial Sequence
<220>
   <223> synthetically generated oligonucleotide
<400> 220
   gtggggaaaa 10
<210> 221
   <211> 10
   <212> DNA
   <213> Artificial Sequence
<220>
   <223> synthetically generated oligonucleotide
<400> 221
   gaggttgagg 10
<210> 222
   <211> 10
   <212> DNA
   <213> Artificial Sequence
<220>
   <223> synthetically generated oligonucleotide
<400> 222
   agagaaggag 10
<210> 223
   <211> 10
   <212> DNA
   <213> Artificial Sequence
<220>
   <223> synthetically generated oligonucleotide
<400> 223
   tgagatgagc 10
<210> 224
   <211> 10
   <212> DNA
   <213> Artificial Sequence
<220>
   <223> synthetically generated oligonucleotide
<400> 224
   ggagaggctc 10
<210> 225
   <211> 10
   <212> DNA
   <213> Artificial Sequence
<220>
   <223> synthetically generated oligonucleotide
<400> 225
   agggctgatt 10
<210> 226
   <211> 10
   <212> DNA
   <213> Artificial Sequence
<220>
   <223> synthetically generated oligonucleotide
<400> 226
   ggggaggaaa 10
<210> 227
   <211> 10
   <212> DNA
   <213> Artificial Sequence
<220>
   <223> synthetically generated oligonucleotide
<400> 227
   agaggaaggt 10
<210> 228
   <211> 10
   <212> DNA
   <213> Artificial Sequence
<220>
   <223> synthetically generated oligonucleotide
<400> 228
   ggagaagtag 10
<210> 229
   <211> 10
   <212> DNA
   <213> Artificial Sequence
<220>
   <223> synthetically generated oligonucleotide
<400> 229
   ggtggcaggt 10
<210> 230
   <211> 10
   <212> DNA
   <213> Artificial Sequence
<220>
   <223> synthetically generated oligonucleotide
<400> 230
   gctggagggg 10
<210> 231
   <211> 10
   <212> DNA
   <213> Artificial Sequence
<220>
   <223> synthetically generated oligonucleotide
<400> 231
   gcggggaggg 10
<210> 232
   <211> 10
   <212> DNA .
   <213> Artificial Sequence
<220>
   <223> synthetically generated oligonucleotide
<400> 232
   aaagtgggga 10
<210> 233
   <211>10
   <212> DNA
   <213> Artificial Sequence
<220>
   <223> synthetically generated oligonucleotide
<400> 233
   agaaaaaata 10
<210> 234
   <211> 10
   <212> DNA
   <213> Artificial Sequence
<220>
   <223> synthetically generated oligonucleotide
<400> 234
   gacaggggag 10
<210> 235
   <211> 10
   <212> DNA
   <213> Artificial Sequence
<220>
   <223> synthetically generated oligonucleotide
<400> 235
   tgagttggga 10
<210> 236
   <211> 10
   <212> DNA
   <213> Artificial Sequence
<220>
   <223> synthetically generated oligonucleotide
<400> 236
   gaaggaaaat 10
<210> 237
   <211> 10
   <212> DNA
   <213> Artificial Sequence
<220>
   <223> synthetically generated oligonucleotide
<400> 237
   ggggctgagg 10
<210> 238
   <211> 10
   <212> DNA
   <213> Artificial Sequence
<220>
   <223> synthetically generated oligonucleotide
<400> 238
   tgagacggag 10
<210> 239
   <211> 10
   <212> DNA
   <213> Artificial Sequence
<220>
   <223> synthetically generated oligonucleotide
<400> 239
   gctggaaatt 10
<210> 240
   <211> 10
   <212> DNA
   <213> Artificial Sequence
<220>
   <223> synthetically generated oligonucleotide
<400> 240
   caagtagaag 10
<210> 241
   <211> 10
   <212> DNA
   <213> Artificial Sequence
<220>
   <223> synthetically generated oligonucleotide
<400> 241
   gaggcggaaa 10
<210> 242
   <211> 10
   <212> DNA
   <213> Artificial Sequence
<220>
   <223> synthetically generated oligonucleotide
<400> 242
   gctgcagcgt 10
<210> 243
   <211> 10
   <212> DNA
   <213> Artificial Sequence
<220>
   <223> synthetically generated oligonucleotide
<400> 243
   gatggggttt 10
<210> 244
   <211> 10
   <212> DNA
   <213> Artificial Sequence
<220>
   <223> synthetically generated oligonucleotide
<400> 244
   gaagcggagg 10
<210> 245
   <211> 10
   <212> DNA
   <213> Artificial Sequence
<220>
   <223> synthetically generated oligonucleotide
<400> 245
   gtggcggaag 10
<210> 246
   <211> 10
   <212> DNA
   <213> Artificial Sequence
<220>
   <223> synthetically generated oligonucleotide
<400> 246
   aaaggaaaag 10
<210> 247
   <211> 10
   <212> DNA
   <213> Artificial Sequence
<220>
   <223> synthetically generated oligonucleotide
<400> 247
   cgggttaaaa 10
<210> 248
   <211> 10
   <212> DNA
   <213> Artificial Sequence
<220>
   <223> synthetically generated oligonucleotide
<400> 248
   gtagctggga 10
<210> 249
   <211> 10
   <212> DNA
   <213> Artificial Sequence
<220>
   <223> synthetically generated oligonucleotide
<400> 249
   ggacaagcgg 10
<210> 250
   <211> 10
   <212> DNA
   <213> Artificial Sequence
<220>
   <223> synthetically generated oligonucleotide
<400> 250
   aaaagaaaaa 10
<210> 251
   <211> 131
   <212> DNA
   <213> Artificial Sequence
<220>
   <223> vector sequence
<400> 251
   gacccaagct tgccaccatg gtgtacccct acgacgtgcc cgactacgcc gaattgcctc 60
   caaaaaagaa gagaaaggta gggatccgaa ttcaagcggc cgcatgagat ctcgagcatg 120
   catctagagg g 131
<210> 252
   <211> 10
   <212> DNA
   <213> Artificial Sequence
<220>
   <223> synthetically generated oligonucleotide
<400> 252
   agagtagaat 10
<210> 253
   <211> 10
   <212> DNA
   <213> Artificial Sequence
<220>
   <223> synthetically generated oligonucleotide
<400> 253
   aaagtaaaaa 10
<210> 254
   <211> 10
   <212> DNA
   <213> Artificial Sequence
<220>
   <223> synthetically generated oligonucleotide
<400> 254
   agggagggag 10
<210> 255
   <211> 10
   <212> DNA
   <213> Artificial Sequence
<220>
   <223> synthetically generated oligonucleotide
<400> 255
   aaaaatgagc 10
<210> 256
   <211> 10
   <212> DNA
   <213> Artificial Sequence
<220>
   <223> synthetically generated oligonucleotide
<400> 256
   cgggaaagaa 10

<210> 257
   <211> 10
   <212> DNA
   <213> Artificial Sequence
<220>
   <223> synthetically generated oligonucleotide
<400> 257
   gtagcaagac 10
<210> 258
   <211> 10
   <212> DNA
   <213> Artificial Sequence
<220>
   <223> synthetically generated oligonucleotide
<400> 258
   aatgtaaaaa 10
<210> 259
   <211> 10
   <212> DNA
   <213> Artificial Sequence
<220>
   <223> synthetically generated oligonucleotide
<400> 259
   cggggagggg 10
<210> 260
   <211> 10
   <212> DNA
   <213> Artificial Sequence
<220>
   <223> synthetically generated oligonucleotide
<400> 260
   caaagagact 10
<210> 261
   <211> 10
   <212> DNA
   <213> Artificial Sequence
<220>
   <223> synthetically generated oligonucleotide
<400> 261
   gaagatgcgt 10
<210> 262
   <211> 10
   <212> DNA
   <213> Artificial Sequence
<220>
   <223> synthetically generated oligonucleotide
<400> 262
   cgagcatggg 10
<210> 263
   <211> 10
   <212> DNA
   <213> Artificial Sequence
<220>
   <223> synthetically generated oligonucleotide
<400> 263
   acaacagggg 10
<210> 264
   <211> 10
   <212> DNA
   <213> Artificial Sequence
<220>
   <223> synthetically generated oligonucleotide
<400> 264
   gttggggggc 10
<210> 265
   <211> 10
   <212> DNA
   <213> Artificial Sequence
<220>
   <223> synthetically generated oligonucleotide
<400> 265
   agggaggtgt 10
<210> 266
   <211> 10
   <212> DNA
   <213> Artificial Sequence
<220>
   <223> synthetically generated oligonucleotide
<400> 266
   tgagacgggg 10
<210> 267
   <211> 10
   <212> DNA
   <213> Artificial Sequence
<220>
   <223> synthetically generated oligonucleotide
<400> 267
   gaagttggaa 10
<210> 268
   <211> 10
   <212> DNA
   <213> Artificial Sequence
<220>
   <223> synthetically generated oligonucleotide
<400> 268
   agaaaagaaa 10
<210> 269
   <211> 10
   <212> DNA
   <213> Artificial Sequence
<220>
   <223> synthetically generated oligonucleotide
<400> 269
   gactgacaat 10
<210> 270
   <211> 10
   <212> DNA
   <213> Artificial Sequence
<220>
   <223> synthetically generated oligonucleotide
<400> 270
   gctgaggatg 10
<210> 271
   <211> 10
   <212> DNA
   <213> Artificial Sequence
<220>
   <223> synthetically generated oligonucleotide
<400> 271
   ggggagaaat 10
<210> 272
   <211> 10
   <212> DNA
   <213> Artificial Sequence
<220>
   <223> synthetically generated oligonucleotide
<400> 272
   tgatgagaaa 10
<210> 273
   <211> 10
   <212> DNA
   <213> Artificial Sequence
<220>
   <223> synthetically generated oligonucleotide
<400> 273
   gcaggagact 10
<210> 274
   <211> 10
   <212> DNA
   <213> Artificial Sequence
<220>
   <223> synthetically generated oligonucleotide
<400> 274
   tggggagatt 10
<210> 275
   <211> 10
   <212> DNA
   <213> Artificial Sequence
<220>
   <223> synthetically generated oligonucleotide
<400> 275
   gcggaatgga 10
<210> 276
   <211> 10
   <212> DNA
   <213> Artificial Sequence
<220>
   <223> synthetically generated oligonucleotide
<400> 276
   gtctggggac 10
<210> 277
   <211> 10
   <212> DNA
   <213> Artificial Sequence
<220>
   <223> synthetically generated oligonucleotide
<400> 277
   gagggggcgt 10
<210> 278
   <211> 10
   <212> DNA
   <213> Artificial Sequence
<220>
   <223> synthetically generated oligonucleotide
<400> 278
   gacgctgctt 10
<210> 279
   <211> 10
   <212> DNA
   <213> Artificial Sequence
<220>
   <223> synthetically generated oligonucleotide
<400> 279
   ggggcaggcg 10
<210> 280
   <211> 10
   <212> DNA
   <213> Artificial Sequence
<220>
   <223> synthetically generated oligonucleotide
<400> 280
   aaaaaaaaag 10
<210> 281
   <211> 10
   <212> DNA
   <213> Artificial Sequence
<220>
   <223> synthetically generated oligonucleotide
<400> 281
   ggaagagagg 10
<210> 282
   <211> 10
   <212> DNA
   <213> Artificial Sequence
<220>
   <223> synthetically generated oligonucleotide
<400> 282
   caagggaaaa 10
<210> 283
   <211> 10
   <212> DNA
   <213> Artificial Sequence
<220>
   <223> synthetically generated oligonucleotide
<400> 283
   ggtgatgcac 10
<210> 284
   <211> 10
   <212> DNA
   <213> Artificial Sequence
<220>
   <223> synthetically generated oligonucleotide
<400> 284
   aaggtcgcgg 10
<210> 285
   <211> 10
   <212> DNA
   <213> Artificial Sequence
<220>
   <223> synthetically generated oligonucleotide
<400> 285
   ggggctggag 10
<210> 286
   <211> 10
   <212> DNA
   <213> Artificial Sequence
<220>
   <223> synthetically generated oligonucleotide
<400> 286
   gggggtgtac 10
<210> 287
   <211> 10
   <212> DNA
   <213> Artificial Sequence
<220>
   <223> synthetically generated oligonucleotide
<400> 287
   ggtgatgctc 10
<210> 288
   <211> 10
   <212> DNA
   <213> Artificial Sequence
<220>
   <223> synthetically generated oligonucleotide
<400> 288
   gtttgagaca 10
<210> 289
   <211> 10
   <212> DNA
   <213> Artificial Sequence
<220>
   <223> synthetically generated oligonucleotide
<400> 289
   gctaaaaatc 10
<210> 290
   <211> 10
   <212> DNA
   <213> Artificial Sequence
<220>
   <223> synthetically generated oligonucleotide
<400> 290
   agaggagctt 10
<210> 291
   <211> 10
   <212> DNA
   <213> Artificial Sequence
<220>
   <223> synthetically generated oligonucleotide
<400> 291
   tgagatgggg 10
<210> 292
   <211> 10
   <212> DNA
   <213> Artificial Sequence
<220>
   <223> synthetically generated oligonucleotide
<400> 292
   gcttggggct 10
<210> 293
   <211> 10
   <212> DNA
   <213> Artificial Sequence
<220>
   <223> synthetically generated oligonucleotide
<400> 293
   gttggggcgg 10
<210> 294
   <211> 10
   <212> DNA
   <213> Artificial Sequence
<220>
   <223> synthetically generated oligonucleotide
<400> 294
   ggagctgctt 10
<210> 295
   <211> 10
   <212> DNA
   <213> Artificial Sequence
<220>
   <223> synthetically generated oligonucleotide
<400> 295
   gatgcaggac 10
<210> 296
   <211> 10
   <212> DNA
   <213> Artificial Sequence
<220>
   <223> synthetically generated oligonucleotide
<400> 296
   ggccgggtcg 10
<210> 297
   <211> 10
   <212> DNA
   <213> Artificial Sequence
<220>
   <223> synthetically generated oligonucleotide
<400> 297
   gatggtggtt 10
<210> 298
   <211> 10
   <212> DNA
   <213> Artificial Sequence
<220>
   <223> synthetically generated oligonucleotide
<400> 298
   gccggggcgg 10
<210> 299
   <211> 10
   <212> DNA
   <213> Artificial Sequence
<220>
   <223> synthetically generated oligonucleotide
<400> 299
   gctggggact 10
<210> 300
   <211> 10
   <212> DNA
   <213> Artificial Sequence
<220>
   <223> synthetically generated oligonucleotide
<400> 300
   gtagctgtaa 10
<210> 301
   <211> 10
   <212> DNA
   <213> Artificial Sequence
<220>
   <223> synthetically generated oligonucleotide
<400> 301
   gggggcggtt 10
<210> 302
   <211> 10
   <212> DNA
   <213> Artificial Sequence
<220>
   <223> synthetically generated oligonucleotide
<400> 302
   ggtgctgatt 10
<210> 303
   <211> 10
   <212> DNA
   <213> Artificial Sequence
<220>
   <223> synthetically generated oligonucleotide
<400> 303
   gcagtaggag 10
<210> 304
   <211> 10
   <212> DNA
   <213> Artificial Sequence
<220>
   <223> synthetically generated oligonucleotide
<400> 304
   gacgaagggg 10
<210> 305
   <211> 10
   <212> DNA
   <213> Artificial Sequence
<220>
   <223> synthetically generated oligonucleotide
<400> 305
   gacgacgctt 10